(19) Europäisches Patentamt
European Patent Office
Office européen des brevets

(11) EP 3 960 874 A1

(12) # EUROPEAN PATENT APPLICATION

(43) Date of publication:
**02.03.2022 Bulletin 2022/09**

(21) Application number: **20193231.6**

(22) Date of filing: **27.08.2020**

(51) International Patent Classification (IPC):
***C12Q 1/6886*** *(2018.01)*

(52) Cooperative Patent Classification (CPC):
**C12Q 1/6886;** C12Q 2600/106; C12Q 2600/158

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Moreira Borralho Relógio, Angela**
**10439 Berlin (DE)**

(72) Inventors:
- **Moreira Borralho Relógio, Angela**
  **10439 Berlin (DE)**
- **Hesse, Janina**
  **13629 Berlin (DE)**
- **Akhondzadeh Basti, Akhondzadeh Basti**
  **14167 Berlin (DE)**

(74) Representative: **Kilger, Ute**
**Boehmert & Boehmert**
**Anwaltspartnerschaft mbB**
**Pettenkoferstrasse 22**
**80336 München (DE)**

(54) **METHOD OF ASSESSING THE CIRCADIAN RHYTHM OF A SUBJECT HAVING CANCER AND/OR ASSESSING A TIMING OF ADMINISTRATION OF A MEDICAMENT TO SAID SUBJECT HAVING CANCER**

(57) Subject matter of the present invention is a method of assessing the circadian rhythm of a subject having cancer and/or assessing a timing of administration of a medicament to said subject having cancer, wherein said method comprises the steps of providing at least three samples of saliva, more preferably four samples of saliva, from said subject, wherein said samples have been taken at different time points over the day; and determining gene expression of at least the following genes in each of said samples: Bmall and Per2, and at least one gene involved in the metabolism of a medicament to be administered to said subject having cancer, including Ces2, and at least one drug target gene that is a target of the medicament to be administered. The method further comprises the step of assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

EP 3 960 874 A1

SEQUENCE LISTING

<110>  Moreira Borralho Relógio Angela

<120>  Method of assessing the circadian rhythm of a subject having cancer and/or assessing a timing of administration of a medicament to said subject having cancer

<130>  C75978EP

<160>  19

<170>  PatentIn version 3.5

<210>  1
<211>  1878
<212>  DNA
<213>  Human

<400>  1
atggcagacc agagaatgga catttcttca accatcagtg atttcatgtc cccgggcccc      60

accgacctgc tttccagctc tcttggtacc agtggtgtgg attgcaaccg caaacggaaa     120

ggcagctcca ctgactacca agaaagcatg gacacagaca aagatgaccc tcatggaagg     180

ttagaatata cagaacacca aggaaggata aaaaatgcaa gggaagctca cagtcagatt     240

gaaaagcggc gtcgggataa aatgaacagt tttatagatg aattggcttc tttggtacca     300

acatgcaacg caatgtccag gaaattagat aaacttactg tgctaaggat ggctgttcag     360

cacatgaaaa cattaagagg tgccaccaat ccatacacag aagcaaacta caaaccaact     420

tttctatcag acgatgaatt gaaacacctc attctcaggg cagcagatgg atttttgttt     480

gtcgtaggat gtgaccgagg gaagatactc tttgtctcag agtctgtctt caagatcctc     540

aactacagcc agaatgatct gattggtcag agtttgtttg actacctgca tcctaaagat     600

attgccaaag tcaaggagca gctctcctcc tctgacaccg caccccggga gcggctcata     660

gatgcaaaaa ctggacttcc agttaaaaca gatataaccc ctgggccatc tcgattatgt     720

tctggagcac gacgttcttt cttctgtagg atgaagtgta acaggccttc agtaaaggtt     780

gaagacaagg acttcccctc tacctgctca aagaaaaaag atcgaaaaag cttctgcaca     840

atccacagca caggctattt gaaaagctgg ccacccacaa agatggggct ggatgaagac     900

aacgaaccag acaatgaggg gtgtaacctc agctgcctcg tcgcaattgg acgactgcat     960

tctcatgtag ttccacaacc agtgaacggg gaaatcaggg tgaaatctat ggaatatgtt    1020

tctcggcacg cgatagatgg aaagtttgtt tttgtagacc agagggcaac agctattttg    1080

gcatatttac cacaagaact tctaggcaca tcgtgttatg aatattttca ccaagatgac    1140

ataggacatc ttgcagaatg tcataggcaa gttttacaga cgagagaaaa aattacaact    1200

aattgctata aatttaaaat caaagatggt tcttttatca cactacggag tcgatggttc    1260

agtttcatga acccttggac caaggaagta gaatatattg tctcaactaa cactgttgtt    1320

ttagccaacg tcctggaagg cggggaccca accttcccac agctcacagc atcccccac 1380

agcatggaca gcatgctgcc ctctggagaa ggtggcccaa agaggaccca ccccactgtt 1440

ccagggattc caggggaac ccgggctggg gcaggaaaaa taggccgaat gattgctgag 1500

gaaatcatgg aaatccacag gataagaggg tcatcgcctt ctagctgtgg ctccagccca 1560

ttgaacatca cgagtacgcc tccccctgat gcctcttctc caggaggcaa gaagatttta 1620

aatggaggga ctccagacat tccttccagt ggcctactat caggccaggc tcaggagaac 1680

ccaggttatc catattctga tagttcttct attcttggtg agaacccca cataggtata 1740

gacatgattg acaacgacca aggatcaagt agtcccagta atgatgaggc agcaatggct 1800

gtcatcatga gcctcttgga agcagatgct ggactgggtg gccctgttga ctttagtgac 1860

ttgccatggc cgctgtaa 1878

<210> 2
<211> 1911
<212> DNA
<213> Human

<400> 2
atggcggcgg aagaggaggc tgcggcggga ggtaaagtgt tgagagagga gaaccagtgc 60

attgctcctg tggtttccag ccgcgtgagt ccagggacaa gaccaacagc tatggggtct 120

ttcagctcac acatgacaga gtttccacga aaacgcaaag gaagtgattc agacccatcc 180

cagtcaggaa tcatgacaga aaaagtggtg gaaaagcttt ctcagaatcc ccttacctat 240

cttctttcaa caaggataga aatatcagcc tccagtggca gcagagtgga agatggtgaa 300

caccaagtta aaatgaaggc cttcagagaa gctcatagcc aaactgaaaa gcggaggaga 360

gataaaatga ataacctgat tgaagaactg tctgcaatga tccctcagtg caacccccatg 420

gcgcgtaaac tggacaaact tacagtttta agaatggctg ttcaacactt gagatcttta 480

aaaggcttga caaattctta tgtgggaagt aattatagac catcatttct tcaggataat 540

gagctcagac atttaatcct taagactgca gaaggcttct tatttgtggt tggatgtgaa 600

agaggaaaaa ttctcttcgt ttctaagtca gtctccaaaa tacttaatta tgatcaggct 660

agtttgactg gacaaagctt atttgacttc ttacatccaa aagatgttgc caaagtaaag 720

gaacaacttt cttcttttga tatttcacca agagaaaagc taatagatgc caaaactggt 780

ttgcaagttc acagtaatct ccacgctgga aggacacgtg tgtattctgg ctcaagacga 840

tctttttttct gtcggataaa gagttgtaaa atctctgtca aagaagagca tggatgctta 900

cccaactcaa agaagaaaga gcacagaaaa ttctatacta tccattgcac tggttacttg 960

agaagctggc ctccaaatat tgttggaatg gaagaagaaa ggaacagtaa gaaagacaac 1020

agtaatttta cctgccttgt ggccattgga agattacagc catatattgt tccacagaac 1080

agtggagaga ttaatgtgaa accaactgaa tttataaccc ggtttgcagt gaatggaaaa 1140

tttgtctatg tagatcaaag ggcaacagcg attttaggat atctgcctca ggaacttttg 1200

ggaacttctt gttatgaata ttttcatcaa gatgaccaca ataatttgac tgacaagcac 1260

aaagcagttc tacagagtaa ggagaaaata cttacagatt cctacaaatt cagagcaaaa 1320

gatggctctt ttgtaacttt aaaaagccaa tggtttagtt tcacaaatcc ttggacaaaa 1380

gaactggaat atattgtatc tgtcaacact ttagttttgg gacatagtga gcctggagaa 1440

gcatcatttt taccttgtag ctctcaatca tcagaagaat cctctagaca gtcctgtatg 1500

agtgtacctg gaatgtctac tggaacagta cttggtgctg gtagtattgg aacagatatt 1560

gcaaatgaaa ttctggattt acagaggtta cagtcttctt cataccttga tgattcgagt 1620

ccaacaggtt taatgaaaga tactcatact gtaaactgca ggagtatgtc aaataaggag 1680

ttgtttccac caagtccttc tgaaatgggg gagctagagg ctaccaggca aaaccagagt 1740

actgttgctg tccacagcca tgagccactc ctcagtgatg gtgcacagtt ggatttcgat 1800

gccctatgtg acaatgatga cacagccatg gctgcattta tgaattactt agaagcagag 1860

gggggcctgg gagaccctgg ggacttcagt gacatccagt ggaccctcta g 1911

<210> 3
<211> 3873
<212> DNA
<213> Human

<400> 3
atgagtggcc ccctagaagg ggctgatggg ggaggggacc ccaggcctgg ggaatcattt 60

tgtcctgggg gcgtcccatc ccctgggccc ccacagcacc ggccttgccc aggccccagc 120

ctggccgatg acaccgatgc caacagcaat ggttcaagtg gcaatgagtc caacgggcat 180

gagtctagag gcgcatctca gcggagctca cacagctcct cctcaggcaa cggcaaggac 240

tcagccctgc tggagaccac tgagagcagc aagagcacaa actctcagag cccatcccca 300

cccagcagtt ccattgccta cagcctcctg agtgccagct cagagcagga caacccgtcc 360

accagtggct gcagcagtga acagtcagcc cgggcaagga ctcagaagga actcatgaca 420

gcacttcgag agctcaagct tcgactgccg ccagagcgcc ggggcaaggg ccgctctggg 480

accctggcca cgctgcagta cgcactggcc tgtgtcaagc aggtgcaggc caaccaggaa 540

tactaccagc agtggagcct ggaggagggc gagccttgct ccatggacat gtccacctat 600

accctggagg agctggagca catcacgtct gagtacacac ttcagaacca ggataccttc 660

tcagtggctg tctccttcct gacgggccga atcgtctaca tttcggagca ggcagccgtc 720

ctgctgcgtt gcaagcggga cgtgttccgg ggtacccgct tctctgagct cctggctccc 780

caggatgtgg gagtcttcta tggttccact gctccatctc gcctgcccac ctggggcaca 840

ggggcctcag caggttcagg cctcagggac tttacccagg agaagtccgt cttctgccgt 900
atcagaggag gtcctgaccg ggatccaggg cctcggtacc agccattccg cctaaccccg 960
tatgtgacca agatccgggt ctcagatggg gcccctgcac agccgtgctg cctgctgatt 1020
gcagagcgca tccattcggg ttacgaagct ccccggatac cccctgacaa gaggattttc 1080
actacgcggc acacacccag ctgcctcttc caggatgtgg atgaaagggc tgccccccctg 1140
ctgggctacc tgccccagga cctcctgggg gccccagtgc tcctgttcct gcatcctgag 1200
gaccgacccc tcatgctggc tatccacaag aagattctgc agttggcggg ccagcccttt 1260
gaccactccc ctatccgctt ctgtgcccgc aacggggagt atgtcaccat ggacaccagc 1320
tgggctggct ttgtgcaccc ctggagccgc aaggtagcct tcgtgttggg ccgccacaaa 1380
gtacgcacgg ccccccctgaa tgaggacgtg ttcactcccc cggcccccag cccagctccc 1440
tccctggaca ctgatatcca ggagctgtca gagcagatcc accggctgct gctgcagccc 1500
gtccacagcc ccagccccac gggactctgt ggagtcggcg ccgtgacatc cccaggccct 1560
ctccacagcc ctgggtcctc cagtgatagc aacgggggtg atgcagaggg gcctgggcct 1620
cctgcgccag tgactttcca gcagatctgt aaggatgtgc atctggtgaa gcaccagggc 1680
cagcagcttt ttattgagtc tcgggcccgg cctcagtccc ggccccgcct ccctgctaca 1740
ggcacgttca aggccaaggc ccttccctgc caatccccag acccagagct ggaggcgggt 1800
tctgctcccg tccaggcccc actagccttg gtccctgagg aggccgagag gaaagaagcc 1860
tccagctgct cctaccagca gatcaactgc ctggacagca tcctcaggta cctggagagc 1920
tgcaacctcc ccagcaccac taagcgtaaa tgtgcctcct cctcctccta taccacctcc 1980
tcagcctctg acgacgacag gcagaggaca ggtccagtct ctgtggggac caagaaagat 2040
ccgccgtcag cagcgctgtc tggggagggg gccaccccac ggaaggagcc agtggtggga 2100
ggcaccctga gcccgctcgc cctggccaat aaggcggaga gtgtggtgtc cgtcaccagt 2160
cagtgtagct tcagctccac catcgtccat gtgggagaca agaagccccc ggagtcggac 2220
atcatcatga tggaggacct gcctggccta gccccaggcc cagcccccag cccagccccc 2280
agccccacag tagcccctga cccagcccca gacgcctacc gtccagtggg gctgaccaag 2340
gccgtgctgt ccctgcacac acagaaggaa gagcaagcct tcctcagccg cttccgagac 2400
ctgggcaggc tgcgtggact cgacagctct tccacagctc cctcagccct tggcgagcga 2460
ggctgccacc acggccccgc acccccaagc cgccgacacc actgccgatc caaagccaag 2520
cgctcacgcc accaccagaa ccctcgggct gaagcgccct gctatgtctc acacccctca 2580
cccgtgccac cctccacccc ctggcccacc ccaccagcca ctaccccctt cccagcggtt 2640
gtccagccct accctctccc agtgttctct cctcgaggag gcccccagcc tcttccccct 2700

```
gctcccacat ctgtgccccc agctgctttc cccgcccctt tggtgacccc aatggtggcc      2760

ttggtgctcc ctaactatct gttcccaacc ccatccagct atccttatgg ggcactccag      2820

acccctgctg aagggcctcc cactcctgcc tcgcactccc cttctccatc cttgcccgcc      2880

ctcgccccga gtcctcctca ccgcccggac tctccactgt tcaactcgag atgcagctct      2940

ccactccagc tcaatctgct gcagctggag gagctccccc gtgctgaggg ggctgctgtt      3000

gcaggaggcc ctgggagcag tgccgggccc ccacctccca gtgcggaggc tgctgagcca      3060

gaggccagac tggcggaggt cactgagtcc tccaatcagg acgcactttc cggctccagt      3120

gacctgctcg aacttctgct gcaagaggac tcgcgctccg gcacaggctc cgcagcctcg      3180

ggctccttgg gctctggctt gggctctggg tctggttcag gctcccatga agggggcagc      3240

acctcagcca gcatcactcg cagcagccag agcagccaca caagcaaata ctttggcagc      3300

atcgactctt ccgaggctga ggctggggct gctcggggcg gggctgagcc tggggaccag      3360

gtgattaagt acgtgctcca ggatcccatt tggctgctca tggccaatgc tgaccagcgc      3420

gtcatgatga cctaccaggt gccctccagg gacatgacct ctgtgctgaa gcaggatcgg      3480

gagcggctcc gagccatgca gaagcagcag cctcggtttt ctgaggacca gcggcgggaa      3540

ctgggtgctg tgcactcctg ggtccggaag ggccaactgc ctcgggctct tgatgtgatg      3600

gcctgtgtgg actgtgggag cagcacccaa gatcctggtc accctgatga cccactcttc      3660

tcagagctgg atggactggg gctggagccc atggaagagg gtggaggcga gcagggcagc      3720

agcggtggcg gcagtggtga gggagagggc tgcgaggagg cccaaggcgg ggccaaggct      3780

tcaagctctc aggacttggc tatggaggag gaggaagaag gcaggagctc atccagtcca      3840

gccttaccta cagcaggaaa ctgcaccagc tag                                  3873


<210>  4
<211>  3768
<212>  DNA
<213>  Human

<400>  4
atgaatggat acgcggaatt tccgcccagc cccagtaacc ccaccaagga gcccgtggag        60

ccccagccca gccaggtccc actgcaggaa gatgtggaca tgagcagtgg ctccagtgga       120

catgagacca acgaaaactg ctccacgggg cgggactcgc agggcagtga ctgtgacgac       180

agtgggaagg agctggggat gctggtggag ccaccggatg cccgccagag tccagatacc       240

tttagcctga tgatggcaaa atctgaacac aacccatcta caagtggctg cagtagcgac       300

cagtcttcga aagtggacac acacaaagaa ctgataaaaa cactaaagga gctgaaggtc       360

cacctccctg cagacaagaa ggccaagggc aaggccagta cgctggccac cttgaagtac       420

gccctcagga gcgtgaagca ggtgaaagcc aatgaagagt attaccagct gctgatgtcc       480
```

agcgagggtc acccctgtgg agcagacgtg ccctcctaca ccgtggagga gatggagagc 540

gttacctctg agcacattgt gaagaatgcc gatatgtttg cggtggccgt gtccctggtg 600

tctgggaaga tcctgtacat ctctgaccag gttgcatcca tatttcactg taaaagagat 660

gccttcagcg atgccaagtt tgtggagttc ctggcgcctc acgatgtggg cgtgttccac 720

agtttcacct ccccgtacaa gcttcccttg tggagcatgt gcagtggagc agattctttt 780

actcaagaat gcatggagga gaaatctttc ttttgccgtg tcagtgtccg gaaaagccac 840

gagaatgaaa tccgctacca ccccttccgc atgacgccct acctggtcaa ggtgcgggac 900

caacaaggtg ctgagagtca gctttgctgc cttctgctgg cagagagagt gcactctggt 960

tatgaagccc ctagaattcc tcctgaaaag agaattttta caaccaccca tacaccaaat 1020

tgtttgttcc aggatgtgga tgaaagggcg gtccctctcc tgggctacct acctcaggac 1080

ctgattgaaa ccccagtgct cgtgcagctc caccctagtg acaggccctt gatgctggcc 1140

atccacaaaa agatcctgca gtcaggcggg cagcctttcg actattctcc cattcggttt 1200

cgcgcccgga acggagagta catcacgttg gacaccagct ggtccagctt catcaaccca 1260

tggagcagga aaatctcctt catcattggg aggcacaaag tcagggtggg ccctttgaat 1320

gaggacgtgt ttgcagccca cccctgcaca gaggagaagg ccctgcaccc cagcattcag 1380

gagctcacag agcagatcca ccggctcctg ctgcagcccg tcccccacag cggctccagt 1440

ggctacggga gtctgggcag caacgggtcc cacgagcacc ttatgagcca gacctcctcc 1500

agcgacagca acggccatga ggactcacgc cggaggagag ccgaaatttg taaaaatggt 1560

aacaagacca aaaatagaag tcattattct catgaatctg gagaacaaaa gaaaaaatcc 1620

gttacagaaa tgcaaactaa tcccccagct gagaagaaag ctgtccctgc catggaaaag 1680

gacagcctgg gggtcagctt ccccgaggag ttggcctgca agaaccagcc cacctgctcc 1740

taccagcaga tcagctgctt ggacagcgtc atcaggtact tggagagctg caatgaggct 1800

gccaccctga agaggaaatg cgagttccca gcaaacgtcc cagcgctaag gtccagtgat 1860

aagcggaagg ccacagtcag cccagggcca cacgctggag aggcagagcc gccctccagg 1920

gtgaacagcc gcacgggagt aggtacgcac ctgacctcgc tggcactgcc gggcaaggca 1980

gagagtgtgg cgtcgctcac cagccagtgc agctacagca gcaccatcgt ccatgtggga 2040

gacaagaagc cgcagccgga gttagagatg gtggaagatg ctgcgagtgg gccagaatcc 2100

ctggactgcc tggcgggccc tgccctggcc tgtggtctca gccaagagaa ggagcccttc 2160

aagaagctgg gcctcaccaa ggaggtactc gctgcacaca cacagaagga ggagcagagc 2220

ttcctgcaga agttcaaaga aataagaaaa ctcagcattt tccagtccca ctgccattac 2280

tacttgcaag aaagatccaa ggggcagcca agtgaacgaa ctgcccctgg actaagaaat 2340

acttccggaa tagattcacc ttggaaaaaa acaggaaaga acagaaaatt gaagtccaag 2400

cgggtcaaac ctcgagactc atctgagagc accggatctg gggggcccgt gtccgcccgg 2460

cccccgctgg tgggcttgaa cgccacagcc tggtcaccct cagacacgtc ccagtccagc 2520

tgcccagccg tgccctttcc cgccccagtg ccagcagctt attcactgcc cgtgtttcca 2580

gcgccaggga ctgtggcagc acccccggca cctccccacg ccagcttcac agtgcctgct 2640

gtgcccgtgg acctccagca ccagtttgca gtccagcccc cacctttccc tgcccctttg 2700

gcgcctgtca tggcattcat gctacccagt tattccttcc cctcggggac cccaaacctg 2760

ccccaggcct tcttccccag ccagcctcag tttccgagcc accccacact cacatccgag 2820

atggcctctg cctcacagcc tgagttcccc agccggacct cgatccccag acagccatgt 2880

gcttgtccag ccacccgggc caccccacca tcggccatgg gtagggcctc cccaccgctc 2940

tttcagtccc gcagcagctc gcccctgcag ctcaacctgc tgcagctgga ggaagcccct 3000

gagggtggca ctggagccat ggggaccaca ggggccacag agacagcagc tgtaggggcg 3060

gactgcaaac ctggcacttc tcgggaccag cagccgaagg cgcctctgac ccgtgatgaa 3120

ccctcagaca cacagaacag tgacgccctt tccacgtcaa gcggcctcct aaacctcctg 3180

ctgaatgagg acctctgctc agcctcgggc tctgctgctt cggagtctct gggctccggc 3240

tcactgggct gcgacgcctc cccgagtggg gcaggcagta gtgacacaag tcataccagc 3300

aaatattttg gaagcattga ctcctcagag aataatcaca aagcaaaaat gaacactggt 3360

atggaagaaa gtgagcattt cattaagtgc gtcctgcagg atcccatctg gctgctgatg 3420

gcagatgcgg acagcagcgt catgatgacg taccagctgc cttcccgaaa tttagaagcg 3480

gttttgaagg aggacagaga gaagctgaag ctcctacaga aactccagcc caggttcacg 3540

gagagtcaga agcaggagct gcgcgaggtc caccagtgga tgcagacggg cggcctgccc 3600

gcagccatcg acgtggcaga atgtgtttac tgtgaaaaca aggaaaaagg taatatttgc 3660

ataccatatg aggaagatat tccttctctg ggactcagcg aagtgtcgga caccaaagaa 3720

gacgaaaatg gatcccccct gaatcacagg atcgaagagc agacgtaa 3768

<210> 5
<211> 3633
<212> DNA
<213> Human

<400> 5
atgccccgcg gggaagctcc tggccccggg agacgggggg ctaaggacga ggccctgggc 60

gaagaatcgg gggagcggtg gagccccgag ttccatctgc agaggaaatt ggcggacagc 120

agccacagtg aacagcaaga tcgaaacaga gtttctgaag aacttatcat ggttgtccaa 180

gaaatgaaaa aatacttccc ctcggagaga cgcaataaac caagcactct agatgccctc 240

aactatgctc tccgctgtgt ccacagcgtt caagcaaaca gtgagttttt ccagattctc 300

agtcagaatg gagcacctca ggcagatgtg agcatgtaca gtcttgagga gctggccact 360
atcgcttcag aacacacttc caaaaacaca gatacctttg tggcagtatt ttcatttctg 420
tctggaaggt tagtgcacat ttctgaacag gctgctttga tcctgaatcg taagaaagat 480
gtcctggcgt cttctcactt tgttgacctg cttgcacctc aagacatgag ggtattctac 540
gcgcacactg ccagagctca gcttcctttc tggaacaact ggacccaaag agcagctgca 600
cggtatgaat gtgctccggt gaaacctttt ttctgcagga tccgtggagg tgaagacaga 660
aagcaagaga agtgtcactc cccattccgg atcatcccct atctgattca tgtacatcac 720
cctgcccagc cagaattgga atcggaacct tgctgtctca ctgtggttga aaagattcac 780
tctggttatg aagctcctcg gatcccagtg aataaaagaa tcttcaccac cacacacacc 840
ccagggtgtg tttttcttga agtagatgaa aaagcagtgc ctttgctggg ttacctacct 900
caggacctga ttggaacatc gatcctaagc tacctgcacc ctgaagatcg ttctctgatg 960
gttgccatac accaaaaagt tttgaagtat gcagggcatc ctccctttga acattctccc 1020
attcgatttt gtactcaaaa cggagactac atcatactgg attccagttg gtccagcttt 1080
gtgaatccct ggagccggaa gatttctttc atcattggtc ggcataaagt tcgaacgagc 1140
ccactaaatg aggatgtttt tgctaccaaa attaaaaaga tgaacgataa tgacaaagac 1200
ataacagaat tacaagaaca aatttacaaa cttctcttac agccagttca cgtgagcgtg 1260
tccagcggct acgggagcct ggggagcagc gggtcgcagg agcagcttgt cagcatcgcc 1320
tcctccagtg aggccagtgg gcaccgtgtg gaggagacga aggcggagca gatgaccttg 1380
cagcaggtct atgccagtgt gaacaaaatt aaaaatctgg gtcagcagct ctacattgag 1440
tcaatgacca aatcatcatt caagccagtg acggggacac gcacagaacc gaatggtggt 1500
ggtgagtcag cgaatggtgg tggtgaatgt aagaccttta cttccttcca ccaaacactg 1560
aaaaacaata gtgtgtacac tgagccctgt gaggatttga ggaacgatga gcacagccca 1620
tcctatcaac agatcaactg tatcgacagt gtcatcagat acctgaagag ctacaacatt 1680
ccagctttga aaagaaagtg tatctcctgt acaaatacaa cttcttcctc ctcagaagaa 1740
gacaaacaga accacaaggc agatgatgtc caagccttac aagctggttt gcaaatccca 1800
gccataccta aatcagaaat gccaacaaat ggacggtcca tagacacagg aggaggagct 1860
ccacagatcc tgtccacggc gatgctgagc ttggggtcgg gcataagcca atgcggttac 1920
agcagcacca ttgtccatgt cccacccccca gagacagcca gggatgctac cctcttctgt 1980
gagccctgga ccctgaacat gcagccagcc cctttgacct cggaagaatt taaacacgtg 2040
gggctcacag cggctgttct gtcagcgcac acccagaagg aagagcagaa ttatgttgat 2100
aaattccgag aaaagatcct gtcatcaccc tacagctcct atcttcagca agaaagcagg 2160

agcaaagcta aatattcata ttttcaagga gattctactt ccaagcagac gcggtcggcc 2220

ggctgcagga aagggaagca caagcggaag aagctgccgg agccgccaga cagcagcagc 2280

tcgaacaccg gctctggtcc ccgcagggga gcgcatcaga acgcacagcc ctgctgcccc 2340

tccgcggcct cctctccgca cacctcgagc ccgaccttcc cacctgccgc catggtgccc 2400

agccaggccc cttacctcgt cccagctttt cccctcccag ccgcgacctc acccggaaga 2460

gaatacgcag cccccggaac tgcaccggaa ggcctgcatg ggctgccctt gtccgagggc 2520

ttgcagcctt acccagcttt ccccttttcct tacttggata cttttatgac cgttttcctg 2580

cctgaccccc ctgtctgtcc tctgttgtcg ccatcgtttt tgccatgtcc attcctgggg 2640

gcgacagcct cttctgcgat atcaccctca atgtcgtcag caatgagtcc aactctggac 2700

ccaccccctt cagtcaccag ccaaaggaga gaggaggaaa agtgggaggc acaaagcgag 2760

gggcacccgt tcattacttc gagaagcagc tcacccttgc agttaaactt acttcaggaa 2820

gagatgccca gaccctctga atctccagat cagatgagaa ggaacacgtg cccacaaact 2880

gagtatcagt gtgttacagg caacaatggc agtgagagca gtcctgctac taccggtgca 2940

ctgtccacgg ggtcacctcc cagggagaat ccatcccatc ctactgccag cgctctgtcc 3000

acaggatcgc ctcccatgaa gaatccatcc catcctactg ccagcgctct gtccacagga 3060

tcgcctccca tgaagaatcc atcccatcct actgccagca cactgtccat gggattgcct 3120

cccagcagga ctccatccca tcctactgcc actgttctgt ccacggggtc acctcccagc 3180

gaatccccat ccagaactgg ttcagcagca tcaggaagca gcgacagcag tatatacctt 3240

actagtagtg tttattcttc taaaatctcc caaaatgggc agcaatctca ggacgtacag 3300

aaaaaagaaa catttcctaa tgtcgccgaa gagcccatct ggagaatgat acggcagaca 3360

cctgagcgca ttctcatgac ataccaggta cctgagaggg ttaaagaagt tgtactaaaa 3420

gaagacctgg aaaagctaga aagtatgagg cagcagcagc cccagttttc tcatgggcaa 3480

aaggaggagc tggctaaggt gtataattgg attcaaagcc agactgtcac tcaagaaatc 3540

gacattcaag cctgtgtcac ttgtgaaaat gaagattcag ctgatggtgc ggccacatcc 3600

tgtggtcagg ttctggtaga agacagctgt tga 3633

<210> 6
<211> 2541
<212> DNA
<213> Human

<400> 6
atgttgttta ccgtaagctg tagtaaaatg agctcgattg ttgacagaga tgacagtagt 60

atttttgatg ggttggtgga agaagatgac aaggacaaag cgaaaagagt atctagaaac 120

aaatctgaaa agaaacgtag agatcaattt aatgttctca ttaaagaact gggatccatg 180

```
cttcctggta atgctagaaa gatggacaaa tctactgttc tgcagaaaag cattgatttt    240
ttacgaaaac ataaagaaat cactgcacag tcagatgcta gtgaaattcg acaggactgg    300
aaacctacat tccttagtaa tgaagagttt acacaattaa tgttagaggc tcttgatggt    360
tttttttag caatcatgac agatggaagc ataatatatg tgtctgagag tgtaacttca    420
ttacttgaac atttaccatc tgatcttgtg gatcaaagta tatttaattt tatcccagaa    480
ggggaacatt cagaggttta taaaatactc tctactcatc tgctggaaag tgattcatta    540
accccagaat atttaaaatc aaaaaatcag ttagaattct gttgtcacat gctgcgagga    600
acaatagacc caaaggagcc atctacctat gaatatgtaa aatttatagg aaatttcaaa    660
tctttaaaca gtgtatcctc ttcagcacac aatggttttg aaggaactat acaacgcaca    720
cataggccat cttatgaaga tagagtttgt tttgtagcta ctgtcaggtt agctacacct    780
cagttcatca aggaaatgtg cactgttgaa gaacccaatg aagagtttac atctagacat    840
agtttagaat ggaagtttct gtttctagat cacagggcac cacccataat agggtatttg    900
ccatttgaag ttctgggaac atcaggctat gattactatc atgtggatga cctagaaaat    960
ttggcaaaat gtcatgagca cttaatgcaa tatgggaaag gcaaatcatg ttattatagg   1020
ttcctgacta aggggcaaca gtggatttgg cttcagactc attattatat cacttaccat   1080
cagtggaatt caaggccaga gtttattgtt tgtactcaca ctgtagtaag ttatgcagaa   1140
gttagggctg aaagacgacg agaacttggc attgaagagt ctcttcctga gacagctgct   1200
gacaaaagcc aagattctgg gtcagataat cgtataaaca cagtcagtct caaggaagca   1260
ttggaaaggt ttgatcacag cccaacccct tctgcctctt ctcggagttc aagaaaatca   1320
tctcacacgg ccgtctcaga cccttcctca acaccaacca agatcccgac ggatacgagc   1380
actccaccca ggcagcattt accagctcat gagaagatgg tgcaaagaag gtcatcattt   1440
agtagtcagt ccataaattc ccagtctgtt ggttcatcat taacacagcc agtgatgtct   1500
caagctacaa atttaccaat tccacaaggc atgtcccagt ttcagttttc agctcaatta   1560
ggagccatgc aacatctgaa agaccaattg gaacaacgga cacgcatgat agaagcaaat   1620
attcatcggc aacaagaaga actaagaaaa attcaagaac aacttcagat ggtccatggt   1680
caggggctgc agatgttttt gcaacaatca aatcctgggt tgaattttgg ttccgttcaa   1740
ctttcttctg gaaattcatc taatatccag caacttgcac ctataaatat gcaaggccaa   1800
gttgttccta ctaaccagat tcaaagtgga atgaatactg gacacattgg cacaactcag   1860
cacatgatac aacaacagac tttacagagt acatcaactc agagtcaaca aaatgtactg   1920
agtgggcaca gtcagcaaac atctctaccc agtcagacac agagcactct tacagcccca   1980
ctgtataaca ctatggtgat ttctcagcct gcagccggaa gcatggtcca gattccatct   2040
agtatgccac aaaacagcac ccagagtgct gcagtaacta cattcactca ggacaggcag   2100
```

ataagatttt ctcaaggtca acaacttgtg accaaattag tgactgctcc tgtagcttgt 2160

ggggcagtca tggtacctag tactatgctt atgggccagg tggtgactgc atatcctact 2220

tttgctacac aacagcaaca gtcacagaca ttgtcagtaa cgcagcagca gcagcagcag 2280

agctcccagg agcagcagct cacttcagtt cagcaaccat ctcaggctca gctgacccag 2340

ccaccgcaac aatttttaca gacttctagg ttgctccatg ggaatccctc aactcaactc 2400

attctctctg ctgcatttcc tctacaacag agcaccttcc ctcagtcaca tcaccagcaa 2460

catcagtctc agcaacagca gcaactcagc cggcacagga ctgacagctt gcccgaccct 2520

tccaaggttc aaccacagta g 2541

<210> 7
<211> 2475
<212> DNA
<213> Human

<400> 7
atggatgaag atgagaaaga cagagccaag agagcttctc gaaacaagtc tgagaagaag 60

cgtcgggacc agttcaatgt tctcatcaaa gagctcagtt ccatgctccc tggcaacacg 120

cggaaaatgg acaaaaccac cgtgttggaa aaggtcatcg gatttttgca gaaacacaat 180

gaagtctcag cgcaaacgga aatctgtgac attcagcaag actggaagcc ttcattcctc 240

agtaatgaag aattcaccca gctgatgttg gaggcattag atggcttcat tatcgcagtg 300

acaacagacg gcagcatcat ctatgtctct gacagtatca cgcctctcct tgggcattta 360

ccgtcggatg tcatggatca gaatttgtta aatttcctcc cagaacaaga acattcagaa 420

gtttataaaa tcctttcttc ccatatgctt gtgacggatt ccccctcccc agaatactta 480

aaatctgaca gcgatttaga gttttattgc catcttctca gaggcagctt gaacccaaag 540

gaatttccaa cttatgaata cataaaattt gtaggaaatt ttcgctctta caacaatgtg 600

cctagcccct cctgtaatgg ttttgacaac acccttttcaa gaccttgccg ggtgccacta 660

ggaaaggagg tttgcttcat tgccaccgtt cgtctggcaa caccacaatt cttaaaggaa 720

atgtgcatag ttgacgaacc tttagaggaa ttcacttcaa ggcatagctt ggaatggaaa 780

tttttatttc tggatcacag agcacctcca atcataggat acctgccttt tgaagtgctg 840

ggaacctcag gctatgacta ctaccacatt gatgacctgg agctcctggc caggtgtcac 900

cagcacctga tgcagtttgg caaagggaag tcgtgttgct accggtttct gaccaaaggt 960

cagcagtgga tctggctgca gactcactac tacatcacct accatcagtg gaactccaag 1020

cccgagttca tcgtgtgcac acactcggtg gtcagttacg cagatgtccg ggtggaaagg 1080

aggcaggagc tggctctgga agacccgcca tccgaggccc tccactcctc agcactaaag 1140

gacaagggct caagcctgga acctcggcag cactttaaca cactcgacgt gggtgcctcg 1200

ggccttaata ccagtcattc gccatcggcg tcctcaagaa gttcccacaa atcctcgcac 1260

acagccatgt cagaacccac ctccactccc accaagctga tggcagaggc cagcaccccg 1320

gctttgccaa gatcagccac cctgccccaa gagttacctg tccccgggct cagccaggca 1380

gccaccatgc cggcccctct gccttcccca tcgtcctgcg acctcacaca gcagctcctg 1440

cctcagaccg ttctgcagag cacgcccgct cccatggcac agttttcggc acagttcagc 1500

atgttccaga ccatcaaaga ccagctagag cagcggacgc ggatcctgca ggccaatatc 1560

cggtggcaac aggaagagct ccacaagatc caggagcagc tctgcctggt ccaggactcc 1620

aacgtccaga tgttcctgca gcagccagct gtatccctga gcttcagcag cacccagcga 1680

cctgaggctc agcagcagct acagcaaagg tcagctgcag tgactcagcc ccagctcggg 1740

gcgggccccc aacttccagg gcagatctcc tctgcccagg tcacaagcca gcacctgctc 1800

agagaatcaa gtgtgatatc aacccagggt ccaaagccaa tgagaagctc acagctaatg 1860

cagagcagcg gccgctctgg aagcagccta gtgtccccgt tcagcagcgc cacagctgcg 1920

ctcccgccaa gtctgaatct gaccacacct gcttccacct cccaggatgc cagccagtgc 1980

cagcccagcc cagacttcag ccatgatcgg cagctcaggc tgttgctgag ccagcccatc 2040

cagcccatga tgcccgggtc ctgtgacgca aggcagccct cggaagtcag caggacggga 2100

cggcaagtca agtacgccca gagccagacc gtgtttcaaa atccagacgc acaccccgcc 2160

aacagcagca gcgccccgat gcccgtcctg ctgatggggc aggcggtgct ccaccccagc 2220

ttccctgcct cccaaccatc gcccctgcag cctgcacagg cccggcagca gccaccgcag 2280

cactacctgc aggtacaggc accaacctct ttgcacagtg agcagcagga ctcgctactt 2340

ctctccacct actcacaaca gccagggacc ctgggctacc cccaaccacc cccagcacag 2400

ccccagcccc tacgtcctcc ccgaagggtc agcagtctgt ctgagtcgtc aggcctccag 2460

cagccgcccc gataa 2475

<210> 8
<211> 1761
<212> DNA
<213> Human

<400> 8
atgggggtga acgccgtgca ctggttccga aaggggctcc ggctccacga caaccccgcc 60

ctgaaggagt gcattcaggg cgccgacacc atccgctgcg tctacatcct ggacccctgg 120

ttcgccggct cctccaatgt gggcatcaac aggtggcgat ttttgcttca gtgtcttgag 180

gatcttgatg ccaatctacg aaaattaaac tcccgtctgt ttgtgattcg tggacaacca 240

gcagatgtgt ttcccaggct tttcaaggaa tggaacatta ctaaactttc aattgagtat 300

gattctgagc cctttggaaa ggaacgagac gcagctatta agaaactggc aactgaagct 360

ggagtagaag tcattgtaag aatttcacat acattatatg acctagacaa gatcatagaa 420

ctcaatggtg gacaaccgcc tctaacttat aaaagattcc agactctcat cagcaaaatg 480

gaaccactag agataccagt agagacaatt acttcagaag tgatagaaaa gtgcacaact 540

cctctgtctg atgaccatga tgagaaatat ggagtccctt cactggaaga gctaggtttt 600

gatacagatg gcttatcctc tgcagtgtgg ccaggtggag aaactgaagc acttactcgt 660

ttggaaaggc atttggaaag aaaagcttgg gtggcaaatt ttgaaagacc tcgaatgaat 720

gcgaattctc tgcttgcaag ccctactgga cttagtcctt atctccgatt tggttgtttg 780

tcatgtcgac tgttttactt caaactaaca gatctctaca aaaaggtaaa gaagaacagt 840

tcccctcccc tttcccttta tgggcaactg ttatggcgtg aatttttcta tacagcagca 900

acaaataatc cacgctttga taaaatggaa ggaaacccta tctgtgttca gattccttgg 960

gataaaaatc ctgaggcttt agccaaatgg gcggaaggcc ggacaggctt tccatggatt 1020

gatgccatca tgacacagct tcgtcaggag ggttggattc atcatctagc caggcatgca 1080

gttgcttgct tcctgacacg aggggacctg tggattagtt gggaagaagg aatgaaggta 1140

tttgaagaat tattgcttga tgcagattgg agcataaatg ctggaagttg gatgtggctg 1200

tcttgtagtt ccttttttca acagtttttt cactgctatt gccctgttgg ttttggtagg 1260

agaacagatc ccaatggaga ctatatcagg cgttatttgc ctgtcctaag aggcttccct 1320

gcaaaatata tctatgatcc ctggaatgca ccagaaggta tccaaaaggt agccaaatgt 1380

ttgataggag ttaattatcc taaaccaatg gtgaaccatg ctgaggcaag ccgtttgaat 1440

atcgaaagga tgaaacagat ctatcagcag ctttcacgat atagaggact aggtcttctg 1500

gcatcagtac cttctaatcc taatgggaat ggaggcttca tgggatattc tgcagaaaat 1560

atcccaggtt gtagcagcag tggaagttgc tctcaaggga gtggtatttt acactatgct 1620

catggcgaca gtcagcaaac tcacctgttg aagcaaggaa gaagctccat gggcactggt 1680

ctcagtggtg ggaaacgtcc tagtcaggaa gaggacacac agagtattgg tcctaaagtc 1740

cagagacaga gcactaatta g 1761

<210> 9
<211> 1845
<212> DNA
<213> Human

<400> 9
atgggcgggg tccacgtcgc ctaccggggc ggagcggggg tggctggagc agtctggaca 60

gtcatggcgg cgactgtggc gacggcggca gctgtggccc cggcgccagc gcccggcacg 120

gacagcgcct cttcggtgca ctggttccgc aaagggctgc gactccacga caacccggcg 180

ttgctggcgg ccgtgcgcgg ggcgcgctgc gtgcgctgcg tttacattct cgacccgtgg 240

```
ttcgcggcct cctcctcagt cgggatcaac cgatggaggt tcctacttca gtctctggaa      300
gatttggaca caagtttaag gaaactgaac tcccgcctgt ttgtagtccg gggacagcca      360
gccgacgtgt tcccaaggct gttcaaggaa tggggagtga cccgcttgac ctttgaatat      420
gactctgaac cctttgggaa agaacgggat gcagccatca tgaagatggc caaggaggct      480
ggtgtggaag tagtgacgga gaattctcat accctctatg acctggacag gatcattgag      540
ctgaatgggc agaagccacc ccttacatac aagcgctttc aggccatcat cagccgcatg      600
gagctgccca agaagccagt gggcttggtg accagccagc agatggagag ctgcagggcc      660
gagatccagg agaaccacga cgagacctac ggcgtgccct ccctggagga gctggggttc      720
cccactgaag gacttggtcc agctgtctgg cagggaggag agacagaagc tctggcccgc      780
ctggataagc acttggaacg gaaggcctgg gttgccaact atgagagacc ccgaatgaac      840
gccaactccc tcctggccag ccccacaggc ctcagcccct acctgcgctt tggttgtctc      900
tcctgccgcc tcttctacta ccgcctgtgg gacctgtata aaaaggtgaa gcggaacagc      960
acacctcccc tctccctatt tgggcaactc ctatggcgag agttcttcta cacggcagct     1020
accaacaacc ccaggtttga ccgcatggag gggaacccca tctgcatcca gatcccctgg     1080
gaccgcaatc ctgaggccct ggccaagtgg gctgagggca agacaggctt cccttggatt     1140
gatgccatca tgacccaact gaggcaggag ggctggatcc accacctggc ccggcatgcc     1200
gtggcctgct tcctgacccg cggggacctc tgggtcagct gggagagcgg ggtccgggta     1260
tttgatgagc tgctcctgga tgcagatttc agcgtgaacg caggcagctg gatgtggctg     1320
tcctgcagtg ctttcttcca gcagttcttc cactgctact gccctgtggg ctttggccgt     1380
cgcacggacc ccagtgggga ctacatcagg cgatacctgc ccaaattgaa agcgttcccc     1440
tctcgataca tctatgagcc ctggaatgcc ccagagtcaa ttcagaaggc agccaagtgc     1500
atcattggtg tggactaccc acggcccatc gtcaaccatg ccgagaccag ccggcttaac     1560
attgaacgaa tgaagcagat ttaccagcag ctttcgcgct accggggact ctgtctactg     1620
gcatctgtcc cttcctgtgt ggaagacctc agtcaccctg tggcagagcc cagctcgagc     1680
caggctggca gcatgagcag tgcaggccca agaccactac ccagtggccc agcatccccc     1740
aaacgcaagc tggaagcagc cgaggaacca cctggtgaag aactcagcaa acgggcccgg     1800
gtggcagagt tgccaacccc agagctgccg agcaaggatg cctga                     1845
```

```
<210>  10
<211>  1845
<212>  DNA
<213>  Human

<400>  10
atgacgaccc tggactccaa caacaacaca ggtggcgtca tcacctacat tggctccagt      60
```

ggctcctccc caagccgcac cagccctgaa tccctctata gtgacaactc caatggcagc 120
ttccagtccc tgacccaagg ctgtcccacc tacttcccac catcccccac tggctccctc 180
acccaagacc cggctcgctc ctttgggagc attccaccca gcctgagtga tgacggctcc 240
ccttcttcct catcttcctc gtcgtcatcc tcctcctcct tctataatgg gagcccccct 300
gggagtctac aagtggccat ggaggacagc agccgagtgt cccccagcaa gagcaccagc 360
aacatcacca agctgaatgg catggtgtta ctgtgtaaag tgtgtgggga cgttgcctcg 420
ggcttccact acggtgtgca cgcctgcgag ggctgcaagg gcttttttccg tcggagcatc 480
cagcagaaca tccagtacaa aaggtgtctg aagaatgaga attgctccat cgtccgcatc 540
aatcgcaacc gctgccagca atgtcgcttc aagaagtgtc tctctgtggg catgtctcga 600
gacgctgtgc gttttgggcg catccccaaa cgagagaagc agcggatgct tgctgagatg 660
cagagtgcca tgaacctggc caacaaccag ttgagcagcc agtgcccgct ggagacttca 720
cccacccagc accccacccc aggccccatg ggcccctcgc caccccctgc tccggtcccc 780
tcacccctgg tgggcttctc ccagtttcca caacagctga cgcctcccag atccccaagc 840
cctgagccca cagtggagga tgtgatatcc caggtggccc gggcccatcg agagatcttc 900
acctacgccc atgacaagct gggcagctca cctggcaact tcaatgccaa ccatgcatca 960
ggtagccctc cagccaccac cccacatcgc tgggaaaatc agggctgccc acctgccccc 1020
aatgacaaca acaccttggc tgcccagcgt cataacgagg ccctaaatgg tctgcgccag 1080
gctccctcct cctaccctcc cacctggcct cctggccctg cacaccacag ctgccaccag 1140
tccaacagca acgggcaccg tctatgcccc acccacgtgt atgcagcccc agaaggcaag 1200
gcacctgcca acagtccccg gcagggcaac tcaaagaatg ttctgctggc atgtcctatg 1260
aacatgtacc cgcatggacg cagtgggcga acggtgcagg agatctggga ggatttctcc 1320
atgagcttca cgcccgctgt gcgggaggtg gtagagtttg ccaaacacat cccgggcttc 1380
cgtgaccttt ctcagcatga ccaagtcacc ctgcttaagg ctggcacctt tgaggtgctg 1440
atggtgcgct ttgcttcgtt gttcaacgtg aaggaccaga cagtgatgtt cctaagccgc 1500
accacctaca gcctgcagga gcttggtgcc atgggcatgg gagacctgct cagtgccatg 1560
ttcgacttca gcgagaagct caactccctg gcgcttaccg aggaggagct gggcctcttc 1620
accgcggtgg tgcttgtctc tgcagaccgc tcgggcatgg agaattccgc ttcggtggag 1680
cagctccagg agacgctgct gcgggctctt cgggctctgg tgctgaagaa ccggcccttg 1740
gagacttccc gcttcaccaa gctgctgctc aagctgccgg acctgcggac cctgaacaac 1800
atgcattccg agaagctgct gtccttccgg gtggacgccc agtga 1845

<210> 11

<211> 1740
<212> DNA
<213> Human

<400> 11

```
atggaggtga atgcaggagg tgtgattgcc tatatcagtt cttccagctc agcctcaagc      60
cctgcctctt gtcacagtga gggttctgag aatagtttcc agtcctcctc ctcttctgtt     120
ccatcttctc caaatagctc taattctgat accaatggta atcccaagaa tggtgatctc     180
gccaatattg aaggcatctt gaagaatgat cgaatagatt gttctatgaa aacaagcaaa     240
tcgagtgcac ctgggatgac aaaaagtcat agtggtgtga caaaatttag tggcatggtt     300
ctactgtgta aagtctgtgg ggatgtggcg tcaggattcc actatggagt tcatgcttgc     360
gaaggctgta agggtttctt tcggagaagt attcaacaaa acatccagta caagaagtgc     420
ctgaagaatg aaaactgttc tataatgaga atgaatagga acagatgtca gcaatgtcgc     480
ttcaaaaagt gtctgtctgt tggaatgtca agagatgctg ttcggtttgg tcgtattcct     540
aagcgtgaaa aacagaggat gctaattgaa atgcaaagtg caatgaagac catgatgaac     600
agccagttca gtggtcactt gcaaaatgac acattagtag aacatcatga acagacagcc     660
ttgccagccc aggaacagct gcgacccaag ccccaactgg agcaagaaaa catcaaaagc     720
tcttctcctc catcttctga ttttgcaaag gaagaagtga ttggcatggt gaccagagct     780
cacaaggata cctttatgta taatcaagag cagcaagaaa actcagctga gagcatgcag     840
ccccagagag gagaacggat tcccaagaac atggagcaat ataatttaaa tcatgatcat     900
tgcggcaatg ggcttagcag ccattttccc tgtagtgaga gccagcagca tctcaatgga     960
cagttcaaag ggaggaatat aatgcattac ccaaatggtc atgccatttg tattgcaaat    1020
ggacattgta tgaacttctc caatgcttat actcaaagag tatgtgatag agttccgata    1080
gatggatttt ctcagaatga gaacaagaat agttacctgt gcaacactgg aggaagaatg    1140
catctggttt gtccaatgag taagtctcca tatgtggatc ctcataaatc aggacatgaa    1200
atctgggaag aattttcgat gagcttcact ccagcagtga aagaagtggt ggaatttgca    1260
aagcgtattc ctgggttcag agatctctct cagcatgacc aggtcaacct tttaaaggct    1320
gggacttttg aggttttaat ggtacggttc gcatcattat ttgatgcaaa ggaacgtact    1380
gtcacctttt taagtggaaa gaaatatagt gtggatgatt tacactcaat gggagcaggg    1440
gatctgctaa actctatgtt tgaatttagt gagaagctaa atgccctcca acttagtgat    1500
gaagagatga gtttgtttac agctgttgtc ctggtatctg cagatcgatc tggaatagaa    1560
aacgtcaact ctgtggaggc tttgcaggaa actctcattc gtgcactaag gaccttaata    1620
atgaaaaacc atccaaatga ggcctctatt tttacaaaac tgcttctaaa gttgccagat    1680
cttcgatctt taaacaacat gcactctgag gagctcttgg cctttaaagt tcacccttaa    1740
```

<210> 12
<211> 1572
<212> DNA
<213> Human

<400> 12
atggagtcag ctccggcagc cccccgaccc gccgccagcg agccaggcag cagcggcgcg 60

gacgcggccg ccggctccag ggagaccccg ctgaaccagg aatccgcccg caagagcgag 120

ccgcctgccc cggtgcgcag acagagctat tccagcacca gcagaggtat ctcagtaacg 180

aagaagacac atacatctca aattgaaatt attccatgca agatctgtgg agacaaatca 240

tcaggaatcc attatggtgt cattacatgt gaaggctgca agggcttttt caggagaagt 300

cagcaaagca atgccaccta ctcctgtcct cgtcagaaga actgtttgat tgatcgaacc 360

agtagaaacc gctgccaaca ctgtcgatta cagaaatgcc ttgccgtagg gatgtctcga 420

gatgctgtaa aatttggccg aatgtcaaaa aagcagagag acagcttgta tgcagaagta 480

cagaaacacc ggatgcagca gcagcagcgc gaccaccagc agcagcctgg agaggctgag 540

ccgctgacgc ccacctacaa catctcggcc aacgggctga cggaacttca cgacgacctc 600

agtaactaca ttgacgggca cacccctgag gggagtaagg cagactccgc cgtcagcagc 660

ttctacctgg acatacagcc ttccccagac cagtcaggtc ttgatatcaa tggaatcaaa 720

ccagaaccaa tatgtgacta cacaccagca tcaggcttct ttccctactg ttcgttcacc 780

aacggcgaga cttccccaac tgtgtccatg gcagaattag aacaccttgc acagaatata 840

tctaaatcgc atctggaaac ctgccaatac ttgagagaag agctccagca gataacgtgg 900

cagacctttt tacaggaaga aattgagaac tatcaaaaca agcagcggga ggtgatgtgg 960

caattgtgtg ccatcaaaat tacagaagct atacagtatg tggtggagtt tgccaaacgc 1020

attgatggat ttatggaact gtgtcaaaat gatcaaattg tgcttctaaa agcaggttct 1080

ctagaggtgg tgtttatcag aatgtgccgt gcctttgact ctcagaacaa caccgtgtac 1140

tttgatggga agtatgccag ccccgacgtc ttcaaatcct taggttgtga agactttatt 1200

agctttgtgt ttgaatttgg aaagagttta tgttctatgc acctgactga agatgaaatt 1260

gcattatttt ctgcatttgt actgatgtca gcagatcgct catggctgca agaaaaggta 1320

aaaattgaaa aactgcaaca gaaaattcag ctagctcttc aacacgtcct acagaagaat 1380

caccgagaag atggaatact aacaaagtta atatgcaagg tgtctacctt aagagcctta 1440

tgtggacgac atacagaaaa gctaatggca tttaaagcaa tatacccaga cattgtgcga 1500

cttcattttc ctccattata caaggagttg ttcacttcag aatttgagcc agcaatgcaa 1560

attgatgggt aa 1572

<210> 13

<211> 1380
<212> DNA
<213> Human

<400> 13
atgcgagcac aaattgaagt gataccatgc aaaatttgtg gcgataagtc ctctgggatc 60

cactacggag tcatcacatg tgaaggctgc aagggattct ttaggaggag ccagcagaac 120

aatgcttctt attcctgccc aaggcagaga aactgtttaa ttgacagaac gaacagaaac 180

cgttgccaac actgccgact gcagaagtgt cttgccctag gaatgtcaag agatgctgtg 240

aagtttggga ggatgtccaa gaagcaaagg gacagcctgt atgctgaggt gcagaagcac 300

cagcagcggc tgcaggaaca gcggcagcag cagagtgggg aggcagaagc ccttgccagg 360

gtgtacagca gcagcattag caacggcctg agcaacctga acaacgagac cagcggcact 420

tatgccaacg ggcacgtcat tgacctgccc aagtctgagg gttattacaa cgtcgattcc 480

ggtcagccgt cccctgatca gtcaggactt gacatgactg gaatcaaaca gataaagcaa 540

gaacctatct atgacctcac atccgtaccc aacttgttta cctatagctc tttcaacaat 600

gggcagttag caccagggat aaccatgact gaaatcgacc gaattgcaca gaacatcatt 660

aagtcccatt tggagacatg tcaatacacc atggaagagc tgcaccagct ggcgtggcag 720

acccacacct atgaagaaat taaagcatat caaagcaagt ccagggaagc actgtggcaa 780

caatgtgcca tccagatcac tcacgccatc caatacgtgg tggagtttgc aaagcggata 840

acaggcttca tggagctctg tcaaaatgat caaattctac ttctgaagtc aggttgcttg 900

gaagtggttt tagtgagaat gtgccgtgcc ttcaacccat taaacaacac tgttctgttt 960

gaaggaaaat atggaggaat gcaaatgttc aaagccttag gttctgatga cctagtgaat 1020

gaagcatttg actttgcaaa gaatttgtgt tccttgcagc tgaccgagga ggagatcgct 1080

ttgttctcat ctgctgttct gatatctcca gaccgagcct ggcttataga accaaggaaa 1140

gtccagaagc ttcaggaaaa aatttatttt gcacttcaac atgtgattca gaagaatcac 1200

ctggatgatg agaccttggc aaagttaata gccaagatac caaccatcac ggcagtttgc 1260

aacttgcacg ggggagaagct gcaggtattt aagcaatctc atccagagat agtgaataca 1320

ctgtttcctc cgttatacaa ggagctcttt aatcctgact gtgccaccgg ctgcaaatga 1380

<210> 14
<211> 1557
<212> DNA
<213> Human

<400> 14
atggacaggg ccccacagag acagcaccga gcctcacggg agctgctggc tgcaaagaag 60

acccacacct cacaaattga agtgatccct tgcaaaatct gtggggacaa gtcgtctggg 120

atccactacg gggttatcac ctgtgagggg tgcaagggct tcttccgccg gagccagcgc 180

tgtaacgcgg cctactcctg cacccgtcag cagaactgcc ccatcgaccg caccagccga 240

aaccgatgcc agcactgccg cctgcagaaa tgcctggcgc tgggcatgtc ccgagatgct 300

gtcaagttcg gccgcatgtc caagaagcag agggacagcc tgcatgcaga agtgcagaaa 360

cagctgcagc agcggcaaca gcagcaacag gaaccagtgg tcaagacccc tccagcaggg 420

gcccaaggag cagataccct cacctacacc ttggggctcc cagacgggca gctgcccctg 480

ggctcctcgc ctgacctgcc tgaggcttct gcctgtcccc ctggcctcct gaaagcctca 540

ggctctgggc cctcatattc caacaacttg gccaaggcag ggctcaatgg ggcctcatgc 600

caccttgaat acagccctga gcggggcaag gctgagggca gagagagctt ctatagcaca 660

ggcagccagc tgacccctga ccgatgtgga cttcgttttg aggaacacag gcatcctggg 720

cttggggaac tgggacaggg cccagacagc tacggcagcc ccagtttccg cagcacaccg 780

gaggcaccct atgcctccct gacagagata gagcacctgg tgcagagcgt ctgcaagtcc 840

tacagggaga catgccagct gcggctggag gacctgctgc ggcagcgctc caacatcttc 900

tcccgggagg aagtgactgg ctaccagagg aagtccatgt gggagatgtg ggaacggtgt 960

gcccaccacc tcaccgaggc cattcagtac gtggtggagt tcgccaagag gctctcaggc 1020

tttatggagc tctgccagaa tgaccagatt gtgcttctca aagcaggagc aatggaagtg 1080

gtgctggtta ggatgtgccg ggcctacaat gctgacaacc gcacggtctt ttttgaaggc 1140

aaatacggtg gcatggagct gttccgagcc ttgggctgca gcgagctcat cagctccatc 1200

tttgacttct cccactccct aagtgccttg cacttttccg aggatgagat tgccctctac 1260

acagcccttg ttctcatcaa tgcccatcgg ccagggctcc aagagaaaag gaaagtagaa 1320

cagctgcagt acaatctgga gctggccttt catcatcatc tctgcaagac tcatcgccaa 1380

agcatcctgg caaagctgcc acccaagggg aagcttcgga gcctgtgtag ccagcatgtg 1440

gaaaggctgc agatcttcca gcacctccac cccatcgtgg tccaagccgc tttccctcca 1500

ctctacaagg agctcttcag cactgaaacc gagtcacctg tggggctgtc caagtga 1557

<210> 15
<211> 2592
<212> DNA
<213> Human

<400> 15
atggcaaggc tgggaaactg cagcctgact tggctgccc tgatcatcct gctgctcccc 60

ggaagtctgg aggagtgcgg gcacatcagt gtctcagccc ccatcgtcca cctgggggat 120

cccatcacag cctcctgcat catcaagcag aactgcagcc atctggaccc ggagccacag 180

attctgtgga gactgggagc agagcttcag cccgggggca ggcagcagcg tctgtctgat 240

gggacccagg aatctatcat caccctgccc cacctcaacc acactcaggc ctttctctcc 300

tgctgcctga actggggcaa cagcctgcag atcctggacc aggttgagct gcgcgcaggc 360
taccctccag ccatacccca caacctctcc tgcctcatga acctcacaac cagcagcctc 420
atctgccagt gggagccagg acctgagacc cacctaccca ccagcttcac tctgaagagt 480
ttcaagagcc ggggcaactg tcagacccaa ggggactcca tcctggactg cgtgcccaag 540
gacgggcaga gccactgctg catcccacgc aaacacctgc tgttgtacca gaatatgggc 600
atctgggtgc aggcagagaa tgcgctgggg accagcatgt ccccacaact gtgtcttgat 660
cccatggatg ttgtgaaact ggagcccccc atgctgcgga ccatggaccc cagccctgaa 720
gcggcccctc cccaggcagg ctgcctacag ctgtgctggg agccatggca gccaggcctg 780
cacataaatc agaagtgtga gctgcgccac aagccgcagc gtggagaagc cagctgggca 840
ctggtgggcc ccctcccctt ggaggccctt cagtatgagc tctgcgggct cctcccagcc 900
acggcctaca ccctgcagat acgctgcatc cgctggcccc tgcctggcca ctggagcgac 960
tggagcccca gcctggagct gagaactacc gaacgggccc ccactgtcag actggacaca 1020
tggtggcggc agaggcagct ggaccccagg acagtgcagc tgttctggaa gccagtgccc 1080
ctggaggaag acagcggacg gatccaaggt tatgtggttt cttggagacc ctcaggccag 1140
gctggggcca tcctgcccct ctgcaacacc acagagctca gctgcacctt ccacctgcct 1200
tcagaagccc aggaggtggc ccttgtggcc tataactcag ccgggacctc tcgtcccact 1260
ccggtggtct tctcagaaag cagaggccca gctctgacca gactccatgc catggcccga 1320
gaccctcaca gcctctgggt aggctgggag cccccccaatc catggcctca gggctatgtg 1380
attgagtggg gcctgggccc ccccagcgcg agcaatagca acaagacctg gaggatggaa 1440
cagaatggga gagccacggg gtttctgctg aaggagaaca tcaggccctt tcagctctat 1500
gagatcatcg tgactcccctt gtaccaggac accatgggac cctcccagca tgtctatgcc 1560
tactctcaag aaatggctcc ctcccatgcc ccagagctgc atctaaagca cattggcaag 1620
acctgggcac agctggagtg ggtgcctgag ccccctgagc tggggaagag ccccccttacc 1680
cactacacca tcttctggac caacgctcag aaccagtcct tctccgccat cctgaatgcc 1740
tcctcccgtg gctttgtcct ccatggcctg gagcccgcca gtctgtatca catccacctc 1800
atggctgcca gccaggctgg ggccaccaac agtacagtcc tcaccctgat gaccttgacc 1860
ccagaggggt cggagctaca catcatcctg ggcctgttcg gcctcctgct gttgctcacc 1920
tgcctctgtg gaactgcctg gctctgttgc agccccaaca ggaagaatcc cctctggcca 1980
agtgtcccag acccagctca cagcagcctg ggctcctggg tgcccacaat catggaggag 2040
ctgcccggac ccagacaggg acagtggctg gggcagacat ctgaaatgag ccgtgctctc 2100
accccacatc cttgtgtgca ggatgccttc cagctgcccg gccttggcac gccacccatc 2160

accaagctca cagtgctgga ggaggatgaa aagaagccgg tgccctggga gtcccataac 2220

agctcagaga cctgtggcct ccccactctg gtccagacct atgtgctcca gggggaccca 2280

agagcagttt ccacccagcc ccaatcccag tctggcacca gcgatcaggt cctttatggg 2340

cagctgctgg gcagccccac aagcccaggg ccagggcact atctccgctg tgactccact 2400

cagcccctct tggcgggcct cacccccagc cccaagtcct atgagaacct ctggttccag 2460

gccagcccct tggggaccct ggtaacccca gccccaagcc aggaggacga ctgtgtcttt 2520

gggccactgc tcaacttccc cctcctgcag gggatccggg tccatgggat ggaggcgctg 2580

gggagcttct ag 2592

<210> 16
<211> 933
<212> DNA
<213> Human

<400> 16
atgggaatcc tgtcattctt acctgtcctt gccactgaga gtgactgggc tgactgcaag 60

tccccccagc cttggggtca tatgcttctg tggacagctg tgctattcct ggctcctgtt 120

gctgggacac ctgcagctcc cccaaaggct gtgctgaaac tcgagcccca gtggatcaac 180

gtgctccagg aggactctgt gactctgaca tgccggggga ctcacagccc tgagagcgac 240

tccattcagt ggttccacaa tgggaatctc attcccaccc acacgcagcc cagctacagg 300

ttcaaggcca acaacaatga cagcggggag tacacgtgcc agactggcca gaccagcctc 360

agcgaccctg tgcatctgac tgtgctttct gagtggctgg tgctccagac ccctcacctg 420

gagttccagg agggagaaac catcgtgctg aggtgccaca gctggaagga caagcctctg 480

gtcaaggtca cattcttcca gaatggaaaa tccaagaaat tttcccgttc ggatcccaac 540

ttctccatcc cacaagcaaa ccacagtcac agtggtgatt accactgcac aggaaacata 600

ggctacacgc tgtactcatc caagcctgtg accatcactg tccaagctcc cagctcttca 660

ccgatgggga tcattgtggc tgtggtcact gggattgctg tagcggccat tgttgctgct 720

gtagtggcct tgatctactg caggaaaaag cggatttcag ctctcccagg ataccctgag 780

tgcagggaaa tgggagagac cctccctgag aaaccagcca atcccactaa tcctgatgag 840

gctgacaaag ttggggctga gaacacaatc acctattcac ttctcatgca cccggatgct 900

ctggaagagc ctgatgacca gaaccgtatt tag 933

<210> 17
<211> 894
<212> DNA
<213> Human

<400> 17
atgacaacac ccagaaattc agtaaatggg actttcccgg cagagccaat gaaaggccct 60

attgctatgc aatctggtcc aaaaccactc ttcaggagga tgtcttcact ggtgggcccc 120

acgcaaagct tcttcatgag ggaatctaag actttggggg ctgtccagat tatgaatggg 180

ctcttccaca ttgccctggg gggtcttctg atgatcccag cagggatcta tgcacccatc 240

tgtgtgactg tgtggtaccc tctctgggga ggcattatgt atattatttc cggatcactc 300

ctggcagcaa cggagaaaaa ctccaggaag tgtttggtca aaggaaaaat gataatgaat 360

tcattgagcc tctttgctgc catttctgga atgattcttt caatcatgga catacttaat 420

attaaaattt cccatttttt aaaaatggag agtctgaatt ttattagagc tcacacacca 480

tatattaaca tatacaactg tgaaccagct aatccctctg agaaaaactc cccatctacc 540

caatactgtt acagcataca atctctgttc ttgggcattt tgtcagtgat gctgatcttt 600

gccttcttcc aggaacttgt aatagctggc atcgttgaga atgaatggaa aagaacgtgc 660

tccagaccca aatctaacat agttctcctg tcagcagaag aaaaaaaaga acagactatt 720

gaaataaaag aagaagtggt tgggctaact gaaacatctt cccaaccaaa gaatgaagaa 780

gacattgaaa ttattccaat ccaagaagag gaagaagaag aaacagagac gaactttcca 840

gaacctcccc aagatcagga atcctcacca atagaaaatg acagctctcc ttaa 894

<210> 18
<211> 702
<212> DNA
<213> Human

<400> 18
atgtggcagc tgctcctccc aactgctctg ctacttctag tttcagctgg catgcggact 60

gaagatctcc caaaggctgt ggtgttcctg gagcctcaat ggtacagcgt gcttgagaag 120

gacagtgtga ctctgaagtg ccagggagcc tactcccctg aggacaattc cacacagtgg 180

tttcacaatg agaacctcat ctcaagccag gcctcgagct acttcattga cgctgccaca 240

gtcaacgaca gtggagagta caggtgccag acaaacctct ccaccctcag tgacccggtg 300

cagctagaag tccatatcgg ctggctgttg ctccaggccc ctcggtgggt gttcaaggag 360

gaagacccta ttcacctgag gtgtcacagc tggaagaaca ctgctctgca taaggtcaca 420

tatttacaga atggcaaaga caggaagtat tttcatcata attctgactt ccacattcca 480

aaagccacac tcaaagatag cggctcctac ttctgcaggg ggcttgttgg gagtaaaaat 540

gtgtcttcag agactgtgaa catcaccatc actcaaggtt tggcagtgtc aaccatctca 600

tcattctctc cacctgggta ccaagtctct ttctgcttgg tgatggtact ccttttgca 660

gtggacacag gactatattt ctctgtgaag acaaacattt ga 702

<210> 19
<211> 2298

<212> DNA
<213> Human

<400> 19
atgagtgggg accacctcca caacgattcc cagatcgaag cggatttccg attgaatgat 60
tctcataaac acaaagataa acacaaagat cgagaacacc ggcacaaaga acacaagaag 120
gagaaggacc gggaaaagtc caagcatagc aacagtgaac ataaagattc tgaaaagaaa 180
cacaaagaga aggagaagac caaacacaaa gatggaagct cagaaaagca taaagacaaa 240
cataaagaca gagacaagga aaaacgaaaa gaggaaaagg ttcgagcctc tggggatgca 300
aaaataaaga aggagaagga aaatggcttc tctagtccac cacaaattaa agatgaacct 360
gaagatgatg gctattttgt tcctcctaaa gaggatataa agccattaaa gagacctcga 420
gatgaggatg atgctgatta taaacctaag aaaattaaaa cagaagatac caagaaggag 480
aagaaaagaa aactagaaga agaagaggat ggtaaattga aaaaacccaa gaataaagat 540
aaagataaaa aagttcctga gccagataac aagaaaaaga agccgaagaa agaagaggaa 600
cagaagtgga aatggtggga agaagagcgc tatcctgaag gcatcaagtg gaaattccta 660
gaacataaag gtccagtatt tgccccacca tatgagcctc ttccagagaa tgtcaagttt 720
tattatgatg gtaaagtcat gaagctgagc cccaaagcag aggaagtagc tacgttcttt 780
gcaaaaatgc tcgaccatga atatactacc aaggaaatat ttaggaaaaa tttctttaaa 840
gactggagaa aggaaatgac taatgaagag aagaatatta tcaccaacct aagcaaatgt 900
gattttaccc agatgagcca gtatttcaaa gcccagacgg aagctcggaa acagatgagc 960
aaggaagaga aactgaaaat caaagaggag aatgaaaaat tactgaaaga atatggattc 1020
tgtattatgg ataaccacaa agagaggatt gctaacttca agatagagcc tcctggactt 1080
ttccgtggcc gcggcaacca ccccaagatg ggcatgctga agagacgaat catgcccgag 1140
gatataatca tcaactgtag caaagatgcc aaggttcctt ctcctcctcc aggacataag 1200
tggaaagaag tccggcatga taacaaggtt acttggctgg tttcctggac agagaacatc 1260
caaggttcca ttaaatacat catgcttaac cctagttcac gaatcaaggg tgagaaggac 1320
tggcagaaat acgagactgc tcggcggctg aaaaaatgtg tggacaagat ccggaaccag 1380
tatcgagaag actggaagtc caaagagatg aaagtccggc agagagctgt agccctgtac 1440
ttcatcgaca agcttgctct gagagcaggc aatgaaaagg aggaaggaga aacagcggac 1500
actgtgggct gctgctcact tcgtgtggag cacatcaatc tacacccaga gttggatggt 1560
caggaatatg tggtagagtt tgacttcctc gggaaggact ccatcagata ctataacaag 1620
gtccctgttg agaaacgagt ttttaagaac ctacaactat ttatggagaa caagcagccc 1680
gaggatgatc tttttgatag actcaatact ggtattctga ataagcatct tcaggatctc 1740
atggagggct tgacagccaa ggtattccgt acatacaatg cctccatcac gctacagcag 1800

```
cagctaaaag aactgacagc cccggatgag aacatcccag cgaagatcct ttcttataac      1860
cgtgccaatc gagctgttgc aattctttgt aaccatcaga gggcaccacc aaaaactttt      1920
gagaagtcta tgatgaactt gcaaactaag attgatgcca agaaggaaca gctagcagat      1980
gcccggagag acctgaaaag tgctaaggct gatgccaagg tcatgaagga tgcaaagacg      2040
aagaaggtag tagagtcaaa gaagaaggct gttcagagac tggaggaaca gttgatgaag      2100
ctggaagttc aagccacaga ccgagaggaa aataaacaga ttgccctggg aacctccaaa      2160
ctcaattatc tggaccctag gatcacagtg gcttggtgca agaagtgggg tgtcccaatt      2220
gagaagattt acaacaaaac ccagcgggag aagtttgcct gggccattga catggctgat      2280
gaagactatg agttttag                                                    2298
```

## Description

Subject matter of the invention is a method of assessing the circadian rhythm of a subject having cancer and/or assessing a timing of administration of a medicament to said subject having cancer, wherein said method comprises the steps of:

- Providing at least three samples of saliva, more preferably four samples of saliva, from said subject, wherein said samples have been taken at different time points over the day;
- Determining gene expression the following genes in each of said samples:

  - ◦ of at least two members of genes for the core-clock network, in particular of at least two members of the group comprising ARNTL (BMAL1), ARNTL2, CLOCK, PER1, PER2, PER3, NPAS2, CRY1, CRY2, NR1D1, NR1D2, RORA, RORB, ROBC in particular ARNTL (BMAL1) and PER2, and
  - ◦ at least one gene involved in the metabolism of a medicament to be administered to said subject having cancer, including Ces2, and
  - ◦ at least one drug target gene that is a target of the medicament to be administered, and

- Assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

## State of the art

The biological clock (also known as circadian clock) regulates several aspects of physiology and behavior via cellular and molecular mechanisms and plays a vital role in maintaining proper human health. This is no wonder, since about half of all human genes are rhythmically expressed in at least one tissue. The disruption of circadian rhythms is associated to several diseases including sleep disorders, depression, diabetes, Alzheimer's disease, obesity and cancer. This disruption might result from conflicting external (environmental) or internal (feeding/resting) signals that are not in synchrony with the internal biological time. This not only affects shift workers, but also most people subjected to societal routine (social jet lag). Furthermore, a desynchronized circadian clock was shown to negatively affect an individual's wellbeing, in particular in the context of metabolism, as well as physical and mental (cognitive) performance.

Recent studies, including work from our research group have reported a role for clock dysregulation in the pathologies mentioned above, raising increased awareness from scientists, clinicians and the public. Thus, it is crucial to characterize the individual's internal clock and to adjust the external/internal factors, to avoid or to overcome circadian rhythm disruption. Moreover, the time for certain activities, such as sleep, sports or medicine intake, can be optimized based on the individual's internal timing. A proper functioning circadian clock, synchronized with the individual's behavioral habits, will improve fitness and reduce therapy/recovery time in patients.

Yet, the available methods for clock assessment are either not very accurate or mostly invasive and often require medical assistance over a period of several hours (tedious, time-consuming, and cost-intensive). So far, there are the following methods among the molecular approaches to determine the biological clock in humans:

1. Determination of time-point at which endogenous melatonin (or cortisol), reaches a predefined threshold value concentration (dim-light-melatonin-onset, aka DLMO). To do this, several blood or saliva samples (usually every 0.5-1 hour) under dim-light conditions are taken to determine the internal clock.

2. Blood samples (with one or more sampling times) are also used to identify biomarkers using a machine learning approach that could determine the expression phase of "time-indicating" genes and thus the internal clock timing.

3. In addition, the circadian phase (peak time of secretion/expression) in hair samples (hair follicle cells) or urine samples could reflect that of the individual behavioral rhythm. This strategy is more suitable for assessing the human peripheral clock.

As mentioned above, current methods of clock assessment are either invasive, require medical supervision, are time-consuming or do not provide detailed information on the gene expression level. Saliva plays numerous protective roles for oral tissue maintenance in humans. Adequate salivary flow and saliva content are directly related to health status. Previous studies have shown the potential of saliva and salivary transcriptome as a diagnostic tool, which underlines the importance of saliva sampling as a non-invasive diagnostic method. Time-course saliva sampling is also commonly used for estimating the evening dim-light melatonin onset (DLMO) in humans in order to determine their circadian phase (peak time of secretion/expression); a method that requires controlled dim-light conditions during the entire sampling time of 5-6h.

The circadian rhythm was previously modeled with different approaches, starting with models that simply show oscillations such as phase-oscillators, and going up to molecular models, which model (part of) the molecular interactions underlying the circadian clock. In the present disclosure it is focused on molecular models, because these contain biological information that might be useful for predictions. Molecular models with simple feedback loops are often based on Goodwin's oscillator, e.g. (Ruoff and Rensing 1996), but the level of detail may also be extensive (Forger and Peskin 2003).

An objective is to provide a model at an intermediate state of complexity, complex enough to capture a significant part of the genetic network, but if the model is too complex, we cannot fit our data to the model without significant overfitting. Relogio et al. have published a model at this level of complexity, with 19 dynamical variables, which we use in the following (Relogio et al. 2011).

Other studies with mammalian clock models are named below, including respective literal citations from the respective papers.

(Becker-Weimann et al. 2004): "We present a mathematical model that reflects the essential features of the mammalian circadian oscillator to characterize the differential roles of negative and positive feedback loops. The oscillations that are obtained have a 24-h period and are robust toward parameter variations even when the positive feedback is replaced by a constantly expressed activator. This demonstrates the crucial role of the negative feedback for rhythm generation." [7 dynamical variables]

(Forger and Peskin 2003): "Here we develop a detailed distinctly mammalian model by using mass action kinetics. Parameters for our model are found from experimental data by using a coordinate search method. The model accurately predicts the phase of entrainment, amplitude of oscillation, and shape of time profiles of clock mRNAs and proteins and is also robust to parameter changes and mutations." [74 dynamical variables] There also is a stochastic version of this model (Forger and Peskin 2005).

(Leloup and Goldbeter 2003): "We present a computational model for the mammalian circadian clock based on the intertwined positive and negative regulatory loops involving the Per, Cry, Bmall, Clock, and Rev-Erb $\alpha$ genes. In agreement with experimental observations, the model can give rise to sustained circadian oscillations in continuous darkness, characterized by an antiphase relationship between Per/Cry/Rev-Erb$\alpha$ and Bmall mRNAs. Sustained oscillations correspond to the rhythms autonomously generated by suprachiasmatic nuclei. For other parameter values, damped oscillations can also be obtained in the model. These oscillations, which transform into sustained oscillations when coupled to a periodic signal, correspond to rhythms produced by peripheral tissues." [19 dynamical variables] Bifurcation analysis of this model published in (Leloup and Goldbeter 2004).

(Mirsky et al. 2009): "In this study, we built a mathematical model from the regulatory structure of the intracellular circadian clock in mice and identified its parameters using an iterative evolutionary strategy, with minimum cost achieved through conformance to phase separations seen in cell-autonomous oscillators. The model was evaluated against the experimentally observed cell-autonomous circadian phenotypes of gene knockouts, particularly retention of rhythmicity and changes in expression level of molecular clock components." "Most importantly, our model addresses the overlapping but differential functions of CRY1 and CRY2 in the clock mechanism: They antagonistically regulate period length and differentially control rhythm persistence and amplitude" [21 dynamical variables] This model focuses on the phase relationship between different genes.

(Kim and Forger 2012): "To understand the biochemical mechanisms of this timekeeping, we have developed a detailed mathematical model of the mammalian circadian clock. Our model can accurately predict diverse experimental data including the phenotypes of mutations or knockdown of clock genes as well as the time courses and relative expression of clock transcripts and proteins. Using this model, we show how a universal motif of circadian timekeeping, where repressors tightly bind activators rather than directly binding to DNA, can generate oscillations when activators and repressors are in stoichiometric balance." [Ref: Kim, Jae Kyoung, and Daniel B Forger. 2012. "A Mechanism for Robust Circadian Timekeeping via Stoichiometric Balance." Molecular Systems Biology 8 (December): 630. https://doi.org/10.1038/msb.2012.62.]

(Jolley et al. 2014): Focus on a new mechanism via D-box, and modern parameter estimation.

Besides these models of the mammalian circadian clock, various models have been published for non-mammalian systems, and intensively investigated, e.g. by bifurcation analysis.

## Summary of the invention

Subject matter of the invention is a method of assessing the circadian rhythm of a subject having cancer and/or assessing a timing of administration of a medicament to said subject having cancer, wherein said method comprises the steps of:

- Providing at least three samples of saliva, more preferably four samples of saliva, from said subject, wherein said samples have been taken at different time points over the day;
- Determining gene expression of genes for the core-clock network, in particular of at least two members of the following genes, in each of said samples:

  a. of at least two members of the groups comprising ARNTL (BMAL1), ARNTL2, CLOCK, PER1, PER2, PER3, NPAS2, CRY1, CRY2, NR1D1, NR1D2, RORA, RORB, ROBC in particular ARNTL (BMAL1) and PER2, and
  b. at least one gene involved in the metabolism of a medicament to be administered to said subject having cancer, including Ces2, and
  c. at least one drug target gene that is a target of the medicament to be administered, and

- Assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

Genes for the core-clock network comprise the following genes but are not limited to these: *Arntl (Bmal1), Arntl2, Clock, Per1, Per2, Per3, Npas2, Cry1, Cry2, Nr1ld1, Nr1d2, Rora, Rorb and Robc.*
Subject matter of the invention is a method of assessing the circadian rhythm of a subject having cancer and/or assessing a timing of administration of a medicament to said subject having cancer, wherein said method comprises the steps of:

- Providing at least three samples of saliva, more preferably four samples of saliva, from said subject, wherein said samples have been taken at different time points over the day;
- Determining gene expression of at least the following genes in each of said samples:

  ◦ ARNTL (BMAL1), ARNTL2, CLOCK, PER1, PER2, PER3, NPAS2 , CRY1, CRY2, NR1D1, NR1D2, RORA, RORB, ROBC in particular ARNTL (BMAL1) and PER2, and
  ◦ at least one gene involved in the metabolism of a medicament to be administered to said subject having cancer, including Ces2, and
  ◦ at least one drug target gene that is a target of the medicament to be administered, and

- Assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

In one embodiment of the method according to the present invention gene expression is determined using a method selected from quantitative PCR (RT-qPCR), NanoString, sequencing and microarray. Any other method for determining gene expression may be used.
In one embodiment of the method according to the present invention gene expression is determined using quantitative PCR (RT-qPCR). https://pubmed.ncbi.nlm.nih.gov/15956331
In one embodiment of the method according to the present invention gene expression is determined using NanoString. Geiss G, et al., Direct multiplexed measurement of gene expression with color-coded probe pairs, 26: 317-25 (2008)., Nature Biotechnology, Feb 08, 2008.
BMALlis also known as ARNTL, Aryl hydrocarbon receptor nuclear translocator-like protein 1 (ARNTL) Or Brain and Muscle ARNT-Like 1 (BMAL1).

The sequence of cDNA ARNTL (BMAL1) comprises SEQ ID No.1:

>ENST00000389707.8 ARNTL-201 cdna:protein_coding

ATGGCAGACCAGAGAATGGACATTTCTTCAACCATCAGTGATTTCATGTCCCCGGGCCCC
ACCGACCTGCTTTCCAGCTCTCTTGGTACCAGTGGTGTGGATTGCAACCGCAAACGGAAA
GGCAGCTCCACTGACTACCAAGAAAGCATGGACACAGACAAAGATGACCCTCATGGAAGG
TTAGAATATACAGAACACCAAGGAAGGATAAAAAATGCAAGGGAAGCTCACAGTCAGATT
GAAAAGCGGCGTCGGGATAAAATGAACAGTTTTATAGATGAATTGGCTTCTTTGGTACCA
ACATGCAACGCAATGTCCAGGAAATTAGATAAACTTACTGTGCTAAGGATGGCTGTTCAG
CACATGAAAACATTAAGAGGTGCCACCAATCCATACACAGAAGCAAACTACAAACCAACT
TTTCTATCAGACGATGAATTGAAACACCTCATTCTCAGGGCAGCAGATGGATTTTTGTTT
GTCGTAGGATGTGACCGAGGGAAGATACTCTTTGTCTCAGAGTCTGTCTTCAAGATCCTC
AACTACAGCCAGAATGATCTGATTGGTCAGAGTTTGTTTGACTACCTGCATCCTAAAGAT
ATTGCCAAAGTCAAGGAGCAGCTCTCCTCCTCTGACACCGCACCCCGGGAGCGGCTCATA
GATGCAAAAACTGGACTTCCAGTTAAAACAGATATAACCCCTGGGCCATCTCGATTATGT
TCTGGAGCACGACGTTCTTTCTTCTGTAGGATGAAGTGTAACAGGCCTTCAGTAAAGGTT
GAAGACAAGGACTTCCCCTCTACCTGCTCAAAGAAAAAAGATCGAAAAAGCTTCTGCACA
ATCCACAGCACAGGCTATTTGAAAAGCTGGCCACCCACAAAGATGGGGCTGGATGAAGAC
AACGAACCAGACAATGAGGGGTGTAACCTCAGCTGCCTCGTCGCAATTGGACGACTGCAT
TCTCATGTAGTTCCACAACCAGTGAACGGGGAAATCAGGGTGAAATCTATGGAATATGTT
TCTCGGCACGCGATAGATGGAAAGTTTGTTTTTGTAGACCAGAGGGCAACAGCTATTTTG
GCATATTTACCACAAGAACTTCTAGGCACATCGTGTTATGAATATTTTCACCAAGATGAC
ATAGGACATCTTGCAGAATGTCATAGGCAAGTTTTACAGACGAGAGAAAAAATTACAACT
AATTGCTATAAATTTAAAATCAAAGATGGTTCTTTTATCACACTACGGAGTCGATGGTTC
AGTTTCATGAACCCTTGGACCAAGGAAGTAGAATATATTGTCTCAACTAACACTGTTGTT
TTAGCCAACGTCCTGGAAGGCGGGGACCCAACCTTCCCACAGCTCACAGCATCCCCCCAC
AGCATGGACAGCATGCTGCCCTCTGGAGAAGGTGGCCCAAAGAGGACCCACCCCACTGTT
CCAGGGATTCCAGGGGGAACCCGGGCTGGGGCAGGAAAAATAGGCCGAATGATTGCTGAG
GAAATCATGGAAATCCACAGGATAAGAGGGTCATCGCCTTCTAGCTGTGGCTCCAGCCCA
TTGAACATCACGAGTACGCCTCCCCCTGATGCCTCTTCTCCAGGAGGCAAGAAGATTTTA
AATGGAGGGACTCCAGACATTCCTTCCAGTGGCCTACTATCAGGCCAGGCTCAGGAGAAC
CCAGGTTATCCATATTCTGATAGTTCTTCTATTCTTGGTGAGAACCCCCACATAGGTATA
GACATGATTGACAACGACCAAGGATCAAGTAGTCCCAGTAATGATGAGGCAGCAATGGCT
GTCATCATGAGCCTCTTGGAAGCAGATGCTGGACTGGGTGGCCCTGTTGACTTTAGTGAC
TTGCCATGGCCGCTGTAA

The sequence of cDNA ARNTL2 comprises SEQ ID No.2

>ENST00000266503.9 ARNTL2-202 cdna:protein_coding

ATGGCGGCGGAAGAGGAGGCTGCGGCGGGAGGTAAAGTGTTGAGAGAGGAGAACCAGTGC
ATTGCTCCTGTGGTTTCCAGCCGCGTGAGTCCAGGGACAAGACCAACAGCTATGGGGTCT
TTCAGCTCACACATGACAGAGTTTCCACGAAAACGCAAAGGAAGTGATTCAGACCCATCC
CAGTCAGGAATCATGACAGAAAAAGTGGTGGAAAAGCTTTCTCAGAATCCCCTTACCTAT

CTTCTTTCAACAAGGATAGAAATATCAGCCTCCAGTGGCAGCAGAGTGGAAGATGGTGAA
CACCAAGTTAAAATGAAGGCCTTCAGAGAAGCTCATAGCCAAACTGAAAAGCGGAGGAGA
GATAAAATGAATAACCTGATTGAAGAACTGTCTGCAATGATCCCTCAGTGCAACCCCATG
GCGCGTAAACTGGACAAACTTACAGTTTTAAGAATGGCTGTTCAACACTTGAGATCTTTA
AAAGGCTTGACAAATTCTTATGTGGGAAGTAATTATAGACCATCATTTCTTCAGGATAAT
GAGCTCAGACATTTAATCCTTAAGACTGCAGAAGGCTTCTTATTTGTGGTTGGATGTGAA
AGAGGAAAAATTCTCTTCGTTTCTAAGTCAGTCTCCAAAATACTTAATTATGATCAGGCT
AGTTTGACTGGACAAAGCTTATTTGACTTCTTACATCCAAAAGATGTTGCCAAAGTAAAG
GAACAACTTTCTTCTTTTGATATTTCACCAAGAGAAAAGCTAATAGATGCCAAAACTGGT
TTGCAAGTTCACAGTAATCTCCACGCTGGAAGGACACGTGTGTATTCTGGCTCAAGACGA
TCTTTTTTCTGTCGGATAAAGAGTTGTAAAATCTCTGTCAAAGAAGAGCATGGATGCTTA
CCCAACTCAAAGAAGAAAGAGCACAGAAAATTCTATACTATCCATTGCACTGGTTACTTG
AGAAGCTGGCCTCCAAATATTGTTGGAATGGAAGAAGAAAGGAACAGTAAGAAAGACAAC
AGTAATTTTACCTGCCTTGTGGCCATTGGAAGATTACAGCCATATATTGTTCCACAGAAC
AGTGGAGAGATTAATGTGAAACCAACTGAATTTATAACCCGGTTTGCAGTGAATGGAAAA
TTTGTCTATGTAGATCAAAGGGCAACAGCGATTTTAGGATATCTGCCTCAGGAACTTTTG
GGAACTTCTTGTTATGAATATTTTCATCAAGATGACCACAATAATTTGACTGACAAGCAC
AAAGCAGTTCTACAGAGTAAGGAGAAAATACTTACAGATTCCTACAAATTCAGAGCAAAA
GATGGCTCTTTTGTAACTTTAAAAAGCCAATGGTTTAGTTTCACAAATCCTTGGACAAAA
GAACTGGAATATATTGTATCTGTCAACACTTTAGTTTTGGGACATAGTGAGCCTGGAGAA
GCATCATTTTTACCTTGTAGCTCTCAATCATCAGAAGAATCCTCTAGACAGTCCTGTATG
AGTGTACCTGGAATGTCTACTGGAACAGTACTTGGTGCTGGTAGTATTGGAACAGATATT
GCAAATGAAATTCTGGATTTACAGAGGTTACAGTCTTCTTCATACCTTGATGATTCGAGT
CCAACAGGTTTAATGAAAGATACTCATACTGTAAACTGCAGGAGTATGTCAAATAAGGAG
TTGTTTCCACCAAGTCCTTCTGAAATGGGGGAGCTAGAGGCTACCAGGCAAAACCAGAGT
ACTGTTGCTGTCCACAGCCATGAGCCACTCCTCAGTGATGGTGCACAGTTGGATTTCGAT
GCCCTATGTGACAATGATGACACAGCCATGGCTGCATTTATGAATTACTTAGAAGCAGAG
GGGGGCCTGGGAGACCCTGGGGACTTCAGTGACATCCAGTGGACCCTCTAG

The sequence of cDNA PER1 comprises SEQ ID No.3

>ENST00000317276.9 PER1-201 cdna:protein_coding

ATGAGTGGCCCCCTAGAAGGGGCTGATGGGGGAGGGGACCCCAGGCCTGGGGAATCATTT
TGTCCTGGGGGCGTCCCATCCCCTGGGCCCCCACAGCACCGGCCTTGCCCAGGCCCCAGC
CTGGCCGATGACACCGATGCCAACAGCAATGGTTCAAGTGGCAATGAGTCCAACGGGCAT
GAGTCTAGAGGCGCATCTCAGCGGAGCTCACACAGCTCCTCCTCAGGCAACGGCAAGGAC
TCAGCCCTGCTGGAGACCACTGAGAGCAGCAAGAGCACAAACTCTCAGAGCCCATCCCCA
CCCAGCAGTTCCATTGCCTACAGCCTCCTGAGTGCCAGCTCAGAGCAGGACAACCCGTCC
ACCAGTGGCTGCAGCAGTGAACAGTCAGCCCGGGCAAGGACTCAGAAGGAACTCATGACA
GCACTTCGAGAGCTCAAGCTTCGACTGCCGCCAGAGCGCCGGGGCAAGGGCCGCTCTGGG
ACCCTGGCCACGCTGCAGTACGCACTGGCCTGTGTCAAGCAGGTGCAGGCCAACCAGGAA
TACTACCAGCAGTGGAGCCTGGAGGAGGGCGAGCCTTGCTCCATGGACATGTCCACCTAT
ACCCTGGAGGAGCTGGAGCACATCACGTCTGAGTACACACTTCAGAACCAGGATACCTTC
TCAGTGGCTGTCTCCTTCCTGACGGGCCGAATCGTCTACATTTCGGAGCAGGCAGCCGTC

CTGCTGCGTTGCAAGCGGGACGTGTTCCGGGGTACCCGCTTCTCTGAGCTCCTGGCTCCC
CAGGATGTGGGAGTCTTCTATGGTTCCACTGCTCCATCTCGCCTGCCCACCTGGGGCACA
GGGGCCTCAGCAGGTTCAGGCCTCAGGGACTTTACCCAGGAGAAGTCCGTCTTCTGCCGT
ATCAGAGGAGGTCCTGACCGGGATCCAGGGCCTCGGTACCAGCCATTCCGCCTAACCCCG
TATGTGACCAAGATCCGGGTCTCAGATGGGGCCCCTGCACAGCCGTGCTGCCTGCTGATT
GCAGAGCGCATCCATTCGGGTTACGAAGCTCCCCGGATACCCCCTGACAAGAGGATTTTC
ACTACGCGGCACACACCCAGCTGCCTCTTCCAGGATGTGGATGAAAGGGCTGCCCCCCTG
CTGGGCTACCTGCCCCAGGACCTCCTGGGGGCCCCAGTGCTCCTGTTCCTGCATCCTGAG
GACCGACCCCTCATGCTGGCTATCCACAAGAAGATTCTGCAGTTGGCGGGCCAGCCCTTT
GACCACTCCCCTATCCGCTTCTGTGCCCGCAACGGGGAGTATGTCACCATGGACACCAGC
TGGGCTGGCTTTGTGCACCCCTGGAGCCGCAAGGTAGCCTTCGTGTTGGGCCGCCACAAA
GTACGCACGGCCCCCCTGAATGAGGACGTGTTCACTCCCCCGGCCCCCAGCCCAGCTCCC
TCCCTGGACACTGATATCCAGGAGCTGTCAGAGCAGATCCACCGGCTGCTGCTGCAGCCC
GTCCACAGCCCCAGCCCCACGGGACTCTGTGGAGTCGGCGCCGTGACATCCCCAGGCCCT
CTCCACAGCCCTGGGTCCTCCAGTGATAGCAACGGGGGTGATGCAGAGGGGCCTGGGCCT
CCTGCGCCAGTGACTTTCCAGCAGATCTGTAAGGATGTGCATCTGGTGAAGCACCAGGGC
CAGCAGCTTTTTATTGAGTCTCGGGCCCGGCCTCAGTCCCGGCCCCGCCTCCCTGCTACA
GGCACGTTCAAGGCCAAGGCCCTTCCCTGCCAATCCCCAGACCCAGAGCTGGAGGCGGGT
TCTGCTCCCGTCCAGGCCCCACTAGCCTTGGTCCCTGAGGAGGCCGAGAGGAAAGAAGCC
TCCAGCTGCTCCTACCAGCAGATCAACTGCCTGGACAGCATCCTCAGGTACCTGGAGAGC
TGCAACCTCCCCAGCACCACTAAGCGTAAATGTGCCTCCTCCTCCTCCTATACCACCTCC
TCAGCCTCTGACGACGACAGGCAGAGGACAGGTCCAGTCTCTGTGGGGACCAAGAAAGAT
CCGCCGTCAGCAGCGCTGTCTGGGGAGGGGGCCACCCCACGGAAGGAGCCAGTGGTGGGA
GGCACCCTGAGCCCGCTCGCCCTGGCCAATAAGGCGGAGAGTGTGGTGTCCGTCACCAGT
CAGTGTAGCTTCAGCTCCACCATCGTCCATGTGGGAGACAAGAAGCCCCCGGAGTCGGAC
ATCATCATGATGGAGGACCTGCCTGGCCTAGCCCCAGGCCCAGCCCCCAGCCCAGCCCCC
AGCCCCACAGTAGCCCCTGACCCAGCCCCAGACGCCTACCGTCCAGTGGGGCTGACCAAG
GCCGTGCTGTCCCTGCACACACAGAAGGAAGAGCAAGCCTTCCTCAGCCGCTTCCGAGAC
CTGGGCAGGCTGCGTGGACTCGACAGCTCTTCCACAGCTCCCTCAGCCCTTGGCGAGCGA
GGCTGCCACCACGGCCCCGCACCCCCAAGCCGCCGACACCACTGCCGATCCAAAGCCAAG
CGCTCACGCCACCACCAGAACCCTCGGGCTGAAGCGCCCTGCTATGTCTCACACCCCTCA
CCCGTGCCACCCTCCACCCCCTGGCCCACCCCACCAGCCACTACCCCCTTCCCAGCGGTT
GTCCAGCCCTACCCTCTCCCAGTGTTCTCTCCTCGAGGAGGCCCCCAGCCTCTTCCCCCT
GCTCCCACATCTGTGCCCCCAGCTGCTTTCCCCGCCCCTTTGGTGACCCCAATGGTGGCC
TTGGTGCTCCCTAACTATCTGTTCCCAACCCCATCCAGCTATCCTTATGGGGCACTCCAG
ACCCCTGCTGAAGGGCCTCCCACTCCTGCCTCGCACTCCCCTTCTCCATCCTTGCCCGCC
CTCGCCCCGAGTCCTCCTCACCGCCCGGACTCTCCACTGTTCAACTCGAGATGCAGCTCT
CCACTCCAGCTCAATCTGCTGCAGCTGGAGGAGCTCCCCCGTGCTGAGGGGGCTGCTGTT
GCAGGAGGCCCTGGGAGCAGTGCCGGGCCCCCACCTCCCAGTGCGGAGGCTGCTGAGCCA
GAGGCCAGACTGGCGGAGGTCACTGAGTCCTCCAATCAGGACGCACTTTCCGGCTCCAGT
GACCTGCTCGAACTTCTGCTGCAAGAGGACTCGCGCTCCGGCACAGGCTCCGCAGCCTCG
GGCTCCTTGGGCTCTGGCTTGGGCTCTGGGTCTGGTTCAGGCTCCCATGAAGGGGGCAGC
ACCTCAGCCAGCATCACTCGCAGCAGCCAGAGCAGCCACACAAGCAAATACTTTGGCAGC
ATCGACTCTTCCGAGGCTGAGGCTGGGGCTGCTCGGGGCGGGGCTGAGCCTGGGGACCAG
GTGATTAAGTACGTGCTCCAGGATCCCATTTGGCTGCTCATGGCCAATGCTGACCAGCGC

GTCATGATGACCTACCAGGTGCCCTCCAGGGACATGACCTCTGTGCTGAAGCAGGATCGG
GAGCGGCTCCGAGCCATGCAGAAGCAGCAGCCTCGGTTTTCTGAGGACCAGCGGCGGGAA
CTGGGTGCTGTGCACTCCTGGGTCCGGAAGGGCCAACTGCCTCGGGCTCTTGATGTGATG
GCCTGTGTGGACTGTGGGAGCAGCACCCAAGATCCTGGTCACCCTGATGACCCACTCTTC
TCAGAGCTGGATGGACTGGGGCTGGAGCCCATGGAAGAGGGTGGAGGCGAGCAGGGCAGC
AGCGGTGGCGGCAGTGGTGAGGGAGAGGGCTGCGAGGAGGCCCAAGGCGGGGCCAAGGCT
TCAAGCTCTCAGGACTTGGCTATGGAGGAGGAGGAAGAAGGCAGGAGCTCATCCAGTCCA
GCCTTACCTACAGCAGGAAACTGCACCAGCTAG

The sequence of PER2 cDNA comprises SEQ ID No.4:

>ENST00000254657.8 PER2-201 cdna:protein_coding

ATGAATGGATACGCGGAATTTCCGCCCAGCCCCAGTAACCCCACCAAGGAGCCCGTGGAG
CCCCAGCCCAGCCAGGTCCCACTGCAGGAAGATGTGGACATGAGCAGTGGCTCCAGTGGA
CATGAGACCAACGAAAACTGCTCCACGGGGCGGGACTCGCAGGGCAGTGACTGTGACGAC
AGTGGGAAGGAGCTGGGGATGCTGGTGGAGCCACCGGATGCCCGCCAGAGTCCAGATACC
TTTAGCCTGATGATGGCAAAATCTGAACACAACCCATCTACAAGTGGCTGCAGTAGCGAC
CAGTCTTCGAAAGTGGACACACACAAAGAACTGATAAAAACACTAAAGGAGCTGAAGGTC
CACCTCCCTGCAGACAAGAAGGCCAAGGGCAAGGCCAGTACGCTGGCCACCTTGAAGTAC
GCCCTCAGGAGCGTGAAGCAGGTGAAAGCCAATGAAGAGTATTACCAGCTGCTGATGTCC
AGCGAGGGTCACCCCTGTGGAGCAGACGTGCCCTCCTACACCGTGGAGGAGATGGAGAGC
GTTACCTCTGAGCACATTGTGAAGAATGCCGATATGTTTGCGGTGGCCGTGTCCCTGGTG
TCTGGGAAGATCCTGTACATCTCTGACCAGGTTGCATCCATATTTCACTGTAAAAGAGAT
GCCTTCAGCGATGCCAAGTTTGTGGAGTTCCTGGCGCCTCACGATGTGGGCGTGTTCCAC
AGTTTCACCTCCCCGTACAAGCTTCCCTTGTGGAGCATGTGCAGTGGAGCAGATTCTTTT
ACTCAAGAATGCATGGAGGAGAAATCTTTCTTTTGCCGTGTCAGTGTCCGGAAAAGCCAC
GAGAATGAAATCCGCTACCACCCCTTCCGCATGACGCCCTACCTGGTCAAGGTGCGGGAC
CAACAAGGTGCTGAGAGTCAGCTTTGCTGCCTTCTGCTGGCAGAGAGAGTGCACTCTGGT
TATGAAGCCCCTAGAATTCCTCCTGAAAAGAGAATTTTTACAACCACCCATACACCAAAT
TGTTTGTTCCAGGATGTGGATGAAAGGGCGGTCCCTCTCCTGGGCTACCTACCTCAGGAC
CTGATTGAAACCCCAGTGCTCGTGCAGCTCCACCCTAGTGACAGGCCCTTGATGCTGGCC
ATCCACAAAAAGATCCTGCAGTCAGGCGGGCAGCCTTTCGACTATTCTCCCATTCGGTTT
CGCGCCCGGAACGGAGAGTACATCACGTTGGACACCAGCTGGTCCAGCTTCATCAACCCA
TGGAGCAGGAAAATCTCCTTCATCATTGGGAGGCACAAAGTCAGGGTGGGCCCTTTGAAT
GAGGACGTGTTTGCAGCCCACCCCTGCACAGAGGAGAAGGCCCTGCACCCCAGCATTCAG
GAGCTCACAGAGCAGATCCACCGGCTCCTGCTGCAGCCCGTCCCCCACAGCGGCTCCAGT
GGCTACGGGAGTCTGGGCAGCAACGGGTCCCACGAGCACCTTATGAGCCAGACCTCCTCC
AGCGACAGCAACGGCCATGAGGACTCACGCCGGAGGAGAGCCGAAATTTGTAAAAATGGT
AACAAGACCAAAAATAGAAGTCATTATTCTCATGAATCTGGAGAACAAAAGAAAAAATCC
GTTACAGAAATGCAAACTAATCCCCCAGCTGAGAAGAAAGCTGTCCCTGCCATGGAAAAG
GACAGCCTGGGGGTCAGCTTCCCCGAGGAGTTGGCCTGCAAGAACCAGCCCACCTGCTCC
TACCAGCAGATCAGCTGCTTGGACAGCGTCATCAGGTACTTGGAGAGCTGCAATGAGGCT
GCCACCCTGAAGAGGAAATGCGAGTTCCCAGCAAACGTCCCAGCGCTAAGGTCCAGTGAT
AAGCGGAAGGCCACAGTCAGCCCAGGGCCACACGCTGGAGAGGCAGAGCCGCCCTCCAGG

GTGAACAGCCGCACGGGAGTAGGTACGCACCTGACCTCGCTGGCACTGCCGGGCAAGGCA
GAGAGTGTGGCGTCGCTCACCAGCCAGTGCAGCTACAGCAGCACCATCGTCCATGTGGGA
GACAAGAAGCCGCAGCCGGAGTTAGAGATGGTGGAAGATGCTGCGAGTGGGCCAGAATCC
CTGGACTGCCTGGCGGGCCCTGCCCTGGCCTGTGGTCTCAGCCAAGAGAAGGAGCCCTTC
AAGAAGCTGGGCCTCACCAAGGAGGTACTCGCTGCACACACACAGAAGGAGGAGCAGAGC
TTCCTGCAGAAGTTCAAAGAAATAAGAAAACTCAGCATTTTCCAGTCCCACTGCCATTAC
TACTTGCAAGAAAGATCCAAGGGGCAGCCAAGTGAACGAACTGCCCCTGGACTAAGAAAT
ACTTCCGGAATAGATTCACCTTGGAAAAAAACAGGAAAGAACAGAAAATTGAAGTCCAAG
CGGGTCAAACCTCGAGACTCATCTGAGAGCACCGGATCTGGGGGGCCCGTGTCCGCCCGG
CCCCCGCTGGTGGGCTTGAACGCCACAGCCTGGTCACCCTCAGACACGTCCCAGTCCAGC
TGCCCAGCCGTGCCCTTTCCCGCCCCAGTGCCAGCAGCTTATTCACTGCCCGTGTTTCCA
GCGCCAGGGACTGTGGCAGCACCCCCGGCACCTCCCCACGCCAGCTTCACAGTGCCTGCT
GTGCCCGTGGACCTCCAGCACCAGTTTGCAGTCCAGCCCCCACCTTTCCCTGCCCCTTTG
GCGCCTGTCATGGCATTCATGCTACCCAGTTATTCCTTCCCCTCGGGGACCCCAAACCTG
CCCCAGGCCTTCTTCCCCAGCCAGCCTCAGTTTCCGAGCCACCCCACACTCACATCCGAG
ATGGCCTCTGCCTCACAGCCTGAGTTCCCCAGCCGGACCTCGATCCCCAGACAGCCATGT
GCTTGTCCAGCCACCCGGGCCACCCCACCATCGGCCATGGGTAGGGCCTCCCCACCGCTC
TTTCAGTCCCGCAGCAGCTCGCCCCTGCAGCTCAACCTGCTGCAGCTGGAGGAAGCCCCT
GAGGGTGGCACTGGAGCCATGGGGACCACAGGGGCCACAGAGACAGCAGCTGTAGGGGCG
GACTGCAAACCTGGCACTTCTCGGGACCAGCAGCCGAAGGCGCCTCTGACCCGTGATGAA
CCCTCAGACACACAGAACAGTGACGCCCTTTCCACGTCAAGCGGCCTCCTAAACCTCCTG
CTGAATGAGGACCTCTGCTCAGCCTCGGGCTCTGCTGCTTCGGAGTCTCTGGGCTCCGGC
TCACTGGGCTGCGACGCCTCCCCGAGTGGGGCAGGCAGTAGTGACACAAGTCATACCAGC
AAATATTTTGGAAGCATTGACTCCTCAGAGAATAATCACAAAGCAAAAATGAACACTGGT
ATGGAAGAAAGTGAGCATTTCATTAAGTGCGTCCTGCAGGATCCCATCTGGCTGCTGATG
GCAGATGCGGACAGCAGCGTCATGATGACGTACCAGCTGCCTTCCCGAAATTTAGAAGCG
GTTTTGAAGGAGGACAGAGAGAAGCTGAAGCTCCTACAGAAACTCCAGCCCAGGTTCACG
GAGAGTCAGAAGCAGGAGCTGCGCGAGGTCCACCAGTGGATGCAGACGGGCGGCCTGCCC
GCAGCCATCGACGTGGCAGAATGTGTTTACTGTGAAAACAAGGAAAAAGGTAATATTTGC
ATACCATATGAGGAAGATATTCCTTCTCTGGGACTCAGCGAAGTGTCGGACACCAAAGAA
GACGAAAATGGATCCCCCTTGAATCACAGGATCGAAGAGCAGACGTAA

The sequence of PER3 cDNA comprises SEQ ID No.5:

>ENST00000613533.4 PER3-208 cdna:protein_coding

ATGCCCCGCGGGGAAGCTCCTGGCCCCGGGAGACGGGGGGCTAAGGACGAGGCCCTGGGC
GAAGAATCGGGGGAGCGGTGGAGCCCCGAGTTCCATCTGCAGAGGAAATTGGCGGACAGC
AGCCACAGTGAACAGCAAGATCGAAACAGAGTTTCTGAAGAACTTATCATGGTTGTCCAA
GAAATGAAAAAATACTTCCCCTCGGAGAGACGCAATAAACCAAGCACTCTAGATGCCCTC
AACTATGCTCTCCGCTGTGTCCACAGCGTTCAAGCAAACAGTGAGTTTTTCCAGATTCTC
AGTCAGAATGGAGCACCTCAGGCAGATGTGAGCATGTACAGTCTTGAGGAGCTGGCCACT
ATCGCTTCAGAACACACTTCCAAAAACACAGATACCTTTGTGGCAGTATTTTCATTTCTG
TCTGGAAGGTTAGTGCACATTTCTGAACAGGCTGCTTTGATCCTGAATCGTAAGAAAGAT
GTCCTGGCGTCTTCTCACTTTGTTGACCTGCTTGCACCTCAAGACATGAGGGTATTCTAC
GCGCACACTGCCAGAGCTCAGCTTCCTTTCTGGAACAACTGGACCCAAAGAGCAGCTGCA

CGGTATGAATGTGCTCCGGTGAAACCTTTTTCTGCAGGATCCGTGGAGGTGAAGACAGA
AAGCAAGAGAAGTGTCACTCCCCATTCCGGATCATCCCCTATCTGATTCATGTACATCAC
CCTGCCCAGCCAGAATTGGAATCGGAACCTTGCTGTCTCACTGTGGTTGAAAAGATTCAC
TCTGGTTATGAAGCTCCTCGGATCCCAGTGAATAAAAGAATCTTCACCACCACACACACC
CCAGGGTGTGTTTTTCTTGAAGTAGATGAAAAAGCAGTGCCTTTGCTGGGTTACCTACCT
CAGGACCTGATTGGAACATCGATCCTAAGCTACCTGCACCCTGAAGATCGTTCTCTGATG
GTTGCCATACACCAAAAAGTTTTGAAGTATGCAGGGCATCCTCCCTTTGAACATTCTCCC
ATTCGATTTTGTACTCAAAACGGAGACTACATCATACTGGATTCCAGTTGGTCCAGCTTT
GTGAATCCCTGGAGCCGGAAGATTTCTTTCATCATTGGTCGGCATAAAGTTCGAACGAGC
CCACTAAATGAGGATGTTTTTGCTACCAAAATTAAAAAGATGAACGATAATGACAAAGAC
ATAACAGAATTACAAGAACAAATTTACAAACTTCTCTTACAGCCAGTTCACGTGAGCGTG
TCCAGCGGCTACGGGAGCCTGGGGAGCAGCGGGTCGCAGGAGCAGCTTGTCAGCATCGCC
TCCTCCAGTGAGGCCAGTGGGCACCGTGTGGAGGAGACGAAGGCGGAGCAGATGACCTTG
CAGCAGGTCTATGCCAGTGTGAACAAAATTAAAAATCTGGGTCAGCAGCTCTACATTGAG
TCAATGACCAAATCATCATTCAAGCCAGTGACGGGGACACGCACAGAACCGAATGGTGGT
GGTGAGTCAGCGAATGGTGGTGGTGAATGTAAGACCTTTACTTCCTTCCACCAAACACTG
AAAAACAATAGTGTGTACACTGAGCCCTGTGAGGATTTGAGGAACGATGAGCACAGCCCA
TCCTATCAACAGATCAACTGTATCGACAGTGTCATCAGATACCTGAAGAGCTACAACATT
CCAGCTTTGAAAAGAAAGTGTATCTCCTGTACAAATACAACTTCTTCCTCCTCAGAAGAA
GACAAACAGAACCACAAGGCAGATGATGTCCAAGCCTTACAAGCTGGTTTGCAAATCCCA
GCCATACCTAAATCAGAAATGCCAACAAATGGACGGTCCATAGACACAGGAGGAGGAGCT
CCACAGATCCTGTCCACGGCGATGCTGAGCTTGGGGTCGGGCATAAGCCAATGCGGTTAC
AGCAGCACCATTGTCCATGTCCCACCCCCAGAGACAGCCAGGGATGCTACCCTCTTCTGT
GAGCCCTGGACCCTGAACATGCAGCCAGCCCCTTTGACCTCGGAAGAATTTAAACACGTG
GGGCTCACAGCGGCTGTTCTGTCAGCGCACACCCAGAAGGAAGAGCAGAATTATGTTGAT
AAATTCCGAGAAAAGATCCTGTCATCACCCTACAGCTCCTATCTTCAGCAAGAAAGCAGG
AGCAAAGCTAAATATTCATATTTTCAAGGAGATTCTACTTCCAAGCAGACGCGGTCGGCC
GGCTGCAGGAAAGGGAAGCACAAGCGGAAGAAGCTGCCGGAGCCGCCAGACAGCAGCAGC
TCGAACACCGGCTCTGGTCCCCGCAGGGGAGCGCATCAGAACGCACAGCCCTGCTGCCCC
TCCGCGGCCTCCTCTCCGCACACCTCGAGCCCGACCTTCCCACCTGCCGCCATGGTGCCC
AGCCAGGCCCCTTACCTCGTCCCAGCTTTTCCCCTCCCAGCCGCGACCTCACCCGGAAGA
GAATACGCAGCCCCCGGAACTGCACCGGAAGGCCTGCATGGGCTGCCCTTGTCCGAGGGC
TTGCAGCCTTACCCAGCTTTCCCTTTTCCTTACTTGGATACTTTTATGACCGTTTTCCTG
CCTGACCCCCCTGTCTGTCCTCTGTTGTCGCCATCGTTTTTGCCATGTCCATTCCTGGGG
GCGACAGCCTCTTCTGCGATATCACCCTCAATGTCGTCAGCAATGAGTCCAACTCTGGAC
CCACCCCCTTCAGTCACCAGCCAAAGGAGAGAGGAGGAAAAGTGGGAGGCACAAAGCGAG
GGGCACCCGTTCATTACTTCGAGAAGCAGCTCACCCTTGCAGTTAAACTTACTTCAGGAA
GAGATGCCCAGACCCTCTGAATCTCCAGATCAGATGAGAAGGAACACGTGCCCACAAACT
GAGTATCAGTGTGTTACAGGCAACAATGGCAGTGAGAGCAGTCCTGCTACTACCGGTGCA
CTGTCCACGGGGTCACCTCCCAGGGAGAATCCATCCCATCCTACTGCCAGCGCTCTGTCC
ACAGGATCGCCTCCCATGAAGAATCCATCCCATCCTACTGCCAGCGCTCTGTCCACAGGA
TCGCCTCCCATGAAGAATCCATCCCATCCTACTGCCAGCACACTGTCCATGGGATTGCCT
CCCAGCAGGACTCCATCCCATCCTACTGCCACTGTTCTGTCCACGGGGTCACCTCCCAGC
GAATCCCCATCCAGAACTGGTTCAGCAGCATCAGGAAGCAGCGACAGCAGTATATACCTT
ACTAGTAGTGTTTATTCTTCTAAAATCTCCCAAAATGGGCAGCAATCTCAGGACGTACAG

AAAAAAGAAACATTTCCTAATGTCGCCGAAGAGCCCATCTGGAGAATGATACGGCAGACA
CCTGAGCGCATTCTCATGACATACCAGGTACCTGAGAGGGTTAAAGAAGTTGTACTAAAA
GAAGACCTGGAAAAGCTAGAAAGTATGAGGCAGCAGCAGCCCCAGTTTTCTCATGGGCAA
AAGGAGGAGCTGGCTAAGGTGTATAATTGGATTCAAAGCCAGACTGTCACTCAAGAAATC
GACATTCAAGCCTGTGTCACTTGTGAAAATGAAGATTCAGCTGATGGTGCGGCCACATCC
TGTGGTCAGGTTCTGGTAGAAGACAGCTGTTGA

The sequence of cDNA CLOCK comprises SEQ ID No.6

>ENST00000513440.6 CLOCK-211 cdna:protein_coding

ATGTTGTTTACCGTAAGCTGTAGTAAAATGAGCTCGATTGTTGACAGAGATGACAGTAGT
ATTTTTGATGGGTTGGTGGAAGAAGATGACAAGGACAAAGCGAAAAGAGTATCTAGAAAC
AAATCTGAAAAGAAACGTAGAGATCAATTTAATGTTCTCATTAAAGAACTGGGATCCATG
CTTCCTGGTAATGCTAGAAAGATGGACAAATCTACTGTTCTGCAGAAAAGCATTGATTTT
TTACGAAAACATAAAGAAATCACTGCACAGTCAGATGCTAGTGAAATTCGACAGGACTGG
AAACCTACATTCCTTAGTAATGAAGAGTTTACACAATTAATGTTAGAGGCTCTTGATGGT
TTTTTTTTAGCAATCATGACAGATGGAAGCATAATATATGTGTCTGAGAGTGTAACTTCA
TTACTTGAACATTTACCATCTGATCTTGTGGATCAAAGTATATTTAATTTTATCCCAGAA
GGGGAACATTCAGAGGTTTATAAAATACTCTCTACTCATCTGCTGGAAAGTGATTCATTA
ACCCCAGAATATTTAAAATCAAAAAATCAGTTAGAATTCTGTTGTCACATGCTGCGAGGA
ACAATAGACCCAAAGGAGCCATCTACCTATGAATATGTAAAATTTATAGGAAATTTCAAA
TCTTTAAACAGTGTATCCTCTTCAGCACACAATGGTTTTGAAGGAACTATACAACGCACA
CATAGGCCATCTTATGAAGATAGAGTTTGTTTTGTAGCTACTGTCAGGTTAGCTACACCT
CAGTTCATCAAGGAAATGTGCACTGTTGAAGAACCCAATGAAGAGTTTACATCTAGACAT
AGTTTAGAATGGAAGTTTCTGTTTCTAGATCACAGGGCACCACCCATAATAGGGTATTTG
CCATTTGAAGTTCTGGGAACATCAGGCTATGATTACTATCATGTGGATGACCTAGAAAAT
TTGGCAAAATGTCATGAGCACTTAATGCAATATGGGAAAGGCAAATCATGTTATTATAGG
TTCCTGACTAAGGGGCAACAGTGGATTTGGCTTCAGACTCATTATTATATCACTTACCAT
CAGTGGAATTCAAGGCCAGAGTTTATTGTTTGTACTCACACTGTAGTAAGTTATGCAGAA
GTTAGGGCTGAAAGACGACGAGAACTTGGCATTGAAGAGTCTCTTCCTGAGACAGCTGCT
GACAAAAGCCAAGATTCTGGGTCAGATAATCGTATAAACACAGTCAGTCTCAAGGAAGCA
TTGGAAAGGTTTGATCACAGCCCAACCCCTTCTGCCTCTTCTCGGAGTTCAAGAAAATCA
TCTCACACGGCCGTCTCAGACCCTTCCTCAACACCAACCAAGATCCCGACGGATACGAGC
ACTCCACCCAGGCAGCATTTACCAGCTCATGAGAAGATGGTGCAAAGAAGGTCATCATTT
AGTAGTCAGTCCATAAATTCCCAGTCTGTTGGTTCATCATTAACACAGCCAGTGATGTCT
CAAGCTACAAATTTACCAATTCCACAAGGCATGTCCCAGTTTCAGTTTTCAGCTCAATTA
GGAGCCATGCAACATCTGAAAGACCAATTGGAACAACGGACACGCATGATAGAAGCAAAT
ATTCATCGGCAACAAGAAGAACTAAGAAAAATTCAAGAACAACTTCAGATGGTCCATGGT
CAGGGGCTGCAGATGTTTTTGCAACAATCAAATCCTGGGTTGAATTTTGGTTCCGTTCAA
CTTTCTTCTGGAAATTCATCTAATATCCAGCAACTTGCACCTATAAATATGCAAGGCCAA
GTTGTTCCTACTAACCAGATTCAAAGTGGAATGAATACTGGACACATTGGCACAACTCAG
CACATGATACAACAACAGACTTTACAGAGTACATCAACTCAGAGTCAACAAAATGTACTG
AGTGGGCACAGTCAGCAAACATCTCTACCCAGTCAGACACAGAGCACTCTTACAGCCCCA
CTGTATAACACTATGGTGATTTCTCAGCCTGCAGCCGGAAGCATGGTCCAGATTCCATCT
AGTATGCCACAAAACAGCACCCAGAGTGCTGCAGTAACTACATTCACTCAGGACAGGCAG

ATAAGATTTTCTCAAGGTCAACAACTTGTGACCAAATTAGTGACTGCTCCTGTAGCTTGT
GGGGCAGTCATGGTACCTAGTACTATGCTTATGGGCCAGGTGGTGACTGCATATCCTACT
TTTGCTACACAACAGCAACAGTCACAGACATTGTCAGTAACGCAGCAGCAGCAGCAGCAG
AGCTCCCAGGAGCAGCAGCTCACTTCAGTTCAGCAACCATCTCAGGCTCAGCTGACCCAG
CCACCGCAACAATTTTTACAGACTTCTAGGTTGCTCCATGGGAATCCCTCAACTCAACTC
ATTCTCTCTGCTGCATTTCCTCTACAACAGAGCACCTTCCCTCAGTCACATCACCAGCAA
CATCAGTCTCAGCAACAGCAGCAACTCAGCCGGCACAGGACTGACAGCTTGCCCGACCCT
TCCAAGGTTCAACCACAGTAG

The sequence of cDNA NPAS2 comprises SEQ ID No.7

>ENST00000335681.10 NPAS2-201 cdna:protein_coding

ATGGATGAAGATGAGAAAGACAGAGCCAAGAGAGCTTCTCGAAACAAGTCTGAGAAGAAG
CGTCGGGACCAGTTCAATGTTCTCATCAAAGAGCTCAGTTCCATGCTCCCTGGCAACACG
CGGAAAATGGACAAAACCACCGTGTTGGAAAAGGTCATCGGATTTTTGCAGAAACACAAT
GAAGTCTCAGCGCAAACGGAAATCTGTGACATTCAGCAAGACTGGAAGCCTTCATTCCTC
AGTAATGAAGAATTCACCCAGCTGATGTTGGAGGCATTAGATGGCTTCATTATCGCAGTG
ACAACAGACGGCAGCATCATCTATGTCTCTGACAGTATCACGCCTCTCCTTGGGCATTTA
CCGTCGGATGTCATGGATCAGAATTTGTTAAATTTCCTCCCAGAACAAGAACATTCAGAA
GTTTATAAAATCCTTTCTTCCCATATGCTTGTGACGGATTCCCCCTCCCCAGAATACTTA
AAATCTGACAGCGATTTAGAGTTTTATTGCCATCTTCTCAGAGGCAGCTTGAACCCAAAG
GAATTTCCAACTTATGAATACATAAAATTTGTAGGAAATTTTCGCTCTTACAACAATGTG
CCTAGCCCCTCCTGTAATGGTTTTGACAACACCCTTTCAAGACCTTGCCGGGTGCCACTA
GGAAAGGAGGTTTGCTTCATTGCCACCGTTCGTCTGGCAACACCACAATTCTTAAAGGAA
ATGTGCATAGTTGACGAACCTTTAGAGGAATTCACTTCAAGGCATAGCTTGGAATGGAAA
TTTTTATTTCTGGATCACAGAGCACCTCCAATCATAGGATACCTGCCTTTTGAAGTGCTG
GGAACCTCAGGCTATGACTACTACCACATTGATGACCTGGAGCTCCTGGCCAGGTGTCAC
CAGCACCTGATGCAGTTTGGCAAAGGGAAGTCGTGTTGCTACCGGTTTCTGACCAAAGGT
CAGCAGTGGATCTGGCTGCAGACTCACTACTACATCACCTACCATCAGTGGAACTCCAAG
CCCGAGTTCATCGTGTGCACACACTCGGTGGTCAGTTACGCAGATGTCCGGGTGGAAAGG
AGGCAGGAGCTGGCTCTGGAAGACCCGCCATCCGAGGCCCTCCACTCCTCAGCACTAAAG
GACAAGGGCTCAAGCCTGGAACCTCGGCAGCACTTTAACACACTCGACGTGGGTGCCTCG
GGCCTTAATACCAGTCATTCGCCATCGGCGTCCTCAAGAAGTTCCCACAAATCCTCGCAC
ACAGCCATGTCAGAACCCACCTCCACTCCCACCAAGCTGATGGCAGAGGCCAGCACCCCG
GCTTTGCCAAGATCAGCCACCCTGCCCCAAGAGTTACCTGTCCCCGGGCTCAGCCAGGCA
GCCACCATGCCGGCCCCTCTGCCTTCCCCATCGTCCTGCGACCTCACACAGCAGCTCCTG
CCTCAGACCGTTCTGCAGAGCACGCCCGCTCCCATGGCACAGTTTTCGGCACAGTTCAGC
ATGTTCCAGACCATCAAAGACCAGCTAGAGCAGCGGACGCGGATCCTGCAGGCCAATATC
CGGTGGCAACAGGAAGAGCTCCACAAGATCCAGGAGCAGCTCTGCCTGGTCCAGGACTCC
AACGTCCAGATGTTCCTGCAGCAGCCAGCTGTATCCCTGAGCTTCAGCAGCACCCAGCGA
CCTGAGGCTCAGCAGCAGCTACAGCAAAGGTCAGCTGCAGTGACTCAGCCCCAGCTCGGG
GCGGGCCCCCAACTTCCAGGGCAGATCTCCTCTGCCCAGGTCACAAGCCAGCACCTGCTC
AGAGAATCAAGTGTGATATCAACCCAGGGTCCAAAGCCAATGAGAAGCTCACAGCTAATG
CAGAGCAGCGGCCGCTCTGGAAGCAGCCTAGTGTCCCCGTTCAGCAGCGCCACAGCTGCG
CTCCCGCCAAGTCTGAATCTGACCACACCTGCTTCCACCTCCCAGGATGCCAGCCAGTGC

CAGCCCAGCCCAGACTTCAGCCATGATCGGCAGCTCAGGCTGTTGCTGAGCCAGCCCATC
CAGCCCATGATGCCCGGGTCCTGTGACGCAAGGCAGCCCTCGGAAGTCAGCAGGACGGGA
CGGCAAGTCAAGTACGCCCAGAGCCAGACCGTGTTTCAAAATCCAGACGCACACCCCGCC
AACAGCAGCAGCGCCCCGATGCCCGTCCTGCTGATGGGGCAGGCGGTGCTCCACCCCAGC
TTCCCTGCCTCCCAACCATCGCCCCTGCAGCCTGCACAGGCCCGGCAGCAGCCACCGCAG
CACTACCTGCAGGTACAGGCACCAACCTCTTTGCACAGTGAGCAGCAGGACTCGCTACTT
CTCTCCACCTACTCACAACAGCCAGGGACCCTGGGCTACCCCCAACCACCCCCAGCACAG
CCCCAGCCCCTACGTCCTCCCCGAAGGGTCAGCAGTCTGTCTGAGTCGTCAGGCCTCCAG
CAGCCGCCCCGATAA

The sequence of cDNA CRY1 comprises SEQ ID No.8

>ENST00000008527.10 CRY1-201 cdna:protein_coding

ATGGGGGTGAACGCCGTGCACTGGTTCCGAAAGGGGCTCCGGCTCCACGACAACCCCGCC
CTGAAGGAGTGCATTCAGGGCGCCGACACCATCCGCTGCGTCTACATCCTGGACCCCTGG
TTCGCCGGCTCCTCCAATGTGGGCATCAACAGGTGGCGATTTTTGCTTCAGTGTCTTGAG
GATCTTGATGCCAATCTACGAAAATTAAACTCCCGTCTGTTTGTGATTCGTGGACAACCA
GCAGATGTGTTTCCCAGGCTTTTCAAGGAATGGAACATTACTAAACTTTCAATTGAGTAT
GATTCTGAGCCCTTTGGAAAGGAACGAGACGCAGCTATTAAGAAACTGGCAACTGAAGCT
GGAGTAGAAGTCATTGTAAGAATTTCACATACATTATATGACCTAGACAAGATCATAGAA
CTCAATGGTGGACAACCGCCTCTAACTTATAAAAGATTCCAGACTCTCATCAGCAAAATG
GAACCACTAGAGATACCAGTAGAGACAATTACTTCAGAAGTGATAGAAAAGTGCACAACT
CCTCTGTCTGATGACCATGATGAGAAATATGGAGTCCCTTCACTGGAAGAGCTAGGTTTT
GATACAGATGGCTTATCCTCTGCAGTGTGGCCAGGTGGAGAAACTGAAGCACTTACTCGT
TTGGAAAGGCATTTGGAAAGAAAAGCTTGGGTGGCAAATTTTGAAAGACCTCGAATGAAT
GCGAATTCTCTGCTTGCAAGCCCTACTGGACTTAGTCCTTATCTCCGATTTGGTTGTTTG
TCATGTCGACTGTTTTACTTCAAACTAACAGATCTCTACAAAAAGGTAAAGAAGAACAGT
TCCCCTCCCCTTTCCCTTTATGGGCAACTGTTATGGCGTGAATTTTTCTATACAGCAGCA
ACAAATAATCCACGCTTTGATAAAATGGAAGGAAACCCTATCTGTGTTCAGATTCCTTGG
GATAAAAATCCTGAGGCTTTAGCCAAATGGGCGGAAGGCCGGACAGGCTTTCCATGGATT
GATGCCATCATGACACAGCTTCGTCAGGAGGGTTGGATTCATCATCTAGCCAGGCATGCA
GTTGCTTGCTTCCTGACACGAGGGGACCTGTGGATTAGTTGGGAAGAAGGAATGAAGGTA
TTTGAAGAATTATTGCTTGATGCAGATTGGAGCATAAATGCTGGAAGTTGGATGTGGCTG
TCTTGTAGTTCCTTTTTTCAACAGTTTTTTCACTGCTATTGCCCTGTTGGTTTTGGTAGG
AGAACAGATCCCAATGGAGACTATATCAGGCGTTATTTGCCTGTCCTAAGAGGCTTCCCT
GCAAAATATATCTATGATCCCTGGAATGCACCAGAAGGTATCCAAAAGGTAGCCAAATGT
TTGATAGGAGTTAATTATCCTAAACCAATGGTGAACCATGCTGAGGCAAGCCGTTTGAAT
ATCGAAAGGATGAAACAGATCTATCAGCAGCTTTCACGATATAGAGGACTAGGTCTTCTG
GCATCAGTACCTTCTAATCCTAATGGGAATGGAGGCTTCATGGGATATTCTGCAGAAAAT
ATCCCAGGTTGTAGCAGCAGTGGAAGTTGCTCTCAAGGGAGTGGTATTTTACACTATGCT
CATGGCGACAGTCAGCAAACTCACCTGTTGAAGCAAGGAAGAAGCTCCATGGGCACTGGT
CTCAGTGGTGGGAAACGTCCTAGTCAGGAAGAGGACACACAGAGTATTGGTCCTAAAGTC
CAGAGACAGAGCACTAATTAG

The sequence of cDNA CRY2 comprises SEQ ID No.9

>ENST00000616623.4 CRY2-212 cdna:protein_coding

ATGGGCGGGGTCCACGTCGCCTACCGGGGCGGAGCGGGGGTGGCTGGAGCAGTCTGGACA
GTCATGGCGGCGACTGTGGCGACGGCGGCAGCTGTGGCCCCGGCGCCAGCGCCCGGCACG
GACAGCGCCTCTTCGGTGCACTGGTTCCGCAAAGGGCTGCGACTCCACGACAACCCGGCG
TTGCTGGCGGCCGTGCGCGGGGCGCGCTGCGTGCGCTGCGTTTACATTCTCGACCCGTGG
TTCGCGGCCTCCTCCTCAGTCGGGATCAACCGATGGAGGTTCCTACTTCAGTCTCTGGAA
GATTTGGACACAAGTTTAAGGAAACTGAACTCCCGCCTGTTTGTAGTCCGGGGACAGCCA
GCCGACGTGTTCCCAAGGCTGTTCAAGGAATGGGGAGTGACCCGCTTGACCTTTGAATAT
GACTCTGAACCCTTTGGGAAAGAACGGGATGCAGCCATCATGAAGATGGCCAAGGAGGCT
GGTGTGGAAGTAGTGACGGAGAATTCTCATACCCTCTATGACCTGGACAGGATCATTGAG
CTGAATGGGCAGAAGCCACCCCTTACATACAAGCGCTTTCAGGCCATCATCAGCCGCATG
GAGCTGCCCAAGAAGCCAGTGGGCTTGGTGACCAGCCAGCAGATGGAGAGCTGCAGGGCC
GAGATCCAGGAGAACCACGACGAGACCTACGGCGTGCCCTCCCTGGAGGAGCTGGGGTTC
CCCACTGAAGGACTTGGTCCAGCTGTCTGGCAGGGAGGAGAGACAGAAGCTCTGGCCCGC
CTGGATAAGCACTTGGAACGGAAGGCCTGGGTTGCCAACTATGAGAGACCCCGAATGAAC
GCCAACTCCCTCCTGGCCAGCCCCACAGGCCTCAGCCCCTACCTGCGCTTTGGTTGTCTC
TCCTGCCGCCTCTTCTACTACCGCCTGTGGGACCTGTATAAAAAGGTGAAGCGGAACAGC
ACACCTCCCCTCTCCCTATTTGGGCAACTCCTATGGCGAGAGTTCTTCTACACGGCAGCT
ACCAACAACCCCAGGTTTGACCGCATGGAGGGGAACCCCATCTGCATCCAGATCCCCTGG
GACCGCAATCCTGAGGCCCTGGCCAAGTGGGCTGAGGGCAAGACAGGCTTCCCTTGGATT
GATGCCATCATGACCCAACTGAGGCAGGAGGGCTGGATCCACCACCTGGCCCGGCATGCC
GTGGCCTGCTTCCTGACCCGCGGGGACCTCTGGGTCAGCTGGGAGAGCGGGGTCCGGGTA
TTTGATGAGCTGCTCCTGGATGCAGATTTCAGCGTGAACGCAGGCAGCTGGATGTGGCTG
TCCTGCAGTGCTTTCTTCCAGCAGTTCTTCCACTGCTACTGCCCTGTGGGCTTTGGCCGT
CGCACGGACCCCAGTGGGGACTACATCAGGCGATACCTGCCCAAATTGAAAGCGTTCCCC
TCTCGATACATCTATGAGCCCTGGAATGCCCCAGAGTCAATTCAGAAGGCAGCCAAGTGC
ATCATTGGTGTGGACTACCCACGGCCCATCGTCAACCATGCCGAGACCAGCCGGCTTAAC
ATTGAACGAATGAAGCAGATTTACCAGCAGCTTTCGCGCTACCGGGGACTCTGTCTACTG
GCATCTGTCCCTTCCTGTGTGGAAGACCTCAGTCACCCTGTGGCAGAGCCCAGCTCGAGC
CAGGCTGGCAGCATGAGCAGTGCAGGCCCAAGACCACTACCCAGTGGCCCAGCATCCCCC
AAACGCAAGCTGGAAGCAGCCGAGGAACCACCTGGTGAAGAACTCAGCAAACGGGCCCGG
GTGGCAGAGTTGCCAACCCCAGAGCTGCCGAGCAAGGATGCCTGA

The sequence of cDNA NR1D1 comprises SEQ ID No.10

>ENST00000246672.4 NR1D1-201 cdna:protein_coding

ATGACGACCCTGGACTCCAACAACAACACAGGTGGCGTCATCACCTACATTGGCTCCAGT
GGCTCCTCCCCAAGCCGCACCAGCCCTGAATCCCTCTATAGTGACAACTCCAATGGCAGC
TTCCAGTCCCTGACCCAAGGCTGTCCCACCTACTTCCCACCATCCCCCACTGGCTCCCTC
ACCCAAGACCCGGCTCGCTCCTTTGGGAGCATTCCACCCAGCCTGAGTGATGACGGCTCC
CCTTCTTCCTCATCTTCCTCGTCGTCATCCTCCTCCTCCTTCTATAATGGGAGCCCCCCT

GGGAGTCTACAAGTGGCCATGGAGGACAGCAGCCGAGTGTCCCCCAGCAAGAGCACCAGC
AACATCACCAAGCTGAATGGCATGGTGTTACTGTGTAAAGTGTGTGGGGACGTTGCCTCG
GGCTTCCACTACGGTGTGCACGCCTGCGAGGGCTGCAAGGGCTTTTTCCGTCGGAGCATC
CAGCAGAACATCCAGTACAAAAGGTGTCTGAAGAATGAGAATTGCTCCATCGTCCGCATC
AATCGCAACCGCTGCCAGCAATGTCGCTTCAAGAAGTGTCTCTCTGTGGGCATGTCTCGA
GACGCTGTGCGTTTTGGGCGCATCCCCAAACGAGAGAAGCAGCGGATGCTTGCTGAGATG
CAGAGTGCCATGAACCTGGCCAACAACCAGTTGAGCAGCCAGTGCCCGCTGGAGACTTCA
CCCACCCAGCACCCCACCCCAGGCCCCATGGGCCCCTCGCCACCCCCTGCTCCGGTCCCC
TCACCCCTGGTGGGCTTCTCCCAGTTTCCACAACAGCTGACGCCTCCCAGATCCCCAAGC
CCTGAGCCCACAGTGGAGGATGTGATATCCCAGGTGGCCCGGGCCCATCGAGAGATCTTC
ACCTACGCCCATGACAAGCTGGGCAGCTCACCTGGCAACTTCAATGCCAACCATGCATCA
GGTAGCCCTCCAGCCACCACCCCACATCGCTGGGAAAATCAGGGCTGCCCACCTGCCCCC
AATGACAACAACACCTTGGCTGCCCAGCGTCATAACGAGGCCCTAAATGGTCTGCGCCAG
GCTCCCTCCTCCTACCCTCCCACCTGGCCTCCTGGCCCTGCACACCACAGCTGCCACCAG
TCCAACAGCAACGGGCACCGTCTATGCCCCACCCACGTGTATGCAGCCCCAGAAGGCAAG
GCACCTGCCAACAGTCCCCGGCAGGGCAACTCAAAGAATGTTCTGCTGGCATGTCCTATG
AACATGTACCCGCATGGACGCAGTGGGCGAACGGTGCAGGAGATCTGGGAGGATTTCTCC
ATGAGCTTCACGCCCGCTGTGCGGGAGGTGGTAGAGTTTGCCAAACACATCCCGGGCTTC
CGTGACCTTTCTCAGCATGACCAAGTCACCCTGCTTAAGGCTGGCACCTTTGAGGTGCTG
ATGGTGCGCTTTGCTTCGTTGTTCAACGTGAAGGACCAGACAGTGATGTTCCTAAGCCGC
ACCACCTACAGCCTGCAGGAGCTTGGTGCCATGGGCATGGGAGACCTGCTCAGTGCCATG
TTCGACTTCAGCGAGAAGCTCAACTCCCTGGCGCTTACCGAGGAGGAGCTGGGCCTCTTC
ACCGCGGTGGTGCTTGTCTCTGCAGACCGCTCGGGCATGGAGAATTCCGCTTCGGTGGAG
CAGCTCCAGGAGACGCTGCTGCGGGCTCTTCGGGCTCTGGTGCTGAAGAACCGGCCCTTG
GAGACTTCCCGCTTCACCAAGCTGCTGCTCAAGCTGCCGGACCTGCGGACCCTGAACAAC
ATGCATTCCGAGAAGCTGCTGTCCTTCCGGGTGGACGCCCAGTGA

The sequence of cDNA NR1D2 comprises SEQ ID No.11

>ENST00000312521.9 NR1D2-201 cdna:protein_coding

ATGGAGGTGAATGCAGGAGGTGTGATTGCCTATATCAGTTCTTCCAGCTCAGCCTCAAGC
CCTGCCTCTTGTCACAGTGAGGGTTCTGAGAATAGTTTCCAGTCCTCCTCCTCTTCTGTT
CCATCTTCTCCAAATAGCTCTAATTCTGATACCAATGGTAATCCCAAGAATGGTGATCTC
GCCAATATTGAAGGCATCTTGAAGAATGATCGAATAGATTGTTCTATGAAAACAAGCAAA
TCGAGTGCACCTGGGATGACAAAAAGTCATAGTGGTGTGACAAAATTTAGTGGCATGGTT
CTACTGTGTAAAGTCTGTGGGGATGTGGCGTCAGGATTCCACTATGGAGTTCATGCTTGC
GAAGGCTGTAAGGGTTTCTTTCGGAGAAGTATTCAACAAAACATCCAGTACAAGAAGTGC
CTGAAGAATGAAAACTGTTCTATAATGAGAATGAATAGGAACAGATGTCAGCAATGTCGC
TTCAAAAAGTGTCTGTCTGTTGGAATGTCAAGAGATGCTGTTCGGTTTGGTCGTATTCCT
AAGCGTGAAAAACAGAGGATGCTAATTGAAATGCAAAGTGCAATGAAGACCATGATGAAC
AGCCAGTTCAGTGGTCACTTGCAAAATGACACATTAGTAGAACATCATGAACAGACAGCC
TTGCCAGCCCAGGAACAGCTGCGACCCAAGCCCCAACTGGAGCAAGAAAACATCAAAAGC
TCTTCTCCTCCATCTTCTGATTTTGCAAAGGAAGAAGTGATTGGCATGGTGACCAGAGCT
CACAAGGATACCTTTATGTATAATCAAGAGCAGCAAGAAAACTCAGCTGAGAGCATGCAG
CCCCAGAGAGGAGAACGGATTCCCAAGAACATGGAGCAATATAATTTAAATCATGATCAT

TGCGGCAATGGGCTTAGCAGCCATTTTCCCTGTAGTGAGAGCCAGCAGCATCTCAATGGA
CAGTTCAAAGGGAGGAATATAATGCATTACCCAAATGGTCATGCCATTTGTATTGCAAAT
GGACATTGTATGAACTTCTCCAATGCTTATACTCAAAGAGTATGTGATAGAGTTCCGATA
GATGGATTTTCTCAGAATGAGAACAAGAATAGTTACCTGTGCAACACTGGAGGAAGAATG
CATCTGGTTTGTCCAATGAGTAAGTCTCCATATGTGGATCCTCATAAATCAGGACATGAA
ATCTGGGAAGAATTTTCGATGAGCTTCACTCCAGCAGTGAAAGAAGTGGTGGAATTTGCA
AAGCGTATTCCTGGGTTCAGAGATCTCTCTCAGCATGACCAGGTCAACCTTTTAAAGGCT
GGGACTTTTGAGGTTTTAATGGTACGGTTCGCATCATTATTTGATGCAAAGGAACGTACT
GTCACCTTTTTAAGTGGAAAGAAATATAGTGTGGATGATTTACACTCAATGGGAGCAGGG
GATCTGCTAAACTCTATGTTTGAATTTAGTGAGAAGCTAAATGCCCTCCAACTTAGTGAT
GAAGAGATGAGTTTGTTTACAGCTGTTGTCCTGGTATCTGCAGATCGATCTGGAATAGAA
AACGTCAACTCTGTGGAGGCTTTGCAGGAAACTCTCATTCGTGCACTAAGGACCTTAATA
ATGAAAAACCATCCAAATGAGGCCTCTATTTTTACAAAACTGCTTCTAAAGTTGCCAGAT
CTTCGATCTTTAAACAACATGCACTCTGAGGAGCTCTTGGCCTTTAAAGTTCACCCTTAA

The sequence of cDNA RORA comprises SEQ ID No.12

>ENST00000335670.11 RORA-203 cdna:protein_coding

ATGGAGTCAGCTCCGGCAGCCCCCGACCCCGCCGCCAGCGAGCCAGGCAGCAGCGGCGCG
GACGCGGCCGCCGGCTCCAGGGAGACCCCGCTGAACCAGGAATCCGCCCGCAAGAGCGAG
CCGCCTGCCCCGGTGCGCAGACAGAGCTATTCCAGCACCAGCAGAGGTATCTCAGTAACG
AAGAAGACACATACATCTCAAATTGAAATTATTCCATGCAAGATCTGTGGAGACAAATCA
TCAGGAATCCATTATGGTGTCATTACATGTGAAGGCTGCAAGGGCTTTTTCAGGAGAAGT
CAGCAAAGCAATGCCACCTACTCCTGTCCTCGTCAGAAGAACTGTTTGATTGATCGAACC
AGTAGAAACCGCTGCCAACACTGTCGATTACAGAAATGCCTTGCCGTAGGGATGTCTCGA
GATGCTGTAAAATTTGGCCGAATGTCAAAAAAGCAGAGAGACAGCTTGTATGCAGAAGTA
CAGAAACACCGGATGCAGCAGCAGCAGCGCGACCACCAGCAGCAGCCTGGAGAGGCTGAG
CCGCTGACGCCCACCTACAACATCTCGGCCAACGGGCTGACGGAACTTCACGACGACCTC
AGTAACTACATTGACGGGCACACCCCTGAGGGGAGTAAGGCAGACTCCGCCGTCAGCAGC
TTCTACCTGGACATACAGCCTTCCCCAGACCAGTCAGGTCTTGATATCAATGGAATCAAA
CCAGAACCAATATGTGACTACACACCAGCATCAGGCTTCTTTCCCTACTGTTCGTTCACC
AACGGCGAGACTTCCCCAACTGTGTCCATGGCAGAATTAGAACACCTTGCACAGAATATA
TCTAAATCGCATCTGGAAACCTGCCAATACTTGAGAGAAGAGCTCCAGCAGATAACGTGG
CAGACCTTTTTACAGGAAGAAATTGAGAACTATCAAAACAAGCAGCGGGAGGTGATGTGG
CAATTGTGTGCCATCAAAATTACAGAAGCTATACAGTATGTGGTGGAGTTTGCCAAACGC
ATTGATGGATTTATGGAACTGTGTCAAAATGATCAAATTGTGCTTCTAAAAGCAGGTTCT
CTAGAGGTGGTGTTTATCAGAATGTGCCGTGCCTTTGACTCTCAGAACAACACCGTGTAC
TTTGATGGGAAGTATGCCAGCCCCGACGTCTTCAAATCCTTAGGTTGTGAAGACTTTATT
AGCTTTGTGTTTGAATTTGGAAAGAGTTTATGTTCTATGCACCTGACTGAAGATGAAATT
GCATTATTTTCTGCATTTGTACTGATGTCAGCAGATCGCTCATGGCTGCAAGAAAAGGTA
AAAATTGAAAAACTGCAACAGAAAATTCAGCTAGCTCTTCAACACGTCCTACAGAAGAAT
CACCGAGAAGATGGAATACTAACAAAGTTAATATGCAAGGTGTCTACCTTAAGAGCCTTA
TGTGGACGACATACAGAAAAGCTAATGGCATTTAAAGCAATATACCCAGACATTGTGCGA
CTTCATTTTCCTCCATTATACAAGGAGTTGTTCACTTCAGAATTTGAGCCAGCAATGCAA
ATTGATGGGTAA

The sequence of cDNA RORB comprises SEQ ID No.13

>ENST00000376896.8 RORB-201 cdna:protein_coding

ATGCGAGCACAAATTGAAGTGATACCATGCAAAATTTGTGGCGATAAGTCCTCTGGGATC
CACTACGGAGTCATCACATGTGAAGGCTGCAAGGGATTCTTTAGGAGGAGCCAGCAGAAC
AATGCTTCTTATTCCTGCCCAAGGCAGAGAAACTGTTTAATTGACAGAACGAACAGAAAC
CGTTGCCAACACTGCCGACTGCAGAAGTGTCTTGCCCTAGGAATGTCAAGAGATGCTGTG
AAGTTTGGGAGGATGTCCAAGAAGCAAAGGGACAGCCTGTATGCTGAGGTGCAGAAGCAC
CAGCAGCGGCTGCAGGAACAGCGGCAGCAGCAGAGTGGGGAGGCAGAAGCCCTTGCCAGG
GTGTACAGCAGCAGCATTAGCAACGGCCTGAGCAACCTGAACAACGAGACCAGCGGCACT
TATGCCAACGGGCACGTCATTGACCTGCCCAAGTCTGAGGGTTATTACAACGTCGATTCC
GGTCAGCCGTCCCCTGATCAGTCAGGACTTGACATGACTGGAATCAAACAGATAAAGCAA
GAACCTATCTATGACCTCACATCCGTACCCAACTTGTTTACCTATAGCTCTTTCAACAAT
GGGCAGTTAGCACCAGGGATAACCATGACTGAAATCGACCGAATTGCACAGAACATCATT
AAGTCCCATTTGGAGACATGTCAATACACCATGGAAGAGCTGCACCAGCTGGCGTGGCAG
ACCCACACCTATGAAGAAATTAAAGCATATCAAAGCAAGTCCAGGGAAGCACTGTGGCAA
CAATGTGCCATCCAGATCACTCACGCCATCCAATACGTGGTGGAGTTTGCAAAGCGGATA
ACAGGCTTCATGGAGCTCTGTCAAAATGATCAAATTCTACTTCTGAAGTCAGGTTGCTTG
GAAGTGGTTTTAGTGAGAATGTGCCGTGCCTTCAACCCATTAAACAACACTGTTCTGTTT
GAAGGAAAATATGGAGGAATGCAAATGTTCAAAGCCTTAGGTTCTGATGACCTAGTGAAT
GAAGCATTTGACTTTGCAAAGAATTTGTGTTCCTTGCAGCTGACCGAGGAGGAGATCGCT
TTGTTCTCATCTGCTGTTCTGATATCTCCAGACCGAGCCTGGCTTATAGAACCAAGGAAA
GTCCAGAAGCTTCAGGAAAAAATTTATTTTGCACTTCAACATGTGATTCAGAAGAATCAC
CTGGATGATGAGACCTTGGCAAAGTTAATAGCCAAGATACCAACCATCACGGCAGTTTGC
AACTTGCACGGGGAGAAGCTGCAGGTATTTAAGCAATCTCATCCAGAGATAGTGAATACA
CTGTTTCCTCCGTTATACAAGGAGCTCTTTAATCCTGACTGTGCCACCGGCTGCAAATGA

The sequence of cDNA RORC comprises SEQ ID No.14

>ENST00000318247.7 RORC-201 cdna:protein_coding

ATGGACAGGGCCCCACAGAGACAGCACCGAGCCTCACGGGAGCTGCTGGCTGCAAAGAAG
ACCCACACCTCACAAATTGAAGTGATCCCTTGCAAAATCTGTGGGGACAAGTCGTCTGGG
ATCCACTACGGGGTTATCACCTGTGAGGGGTGCAAGGGCTTCTTCCGCCGGAGCCAGCGC
TGTAACGCGGCCTACTCCTGCACCCGTCAGCAGAACTGCCCCATCGACCGCACCAGCCGA
AACCGATGCCAGCACTGCCGCCTGCAGAAATGCCTGGCGCTGGGCATGTCCCGAGATGCT
GTCAAGTTCGGCCGCATGTCCAAGAAGCAGAGGGACAGCCTGCATGCAGAAGTGCAGAAA
CAGCTGCAGCAGCGGCAACAGCAGCAACAGGAACCAGTGGTCAAGACCCCTCCAGCAGGG
GCCCAAGGAGCAGATACCCTCACCTACACCTTGGGGCTCCCAGACGGGCAGCTGCCCCTG
GGCTCCTCGCCTGACCTGCCTGAGGCTTCTGCCTGTCCCCCTGGCCTCCTGAAAGCCTCA
GGCTCTGGGCCCTCATATTCCAACAACTTGGCCAAGGCAGGGCTCAATGGGGCCTCATGC
CACCTTGAATACAGCCCTGAGCGGGGCAAGGCTGAGGGCAGAGAGAGCTTCTATAGCACA

GGCAGCCAGCTGACCCCTGACCGATGTGGACTTCGTTTTGAGGAACACAGGCATCCTGGG
CTTGGGGAACTGGGACAGGGCCCAGACAGCTACGGCAGCCCCAGTTTCCGCAGCACACCG
GAGGCACCCTATGCCTCCCTGACAGAGATAGAGCACCTGGTGCAGAGCGTCTGCAAGTCC
TACAGGGAGACATGCCAGCTGCGGCTGGAGGACCTGCTGCGGCAGCGCTCCAACATCTTC
TCCCGGGAGGAAGTGACTGGCTACCAGAGGAAGTCCATGTGGGAGATGTGGGAACGGTGT
GCCCACCACCTCACCGAGGCCATTCAGTACGTGGTGGAGTTCGCCAAGAGGCTCTCAGGC
TTTATGGAGCTCTGCCAGAATGACCAGATTGTGCTTCTCAAAGCAGGAGCAATGGAAGTG
GTGCTGGTTAGGATGTGCCGGGCCTACAATGCTGACAACCGCACGGTCTTTTTGAAGGC
AAATACGGTGGCATGGAGCTGTTCCGAGCCTTGGGCTGCAGCGAGCTCATCAGCTCCATC
TTTGACTTCTCCCACTCCCTAAGTGCCTTGCACTTTTCCGAGGATGAGATTGCCCTCTAC
ACAGCCCTTGTTCTCATCAATGCCCATCGGCCAGGGCTCCAAGAGAAAAGGAAAGTAGAA
CAGCTGCAGTACAATCTGGAGCTGGCCTTTCATCATCATCTCTGCAAGACTCATCGCCAA
AGCATCCTGGCAAAGCTGCCACCCAAGGGGAAGCTTCGGAGCCTGTGTAGCCAGCATGTG
GAAAGGCTGCAGATCTTCCAGCACCTCCACCCCATCGTGGTCCAAGCCGCTTTCCCTCCA
CTCTACAAGGAGCTCTTCAGCACTGAAACCGAGTCACCTGTGGGGCTGTCCAAGTGA

In one embodiment of the method according to the present invention said assessing the timing of administration of said medicament to said subject comprises evaluating the predicted gene expression levels and/or evaluating expression phenotypes based on the determined and/or predicted gene expression levels.

In one embodiment of the method according to the present invention assessing the circadian rhythm of said subject comprises in the computational step determining a periodic function for each of said determined genes that approximates said gene expression levels for each of said genes, preferably comprising curve fitting of a non-linear periodic model function to the respective gene expression levels, wherein the curve fitting is preferably carried out by means of harmonic regression.

In one embodiment of the method according to the invention assessing the circadian rhythm of said subject comprises determining a periodic function for each of said determined genes, in particular at least two core clock genes, in particular for said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, that approximates said expression levels for each of at least two core clock genes, in particular for said at least two members of the groups ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, preferably comprising curve fitting of a non-linear periodic model function to the respective expression levels, wherein the curve fitting is preferably carried out by means of harmonic regression.

It will be appreciated that other curve fitting methods may be applied for determining a mathematical function that has the best fit to the series of the measured gene expressions. The skilled person will understand that curve fitting in the context of this disclosure aims at finding a periodic function (oscillatory function) because of the periodicity of the circadian clock(s). While curve fitting may generally aim at finding an interpolation for exact fitting of the data points, methods that approximate the series of measure gene expressions will be preferred, e.g. smoothing, in which a "smooth" function is constructed that approximately fits the data.

It has been found that regression analysis methods are more appropriate here, which use statistical data, not least because the determined periodic function shall represent not only the measured data points but particularly future values. Polynomial interpolation or polynomial regression may be alternatively applied. Preferably, harmonic regression is used, which is based on the trigonometric functions sine and cosine. As will be appreciated by a person skilled in the art, various methods for minimizing an error between the fitted curve and the measured data points may be applied, such as square errors, which is set forth in more detail below. In the method of harmonic regression, the model $y(t) = m + a\cos(\omega t) + b\sin(\omega t)$ is fitted to the measured data to determine the absolute ($A = \sqrt{(a2 + b2)}$) and relative amplitude as well as the phase ($\tan\varphi = b/a$), the p-value and the confidence interval. The significance level p may be selected as $p < 0.05$.

In one embodiment of the method according to the present invention the computational step comprises processing the determined gene expression levels to derive characteristic data for each of said genes, said processing comprising determining the mean expression level of expression of a gene and normalizing the gene expression levels using the mean expression level.

Particularly in view of the machine learning processes as described below, the "raw data", i.e. the measured gene expression levels, including the obtained periodic functions resulting from the curve fitting, have to be preprocessed to bring them into a form that is suitable for the intended machine learning algorithm. For instance, the preprocessing includes extracting data of interest (characteristic data) and setting the dimensionality for the machine learning, i.e. number of parameters. Further, in order to get comparable parameters, normalization is typically required to achieve a common scale for all parameters. It has been found that using the mean expression level for normalizing the measured data is a suitable approach. Further, in order not to lose the absolute values, the mean level is added to the parameter space. This will be set forth also in more detail below.

In one embodiment of the method according to the present invention said characteristic data comprise:

- the amplitude of change of expression of a gene, and/or the amplitude relative to one of the other genes, and/or
- the mean expression level of expression of a gene and/ or the mean relative to one of the other genes, and/or
- the peak expression level of a gene, and/or the peak relative to one of the other genes, and/or
- the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the amplitudes of change of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the mean expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/ or
- the relative difference of the mean expression levels of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the peak expression levels of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the time of the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
- the relative difference of the times of the peak expression level of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,

wherein the amplitude, period and phase expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

In one embodiment of the method according to the present invention the computational step further comprises

- fitting a network computational model to the derived characteristic data that comprises a representation of the periodic time course of the expression levels for each of the determined genes, in particular at least two core clock genes, in particular for said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, as well as a representation of the periodic time course of the expression level for at least one, preferably a plurality of further gene(s) included in a gene regulatory network that includes said genes; and/or
- training a machine learning algorithm on the derived characteristic data of the network computational model, particularly optimize in terms of the representation of the periodic time course of the expression level for the at least one further gene.

In particular, the network computational model is built to obtain data for at least one further gene that has not been directly measured in the saliva samples, i.e. the network computational model represents a gene network which contains the clock elements, the metabolic elements and the drug target of the respective drug, as well as further elements, which further elements cannot (or at least not with reasonable effort) be measured particularly in saliva samples. This mathematical modelling may use differential equations and also statistical data.

In one embodiment of the method according to the present invention assessing the timing of administration of said medicament to said subject comprises in the computational step fitting a prediction computational model on data obtained from said fitted periodic functions and/or said network computational model, wherein the prediction computational model is based on machine learning including at least one classification method and/or at least one clustering method, wherein said method(s) are preferably selected from the group comprising: K-nearest neighbor algorithm, unsupervised clustering, deep neural networks, random forest algorithm, and support vector machines.

In one embodiment of the method according to the present invention additional physiological data of the subject are provided for fitting the prediction computational model. Said physiological data maybe selected from the group comprising: body temperature, heart rate, eating/fasting patterns and/or sleep/wake patterns. It will be appreciated that one or more of the aforementioned physiological data or other physiological parameters from the subject may be provided. While such data may be obtained manually by the subject (user) and/or by medical staff, it may be envisioned to obtain at least some of the physiological data by means of a portable electronic device, particularly a wearable, such as a fitness watch, wristband or the like. Vice versa, the result of the method of the present invention may be presented on such wearable device so that the user directly sees e.g. their circadian profile (just like they are used to see other physiological or fitness data, e.g. how long and how fast their jogging was, or how their sleep quality was). Of course, the result may be provided by other electronic devices, like a smartphone, tablet or personal computer.

In one embodiment of the method according to the invention the network computational model and/or the prediction computational model form a personalized model for said subject. The personalization particularly comes from the molecular data, i.e. the measurements of the gene expression which are unique for each person. Additional physiological data like temperature, heart rate, sleep/wake cycles as mentioned above can also be used for personalization. These are all circadian events, too, meaning they vary within 24 hours. While such physiological data may be of additional value, the models, and thus predictions are primarily based on the molecular data, i.e. the gene expressions. It is noted that while the network computational model may be personalized because there is a new model for each new person (using the personal gene expressions), the prediction computational model is not. There is one prediction model relating subject data to prediction for all subjects, and this model should generalize to future subjects without being retrained.

In one embodiment of the method according to the present invention samples of at least two consecutive days of said subject are provided and the gene expression levels of said genes in said samples are determined and used, preferably at least three samples per day, more preferably at least four samples per day.

In one embodiment of the method according to the present invention

- the medicament is Filgrastim and the target gene is *Csf3r* and the indication is acute myeloid leukemia; or
- the medicament is Rituximab and the target gene is selected from the group comprising *Fcgr2b, Ms4a1, and Fcgr3;* and the indication is rheumatoid arthritis and Non-Hodgkin's lymphoma; or
- the medicament is bevacizumab and the target gene is *Fcgr2b, Vegfa, Fcgr3;* and the indication is Colorectal cancer and Non-small cell lung cancer; or
- the medicament is trastuzumab and the target gene is Fcgr2b, Erbb2, Egfr, Fcgr3 and the indication is Breast cancer; or
- the medicament is Imatinib and the target gene is Ptgs1, Kit, Slc22a2, Abcg2, Pdgfra, Pdgfrb, Ddr1, Abca3, Abl1, Ret, Abcbla and the indication is Chronic myeloid leukemia; or
- the medicament is Pemetrexed and the target gene is Tyms, Atic, Gart, Slc29a1 and the indication is Mesothelioma and Non-small cell lung cancer; or
- the medicament is Capecitabine and the target gene is Cda, Tymp, Tyms, Ceslg, Dpyd and the indication is Breast cancer and colorectal cancer; or
- the medicament is Erlotinib (tyrosine kinase inhibitor, anticancer drug) and the target gene is EGFR, Ras/Raf/MAPK, and PIK3/AKT (tumour) and the indication is Tumour inhibition (ZT1 >> ZT13); or
- the medicament is Sunitinib (tyrosine kinase inhibitor, anticancer drug) and the target gene is Cyp3a11 (liver, duodenum, jejunum) abcbla (liver, duodenum, jejunum, lung) and the indication is renal cell cancer and pancreatic neuroendocrine tumours; or
- the medicament is Lapatinib (dual tyrosine kinase inhibitor interrupting the HER2/neu and EGFR pathways, anticancer drug) and the target gene is EGFR/Ras/Raf/MAPK, Errfi1, Dusp1 (liver), Hbegf, Tgf$\alpha$, Eref (liver) and the indication is solid tumours such as breast and lung cancer; or
- the medicament is Roscovitine (seliciclib, CDK inhibitor, anticancer drug) and the target gene is Cyp3a11, Cyp3a13(liver) and the indication is non-small cell lung cancer (NSCLC) and leukemia; or
- the medicament is Everolimus (mTOR inhibitor, anticancer drug, immunosuppressant) and the target gene is mTOR/Fbxw7/P70S6K (tumour) and the indication is breast cancer; or
- the medicament is Irinotecan (Top1 inhibitor, anticancer drug) and the target gene is Ces2, Ugt1a1, abcbla, abcblb (liver and ileum), abcc2 (ileum) and the indication for colorectal cancer, advanced pancreatic cancer and small cell lung cancer; or

- the medicament is Tamoxifen (antiestrogenic, anticancer drug) and the target gene is Cyp2d10, Cyp2d22, Cyp3a11 (liver) and the indication is breast cancer; or
- the medicament Bleomycin (toxicant and anticancer drug) and the target gene is NRF2/glutathione antioxidant defence and the indication is pulmonary fibrosis, palliative treatment in the management malignant neoplasm (trachea, bronchus, lung), squamous cell carcinoma, and lymphomas.

The sequence of Csf3r comprises SEQ ID No.15

> ENST00000373103.5 Csf3r-203 cdna:protein_coding

```
ATGGCAAGGCTGGGAAACTGCAGCCTGACTTGGGCTGCCCTGATCATCCTGCTGCTCCCC
GGAAGTCTGGAGGAGTGCGGGCACATCAGTGTCTCAGCCCCCATCGTCCACCTGGGGGAT
CCCATCACAGCCTCCTGCATCATCAAGCAGAACTGCAGCCATCTGGACCCGGAGCCACAG
ATTCTGTGGAGACTGGGAGCAGAGCTTCAGCCCGGGGGCAGGCAGCAGCGTCTGTCTGAT
GGGACCCAGGAATCTATCATCACCCTGCCCCACCTCAACCACACTCAGGCCTTTCTCTCC
TGCTGCCTGAACTGGGGCAACAGCCTGCAGATCCTGGACCAGGTTGAGCTGCGCGCAGGC
TACCCTCCAGCCATACCCCACAACCTCTCCTGCCTCATGAACCTCACAACCAGCAGCCTC
ATCTGCCAGTGGGAGCCAGGACCTGAGACCCACCTACCCACCAGCTTCACTCTGAAGAGT
TTCAAGAGCCGGGGCAACTGTCAGACCCAAGGGGACTCCATCCTGGACTGCGTGCCCAAG
GACGGGCAGAGCCACTGCTGCATCCCACGCAAACACCTGCTGTTGTACCAGAATATGGGC
ATCTGGGTGCAGGCAGAGAATGCGCTGGGGACCAGCATGTCCCCACAACTGTGTCTTGAT
CCCATGGATGTTGTGAAACTGGAGCCCCCCATGCTGCGGACCATGGACCCCAGCCCTGAA
GCGGCCCCTCCCCAGGCAGGCTGCCTACAGCTGTGCTGGGAGCCATGGCAGCCAGGCCTG
CACATAAATCAGAAGTGTGAGCTGCGCCACAAGCCGCAGCGTGGAGAAGCCAGCTGGGCA
CTGGTGGGCCCCCTCCCCTTGGAGGCCCTTCAGTATGAGCTCTGCGGGCTCCTCCCAGCC
```

ACGGCCTACACCCTGCAGATACGCTGCATCCGCTGGCCCCTGCCTGGCCACTGGAGCGAC
TGGAGCCCCAGCCTGGAGCTGAGAACTACCGAACGGGCCCCCACTGTCAGACTGGACACA
TGGTGGCGGCAGAGGCAGCTGGACCCCAGGACAGTGCAGCTGTTCTGGAAGCCAGTGCCC
CTGGAGGAAGACAGCGGACGGATCCAAGGTTATGTGGTTTCTTGGAGACCCTCAGGCCAG
GCTGGGGCCATCCTGCCCCTCTGCAACACCACAGAGCTCAGCTGCACCTTCCACCTGCCT
TCAGAAGCCCAGGAGGTGGCCCTTGTGGCCTATAACTCAGCCGGGACCTCTCGTCCCACT
CCGGTGGTCTTCTCAGAAAGCAGAGGCCCAGCTCTGACCAGACTCCATGCCATGGCCCGA
GACCCTCACAGCCTCTGGGTAGGCTGGGAGCCCCCCAATCCATGGCCTCAGGGCTATGTG
ATTGAGTGGGGCCTGGGCCCCCCCAGCGCGAGCAATAGCAACAAGACCTGGAGGATGGAA
CAGAATGGGAGAGCCACGGGGTTTCTGCTGAAGGAGAACATCAGGCCCTTTCAGCTCTAT
GAGATCATCGTGACTCCCTTGTACCAGGACACCATGGGACCCTCCCAGCATGTCTATGCC
TACTCTCAAGAAATGGCTCCCTCCCATGCCCCAGAGCTGCATCTAAAGCACATTGGCAAG
ACCTGGGCACAGCTGGAGTGGGTGCCTGAGCCCCCTGAGCTGGGGAAGAGCCCCCTTACC
CACTACACCATCTTCTGGACCAACGCTCAGAACCAGTCCTTCTCCGCCATCCTGAATGCC
TCCTCCCGTGGCTTTGTCCTCCATGGCCTGGAGCCCGCCAGTCTGTATCACATCCACCTC
ATGGCTGCCAGCCAGGCTGGGGCCACCAACAGTACAGTCCTCACCCTGATGACCTTGACC
CCAGAGGGGTCGGAGCTACACATCATCCTGGGCCTGTTCGGCCTCCTGCTGTTGCTCACC
TGCCTCTGTGGAACTGCCTGGCTCTGTTGCAGCCCCAACAGGAAGAATCCCCTCTGGCCA
AGTGTCCCAGACCCAGCTCACAGCAGCCTGGGCTCCTGGGTGCCCACAATCATGGAGGAG
CTGCCCGGACCCAGACAGGGACAGTGGCTGGGGCAGACATCTGAAATGAGCCGTGCTCTC
ACCCCACATCCTTGTGTGCAGGATGCCTTCCAGCTGCCCGGCCTTGGCACGCCACCCATC
ACCAAGCTCACAGTGCTGGAGGAGGATGAAAAGAAGCCGGTGCCCTGGGAGTCCCATAAC
AGCTCAGAGACCTGTGGCCTCCCCACTCTGGTCCAGACCTATGTGCTCCAGGGGGACCCA
AGAGCAGTTTCCACCCAGCCCCAATCCCAGTCTGGCACCAGCGATCAGGTCCTTTATGGG
CAGCTGCTGGGCAGCCCCACAAGCCCAGGGCCAGGGCACTATCTCCGCTGTGACTCCACT
CAGCCCCTCTTGGCGGGCCTCACCCCCAGCCCCAAGTCCTATGAGAACCTCTGGTTCCAG
GCCAGCCCCTTGGGGACCCTGGTAACCCCAGCCCCAAGCCAGGAGGACGACTGTGTCTTT
GGGCCACTGCTCAACTTCCCCCTCCTGCAGGGGATCCGGGTCCATGGGATGGAGGCGCTG
GGGAGCTTCTAG

The sequence of Fcgr2b comprises SEQ ID No.16

> ENST00000358671.9 Fcgr2b-202 cdna:protein_coding

ATGGGAATCCTGTCATTCTTACCTGTCCTTGCCACTGAGAGTGACTGGGCTGACTGCAAG
TCCCCCCAGCCTTGGGGTCATATGCTTCTGTGGACAGCTGTGCTATTCCTGGCTCCTGTT
GCTGGGACACCTGCAGCTCCCCCAAAGGCTGTGCTGAAACTCGAGCCCCAGTGGATCAAC
GTGCTCCAGGAGGACTCTGTGACTCTGACATGCCGGGGGACTCACAGCCCTGAGAGCGAC
TCCATTCAGTGGTTCCACAATGGGAATCTCATTCCCACCCACACGCAGCCCAGCTACAGG
TTCAAGGCCAACAACAATGACAGCGGGGAGTACACGTGCCAGACTGGCCAGACCAGCCTC
AGCGACCCTGTGCATCTGACTGTGCTTTCTGAGTGGCTGGTGCTCCAGACCCCTCACCTG
GAGTTCCAGGAGGGAGAAACCATCGTGCTGAGGTGCCACAGCTGGAAGGACAAGCCTCTG
GTCAAGGTCACATTCTTCCAGAATGGAAAATCCAAGAAATTTTCCCGTTCGGATCCCAAC
TTCTCCATCCCACAAGCAAACCACAGTCACAGTGGTGATTACCACTGCACAGGAAACATA
GGCTACACGCTGTACTCATCCAAGCCTGTGACCATCACTGTCCAAGCTCCCAGCTCTTCA
CCGATGGGGATCATTGTGGCTGTGGTCACTGGGATTGCTGTAGCGGCCATTGTTGCTGCT
GTAGTGGCCTTGATCTACTGCAGGAAAAAGCGGATTTCAGCTCTCCCAGGATACCCTGAG

TGCAGGGAAATGGGAGAGACCCTCCCTGAGAAACCAGCCAATCCCACTAATCCTGATGAG
GCTGACAAAGTTGGGGCTGAGAACACAATCACCTATTCACTTCTCATGCACCCGGATGCT
CTGGAAGAGCCTGATGACCAGAACCGTATTTAG

The sequence of Ms4a1 comprises SEQ ID No.17

> ENST00000345732.9 Ms4a1-201 cdna:protein_coding

ATGACAACACCCAGAAATTCAGTAAATGGGACTTTCCCGGCAGAGCCAATGAAAGGCCCT
ATTGCTATGCAATCTGGTCCAAAACCACTCTTCAGGAGGATGTCTTCACTGGTGGGCCCC
ACGCAAAGCTTCTTCATGAGGGAATCTAAGACTTTGGGGGCTGTCCAGATTATGAATGGG
CTCTTCCACATTGCCCTGGGGGGTCTTCTGATGATCCCAGCAGGGATCTATGCACCCATC
TGTGTGACTGTGTGGTACCCTCTCTGGGGAGGCATTATGTATATTATTTCCGGATCACTC
CTGGCAGCAACGGAGAAAAACTCCAGGAAGTGTTTGGTCAAAGGAAAAATGATAATGAAT
TCATTGAGCCTCTTTGCTGCCATTTCTGGAATGATTCTTTCAATCATGGACATACTTAAT
ATTAAAATTTCCCATTTTTTAAAAATGGAGAGTCTGAATTTTATTAGAGCTCACACACCA
TATATTAACATATACAACTGTGAACCAGCTAATCCCTCTGAGAAAAACTCCCCATCTACC
CAATACTGTTACAGCATACAATCTCTGTTCTTGGGCATTTTGTCAGTGATGCTGATCTTT
GCCTTCTTCCAGGAACTTGTAATAGCTGGCATCGTTGAGAATGAATGGAAAAGAACGTGC
TCCAGACCCAAATCTAACATAGTTCTCCTGTCAGCAGAAGAAAAAAAAGAACAGACTATT
GAAATAAAAGAAGAAGTGGTTGGGCTAACTGAAACATCTTCCCAACCAAAGAATGAAGAA
GACATTGAAATTATTCCAATCCAAGAAGAGGAAGAAGAAGAAACAGAGACGAACTTTCCA
GAACCTCCCCAAGATCAGGAATCCTCACCAATAGAAAATGACAGCTCTCCTTAA

The sequence of Fcgr3b comprises SEQ ID No.18

> ENST00000650385.1 Fcgr3b-208 cdna:protein_coding

ATGTGGCAGCTGCTCCTCCCAACTGCTCTGCTACTTCTAGTTTCAGCTGGCATGCGGACT
GAAGATCTCCCAAAGGCTGTGGTGTTCCTGGAGCCTCAATGGTACAGCGTGCTTGAGAAG
GACAGTGTGACTCTGAAGTGCCAGGGAGCCTACTCCCCTGAGGACAATTCCACACAGTGG
TTTCACAATGAGAACCTCATCTCAAGCCAGGCCTCGAGCTACTTCATTGACGCTGCCACA
GTCAACGACAGTGGAGAGTACAGGTGCCAGACAAACCTCTCCACCCTCAGTGACCCGGTG
CAGCTAGAAGTCCATATCGGCTGGCTGTTGCTCCAGGCCCCTCGGTGGGTGTTCAAGGAG
GAAGACCCTATTCACCTGAGGTGTCACAGCTGGAAGAACACTGCTCTGCATAAGGTCACA
TATTTACAGAATGGCAAAGACAGGAAGTATTTTCATCATAATTCTGACTTCCACATTCCA
AAAGCCACACTCAAAGATAGCGGCTCCTACTTCTGCAGGGGGCTTGTTGGGAGTAAAAAT
GTGTCTTCAGAGACTGTGAACATCACCATCACTCAAGGTTTGGCAGTGTCAACCATCTCA
TCATTCTCTCCACCTGGGTACCAAGTCTCTTTCTGCTTGGTGATGGTACTCCTTTTTGCA
GTGGACACAGGACTATATTTCTCTGTGAAGACAAACATTTGA

The sequence of Top1 comprises SEQ ID No.19

> ENST00000361337.3 Top1-201 cdna:protein_coding

ATGAGTGGGGACCACCTCCACAACGATTCCCAGATCGAAGCGGATTTCCGATTGAATGAT
TCTCATAAACACAAAGATAAACACAAAGATCGAGAACACCGGCACAAAGAACACAAGAAG
GAGAAGGACCGGGAAAAGTCCAAGCATAGCAACAGTGAACATAAAGATTCTGAAAAGAAA
CACAAAGAGAAGGAGAAGACCAAACACAAAGATGGAAGCTCAGAAAAGCATAAAGACAAA
CATAAAGACAGAGACAAGGAAAAACGAAAAGAGGAAAAGGTTCGAGCCTCTGGGGATGCA
AAAATAAAGAAGGAGAAGGAAAATGGCTTCTCTAGTCCACCACAAATTAAAGATGAACCT
GAAGATGATGGCTATTTTGTTCCTCCTAAAGAGGATATAAAGCCATTAAAGAGACCTCGA
GATGAGGATGATGCTGATTATAAACCTAAGAAAATTAAAACAGAAGATACCAAGAAGGAG
AAGAAAAGAAAACTAGAAGAAGAAGAGGATGGTAAATTGAAAAAACCCAAGAATAAAGAT
AAAGATAAAAAAGTTCCTGAGCCAGATAACAAGAAAAAGAAGCCGAAGAAAGAAGAGGAA
CAGAAGTGGAAATGGTGGGAAGAAGAGCGCTATCCTGAAGGCATCAAGTGGAAATTCCTA
GAACATAAAGGTCCAGTATTTGCCCCACCATATGAGCCTCTTCCAGAGAATGTCAAGTTT
TATTATGATGGTAAAGTCATGAAGCTGAGCCCCAAAGCAGAGGAAGTAGCTACGTTCTTT
GCAAAAATGCTCGACCATGAATATACTACCAAGGAAATATTTAGGAAAAATTTCTTTAAA
GACTGGAGAAAGGAAATGACTAATGAAGAGAAGAATATTATCACCAACCTAAGCAAATGT
GATTTTACCCAGATGAGCCAGTATTTCAAAGCCCAGACGGAAGCTCGGAAACAGATGAGC
AAGGAAGAGAAACTGAAAATCAAAGAGGAGAATGAAAAATTACTGAAAGAATATGGATTC
TGTATTATGGATAACCACAAAGAGAGGATTGCTAACTTCAAGATAGAGCCTCCTGGACTT
TTCCGTGGCCGCGGCAACCACCCCAAGATGGGCATGCTGAAGAGACGAATCATGCCCGAG
GATATAATCATCAACTGTAGCAAAGATGCCAAGGTTCCTTCTCCTCCTCCAGGACATAAG
TGGAAAGAAGTCCGGCATGATAACAAGGTTACTTGGCTGGTTTCCTGGACAGAGAACATC
CAAGGTTCCATTAAATACATCATGCTTAACCCTAGTTCACGAATCAAGGGTGAGAAGGAC
TGGCAGAAATACGAGACTGCTCGGCGGCTGAAAAAATGTGTGGACAAGATCCGGAACCAG
TATCGAGAAGACTGGAAGTCCAAAGAGATGAAAGTCCGGCAGAGAGCTGTAGCCCTGTAC
TTCATCGACAAGCTTGCTCTGAGAGCAGGCAATGAAAAGGAGGAAGGAGAAACAGCGGAC
ACTGTGGGCTGCTGCTCACTTCGTGTGGAGCACATCAATCTACACCCAGAGTTGGATGGT
CAGGAATATGTGGTAGAGTTTGACTTCCTCGGGAAGGACTCCATCAGATACTATAACAAG
GTCCCTGTTGAGAAACGAGTTTTTAAGAACCTACAACTATTTATGGAGAACAAGCAGCCC
GAGGATGATCTTTTTGATAGACTCAATACTGGTATTCTGAATAAGCATCTTCAGGATCTC
ATGGAGGGCTTGACAGCCAAGGTATTCCGTACATACAATGCCTCCATCACGCTACAGCAG
CAGCTAAAAGAACTGACAGCCCCGGATGAGAACATCCCAGCGAAGATCCTTTCTTATAAC
CGTGCCAATCGAGCTGTTGCAATTCTTTGTAACCATCAGAGGGCACCACCAAAAACTTTT
GAGAAGTCTATGATGAACTTGCAAACTAAGATTGATGCCAAGAAGGAACAGCTAGCAGAT
GCCCGGAGAGACCTGAAAAGTGCTAAGGCTGATGCCAAGGTCATGAAGGATGCAAAGACG
AAGAAGGTAGTAGAGTCAAAGAAGAAGGCTGTTCAGAGACTGGAGGAACAGTTGATGAAG
CTGGAAGTTCAAGCCACAGACCGAGAGGAAAATAAACAGATTGCCCTGGGAACCTCCAAA
CTCAATTATCTGGACCCTAGGATCACAGTGGCTTGGTGCAAGAAGTGGGGTGTCCCAATT
GAGAAGATTTACAACAAAACCCAGCGGGAGAAGTTTGCCTGGGCCATTGACATGGCTGAT
GAAGACTATGAGTTTTAG

In one embodiment of the method according to the present invention each of the time points at which said samples are obtained are at least 2-4 hours apart, and/or wherein the time points span a time period of at least 12 hours of the day, wherein preferably the time points are 4 hours apart, e.g. at 9h, 13h, 17h and 21h. The times can be chosen based on individual daily habits of the subject, such as the individual wake up time. For instance, for someone who usually wakes up at 11h, the sampling may start at 11h (and continue accordingly at 15h, 19h and 23h).

Previous studies focused on predicting the circadian time which means a 24 hours-rhythms. However, given that the prediction of the circadian time is in an application used for a second prediction, the prediction of the timing of behavior, the error accumulates with each prediction. The present invention instead relies on a direct measurement of the behavioral relevant timing directly based on the genetic expression. That means if the genes have a 20h, or 30h or 12h rhythm in expression, the method of the present invention would also be able to detect that. These would be non-circadian rhythms and include infradian and ultradian rhythms. Thus, the present invention assesses and monitors the rhythmic profile. The rhythmic profile could be a circadian or non-circadian rhythm.

According to the present invention "assessing the circadian or non-circadian rhythm" or "assessing the health status" also includes "monitoring the circadian or non-circadian rhythm" or "monitoring the health status". "Monitoring" means at least twice "assessing".

As an objective measure, gene expression may be quantified four times a day (the times mentioned in this disclosure serve as an e.g. of possible sampling times), two days in a row. In particular, four samples of saliva may be taken on two consecutive days, and the gene expression of selected genes in accordance with the present invention is determined in each of the samples. While other studies have focused on a prediction of circadian time (exact estimation of precise internal time), with the aim to allow for a subsequent prediction of the optimal time for a behavior, such as high sports performance, the present invention focuses on a direct prediction of the relevant timing including a circadian profile, without the deviation through circadian time. This means previous studies attempted to tell the exact internal time. The present invention provides a full 24h profile, it may provide a 48h profile, if measured during two consecutive days, each day e.g. 4 saliva samples are taken. If more samples are taken over more days longer profiles may be provided.

Subject matter of the present invention is a kit for sampling saliva for use in a method according to the method of the present invention comprising sampling tubes for receiving the samples of saliva, wherein each of the sampling tubes contains RNA protect reagent and is configured to enclose one of the samples of saliva to be taken together with the reagent, wherein preferably each of the sampling tubes is labelled with the time point at which the respective sample is to be taken and/or includes an indication about the amount of saliva for one sample.

The kit may further comprise at least one of a box, a cool pack, at least one form including instructions and/or information about the kit and the method for the subject.

In one embodiment of the kit the RNA protect reagent is selected from the group comprising. EDTA disodium, dihydrate; sodium citrate trisodium salt, dihydrate; ammonium sulfate, powdered; sterile water, wherein a single reagent or a combination of different reagents may be used.

In one embodiment of the kit said sampling tubes are configured to receive a sample of saliva of 1 mL in addition to 1 mL of the RNA protect reagent. The sampling tubes may be at least 2 mL tubes, preferably at least 3 mL tubes, more preferably at least 4 mL tubes, still preferably at least 5 mL tubes. While the size for the tubes of 2 mL would be sufficient, it may be more convenient for collecting the saliva samples if the tubes are bigger, such as e.g. 5 mL tubes. In order to collect the required number of samples, the kit may at least six sampling tubes, preferably at least eight sampling tubes (i.e. at least three and four, respectively, samples for two days).

While the kit is used for collecting the samples, it may be designed to be used also for storage and transport of the samples. For this purpose, it is advantageous to have a cool pack in the kit. For instance, if someone is outside and needs to collect the samples, the samples could be stored at room temperature anyway for a few hours, or if one know that there will be no fridge for the next two days, one could still freeze the cool pack before the sampling, then place the cool pack in the box and sample as needed, since the box would remain cold for several hours (maybe even for two days, depending on the outside temperature). After the sampling is completed, the same box can be used to send the samples back to a lab, if applicable with all the forms inside as well (it may be required to pack the box in a post box for sending, which however may be enough for preparing the kit to be sent).

Subject matter of the present invention is further a method of RNA extraction for gene expression analysis from a sampling tube for receiving the sample of saliva comprising the steps of:

- Separating the sample of saliva by means of centrifugal force and generating a cell pellet
- Separating the pellet from supernatant and homogenizing the pellet in an acid-guanidinium-phenol based reagent, such as TRIzol.
- Adding an organic compound (such as chloroform) and mixing said homogenate with a shaking device (e.g. vortexer) and obtaining a mixture.
- Separating said mixture by means of centrifugal force resulting in a solution having more than one phase with an upper aqueous phase comprising the RNA to be extracted.
- Removing said RNA to be extracted in said aqueous phase from said solution having more than one phase.

In one embodiment of the method of RNA extraction for gene expression analysis from a sampling tube for receiving the sample of saliva according to the present invention comprising additionally the steps of:

- Performing optionally a processing step for preparation of the extracted RNA samples for determining gene expression.

- Performing gene expression analysis.

In one embodiment of the method of RNA extraction for gene expression analysis from a sampling tube for receiving the sample of saliva according to the present invention, wherein the extracted RNA is reverse described into cDNA and subsequently amplified.

**Detailed description of the invention**

Exemplary embodiments of the present invention will be explained by way of example with reference to the drawings. In the drawings:

Fig. 1 illustrates the circadian core-clock network;

Fig. 2 illustrates two examples of fits of saliva data to a core-clock mathematical model;

Fig. 3 illustrates time-course measurements of unstimulated saliva show fluctuations in gene expression across 45 hours;

Fig. 4 shows a scheme depicting molecular PK-PD of irinotecan (CPT11) (Dulong et al. 2015);

Fig. 5 illustrates a model extension (as an e.g. for the treatment with the drug irinotecan), in which the core-clock network is complemented with additional genes associated with the irinotecan metabolism and provides as an output cytotoxicity which can be taken to predict and optimize the time of treatment of cancer patients, for this particular drug. Similar model extensions are performed for each particular drug mentioned in the application;

Fig. 6 illustrates a 24h-period harmonic regression for experimental data from SW480 cell lines;

Fig. 7 illustrates a simulated gene expression, obtained with the mathematical model, fitted to experimental data from SW480 cell lines;

Fig. 8 shows a schematic representation for the rationale of cancer chronotherapy, applying the described experimental and computational methods;

Fig. 9 illustrates an example for a personalized model fit of core-clock genes (a) and the genes involved in the irinotecan metabolism (b) based on the experimental data. The personalized times for the particular individual (meal timing, sleep and sleep/awake times are marked for better interpretation of the results);

Fig. 10 illustrates the computed prediction result for the temporal dynamics of two additional genes involved in the irinotecan metabolism (a) the optimal treatment time with irinotecan (b) based on the gene expressions from Fig. 9 which can be taken to predict and optimize the time of treatment of cancer patients, for this particular drug. Similar model extensions are performed for each particular drug mentioned in the application;

Fig. 11 illustrates an example for a core-clock network extended with Irinotecan-treatment relevant genetic network, which can be taken to predict and optimize the time of treatment of cancer patients, for this particular drug. Similar model extensions are performed for each particular drug mentioned in the application;

Fig. 12illustrates how BMAL1- and HKDC1-KD leads to metabolic changes in SW480 colorectal cancer cells and altered drug response in a time-dependent manner; see also The Circadian Clock Regulates Metabolic Phenotype Rewiring Via HKDC1 and Modulates Tumor Progression and Drug Response in Colorectal Cancer. Fuhr L, El-Athman R, Scrima R, Cela O, Carbone A, Knoop H, Li Y, Hoffmann K, Laukkanen MO, Corcione F, Steuer R, Meyer TF, Mazzoccoli G, Capitanio N, Relogio A*. EBioMedicine. 2018

Fig. 13 illustrates how BMAL1 promoter activity is altered after treating human cancer cells with different anti-cancer drugs;

Fig. 14 illustrates how BMAL1 promoter activity is altered after treating different human cancer cells with anti-cancer drug Irinotecan;

Fig. 15 illustrates how cell proliferation for an exemplified for a human cancer cell type is affected by Cisplatin treatment in a time-dependent manner;

Fig. 16 illustrates how cytotoxicity assays for time-dependent gemcitabine treatment for two different types of human pancreatic cancer cell lines. The downregulation of core-clock genes and the alteration of SMAD4 expression impacts on drug response in Panc1 and AsPC1 cells. Cytotoxicity assays were performed using either an IncuCyte® Red Cytotoxicity Reagent or NucLight Rapid Red Reagent for the IncuCyte S3 Live Cell System Analysis. (A - C) 0h after cell synchronization, gemcitabine was dissolved in IncuCyte® Red Cytotoxicity Reagent and added into PDA cells containing SMAD4-KD, -OE and corresponding empty vector constructs (Panc1, 9.5$\mu$M and AsPC1, 23.9$\mu$M). A cytotoxic index was calculated on IncuCyte S3 Live-Cell Analysis System in red phases. 72h after treatment, the number of viable cells per well was quantified with IncuCyte S3 Live-Cell Analysis System. Compared to the shCtrl (mean $\pm$ SEM, n = 6, one-way ANOVA, *$p < 0.05$, **$p < 0.01$, ***$p < 0.001$).

Fig. 17 illustrates BMAL1 and PER2 expression display variation during the day in human blood, hair and saliva samples.

Fig. 18 illustrates Gene expression of BMAL1 and AKT1 covary.

Fig. 19 illustrates HST base line measurements.

Fig. 20 illustrates Myotonometric analysis shows daily variation in muscle tone (frequency, F) for female and male participants.

Figure 21 illustrates Standard deviations of normalized sports and muscle tone data

The aim of the invention is to predict, optimal timing of behavior, more specifically the timing of cancer treatment, possibly to monitor (over time) the circadian profile and adjust timing if needed. Previous studies focused on predicting the circadian time. However, given that the prediction of the circadian time is in an application used for a second prediction, the prediction of the timing of behavior, the error accumulates with each prediction. The present invention instead relies on a direct measurement of the behavioral relevant timing directly based on the genetic expression.

As an objective measure, gene expression may be quantified four times a day (the times mentioned in this disclosure serve as an e.g. of possible sampling times), two days in a row. In particular, four samples of saliva may be taken on two consecutive days, and the gene expression of selected genes in accordance with the present invention is determined in each of the samples. While other studies have focused on a prediction of circadian time, with the aim to allow for a subsequent prediction of the optimal time for a behavior, such as timing of administering a certain medicament, the present invention focuses on a direct prediction of the relevant timing, without the deviation through circadian time.

The computational analysis of the measured gene expressed obtained from the saliva samples as set forth above can be separated into three different approaches, which will be discussed in the next sections. First, experimental data can be fitted with a periodic function, in order to establish oscillatory behavior and extract oscillation properties. Machine learning can then be used to predict the timing of behavior based on the gene expression. Modeling the molecular network underlying the circadian rhythm as well as the behavior under consideration can add information. Background and general considerations in view of the present invention and the overall process will be explained first. Following, the procedure according to the present invention will be described with respect to the specific application.

A general problem in chronobiology is the screening for circadian oscillations in data, such as in the series of eight data points obtained from the saliva samples. It has to be determined whether the observed variation is due to some circadian rhythm, or only due to noise. To distinguish oscillating from non-oscillating measures, a periodic, non-constant function is fit to the data, and if the fit is significant, the measure is considered oscillatory. Successful fits allow to read off the oscillation phase, amplitude and period. Fitting the oscillatory data by curve fitting is described below.

If a trigonometric function is fit to the data, this is called harmonic regression, which may be done as set forth in the following. It will be appreciated that this is described by way of example only. Below, other approaches are briefly outlined. Circadian rhythmicity of genes may be tested (significance e.g. bounded by a fit with p-value $< 0.05$) and circadian parameters (phase and relative amplitude) may be determined for sample sets with at least 7 data points (3 hours sampling interval) for a period range of 20 to 28 hours with a 0.1 hour sampling interval by fitting a linear sine-cosine function to the time-course data ($\Delta\Delta$CT normalized to the mean of all time points), for instance using known tools, e.g. the R package HarmonicRegression (Luck et al. 2014). The harmonic regression procedure fits the model $y(t) = m + a\cdot\cos(\omega t) + b\cdot\sin(\omega t)$ in order to estimate absolute amplitudes ($A = \sqrt{(a2 + b2)}$) and phases ($\varphi = a\cdot tan2(b,a)$) along with confidence intervals and p-values (Luck et al. 2014). The fit uses a least-squares minimization. Extensions to this fit method are reviewed in as cosinor-based rhythmometry in (Cornelissen 2014).

A combination of sine waves are also used by other rhythmicity detection methods (Halberg et al., 1967; Straume, 2004; Wichert et al., 2004; Wijnen et al., 2005; Thaben and Westermark, 2014). Yet, Fourier-based methods can have the drawback that they require evenly sampled data. Other alternatives are named in the following. It will be appreciated that the invention is not limited to these packages but any other suitable method for fitting a periodic function to the measured gene expression data may be applied.

The software-packages RAIN (a robust nonparametric method for the detection of rhythms of prespecified periods in biological data that can detect arbitrary wave forms (Thaben and Westermark 2014)), which improves on older methods: a nonparametric method implemented as the program "JTK_CYCLE", which assumes symmetric curves (Hughes et al., 2010), as well as its improvement eJTK_CYCLE that includes multiple hypothesis testing and more general waveforms (Hutchison et al. 2015)[Ref: Hutchison AL, Maienschein-Cline M, Chiang AH, et al. Improved statistical methods enable greater sensitivity in rhythm detection for genome-wide data. PLoS Comput Biol 2015;11:e1004094.], while the HAYSTACK method (Michael et al., 2008) can also detect chain saw type rhythmicity, but relies on a small set of predefined wave form alternatives and is thus not really general.

BIO_CYCLE: "We first curate several large synthetic and biological time series datasets containing labels for both periodic and aperiodic signals. We then use deep learning methods to develop and train BIO_CYCLE, a system to robustly estimate which signals are periodic in high-throughput circadian experiments, producing estimates of amplitudes, periods, phases, as well as several statistical significance measures." (Agostinelli et al. 2016).

A modified version of the empirical Bayes periodicity test to detect periodic expression patterns (Kocak and Mozhui 2020). Their results demonstrate that this approach can capture cyclic patterns from relatively noisy expression data sets.

Especially to find higher harmonics, some studies have exploited Fisher's G-test and COSOPT jointly to recognize rhythmic transcripts, classified, depending on the length of the oscillation period, as circadian (24 ± 4 h) and ultradian (12 ± 2 h and 8 ± 1 h) (Hughes et al. 2009; Genov et al. 2019).

Once the curve fitting to the measured data points has been done, machine learning methods are applied to predict circadian time for human subjects, which has in principle been proposed by several studies. Some example studies are outlined to provide background for the process of the present invention, which will be described in detail thereafter.

While the present invention focusses on studies based on gene expression, continues measures of light exposure and skin temperature as well as metabolites from blood or breath sampling can be used to predict circadian time (Kolodyazhniy et al. 2012; Kasukawa et al. 2012; Sinues et al. 2014). Also skin temperature in combination with questionnaires and activity measurements can predict circadian time, by a method called INTime (Komarzynski et al. 2019). The following studies predict circadian time or time-of-the-day from gene expression data extracted from human blood: BioClock (though only mouse data so far) (Agostinelli et al. 2016): Normalization is Z-score data (subtraction of mean and then divided by standard deviation - this removes any amplitude information), their method is a deep neural network, they use BioCycle to derive rhythmicity, and standard gradient descent with momentum to train the network, the original publication uses different tissues but only from mice.

ZeitZeiger (Hughey 2017): Data normalized and batch-normalized. Discretized and scaled spline fits are used to calculate sparse principal components (SPC), predictions based on fitted splines to SPC with maximum likelihood. They use 15 genes from human blood, only two of those are part of the core-clock. Due to the batch-normalization, the incorporation of new data requires retraining of the algorithm, their algorithm was improved for humans. One sample is enough for predictions.

Partial least squares regression (PLSR) (Laing et al. 2017): Training data is batch-corrected and quantile normalization is applied. No batch correction on test set, to prevent the need for retraining whenever new data is added. Their algorithm uses 100 genes out of 26,000 available ones from blood. One sample is enough for predictions, more is better.

TimeSignature (Braun et al. 2018): Mean-normalized genes, algorithm is optimized with a least squares approach plus elastic net for regularization. They use 40 genes from two samples of blood, 12 h or less apart. This study seems to generalize well, it was validated in 3 studies, one of them with a different experimental method to measure gene expression.

BodyTime (Wittenbrink et al. 2018): ZeitZeiger (see above) + NanoString platform (an experimental machine which allows for high quality counts of gene abundance without the need of an amplification step, thus it measures the original abundances). They use 12 genes from human blood, but also get good predictions for as few as 2 genes (one of which is PER2 which also is used in the sports study). Their algorithm is validated in 1 independent study that uses the same method.

TimeTeller, preprint (Vlachou et al. 2020): They aim to predict clock functionality from a single gene sample. Application to breast cancer, showing that their prediction relates to patient survival. Rhythmicity and synchronicity were analyzed to choose a set of 10-16 genes used for the prediction (all core-clock or clock-controlled). Their algorithm is trained with a set of repeated samples and extracts from them the probability to observe a particular gene expression profile given some time t. The prediction inverts this information; they use a maximal likelihood function to predict for a given gene expression profile the time t. A model of the core-clock was used to test their algorithm.

Machine learning can be used to predict some output based on a (high-dimensional) input consisting of a set of so-called features, i.e. the different dimensions of the input space. A set of input-output pairs is used to train the algorithm, i.e. the algorithm performs some kind of optimization that allows it to optimally predict the output based on the input. This set is called training set. To evaluate the performance of the algorithm, it is fed with an independent set of inputs from a so-called test set, while not presenting the associated outputs. The predictions of the algorithm are then compared to the associated outputs, and the number of correct predictions is counted. Several measures can be used to quantify prediction quality; for instance the accuracy, i.e. the number of correct predictions divided by the total number of predictions, may be used.

For small data sets, as typical with human subjects, the separation of the data into two independent training and test sets means that it would not be taken advantage of the full amount of information available for the prediction. The solution is cross-validation, for which the total set is repeatedly separated into different training and test sets. Especially for very small data sets, one can use all but one subjects to form the training set, and test the algorithm only on the left-out subject. This is called leave-one-out cross-validation (sometimes also leave-one-subject-out cross-validation). Given n subjects, the training on all-but-one subjects is repeated n times, such that each subject has been once selected as test set. The accuracy of the prediction is in this case calculated as the number of correct predictions over n.

While the application of machine learning to genetic data is generally known, the benefits and value of the results highly depend on the input data. Thus, the inventors put their focus on the input of the data, both via normalization and presentation of derived features, and also on understanding in detail what information the algorithm uses. Most published studies focus on their machine learning algorithm, why it is best suited for the prediction at hand, while mentioning their data preparation only on the side, and their discussion of the workings of the algorithm is often restricted to a single evaluation approach (for example, showing that a restricted set of genes is most relevant for prediction, but without stating which characteristics of the genes are important).

When comparing the performance of different machine learning algorithm on standard training and test sets, their performance differs often just in a few percent - an order of magnitude hardly relevant for biological data, which often consists of only few samples with a high level of noise compared to other typical machine learning applications. It has been found that the particular algorithm will not make much difference, but what makes a large difference is the way in which the input is prepared for machine learning. Many machine-learning algorithms are optimized for data with zero mean and a standard deviation of one for each dimension individually. Dimension-wise normalization makes however no sense for time-series data, where dimensions are not independent of each other, but where the information on the temporal development can only be accessed by comparing different dimensions.

As mentioned above, eight saliva samples may be collected, preferably distributed over the day, e.g. at 9h, 13h, 17h and 21h over two consecutive days. This results in a feature space with 8 dimensions. Instead of normalizing each of these dimensions independently over all subjects, the data may be normalized by their temporal mean for each subject independently. In addition, this subject-wise mean normalization of each gene has the advantage of keeping the temporal structure of the data intact (phase and relative amplitude of the oscillation), thus preserving this information for the machine learning algorithm. Yet, what is lost by this normalization is the mean values of the oscillations, and thus also their relative expression mean. To preserve this information for the machine-learning algorithm, this may be added as additional features to the feature space. Subject-wise normalization has to be reconsidered when the molecular profile of a subject was measured repeatedly over a longer interval of time. For the example of multiple measurements during disease progression, we have already published one possibility to normalize data from subsequent molecular profiles such that the result can be compared between sampling dates and even between different experimental procedures (Yalçin et al. 2020).

In many cases, machine learning algorithms are considered as "black boxes". However, as the machine learning algorithms are faced with noisy biological data, derived of a system where lots of additional information are available, the approach of the present disclosure is to let the machine learning optimize the prediction, but then to uncover the underlying information flow from input to prediction output, with the aim to double-check the generalizability of the solution found by the machine learning. Optimally, any additional information can be added as either input to the machine learning or as constraints (in form of a cost function), but in a first step, the formulation of these inputs and constraints is more difficult than to take the algorithm and check a posteriori whether any additionally known information is violated.

Once the prediction was done using the complex feature space created in the step explained above, a simplification of the feature space may be carried out. This serves to identify the relevant features. For example when predicting the optimal treatment time it may be tested whether the peak time of the genes would suffice for the prediction. It was found that this was not the case, i.e. the algorithm uses more than this information. In general, dimensionality reduction methods may be used first, which results in fewer, new features that are combinations of the original features. Then it may be tested which combinations of individual original features is sufficient for successful predictions, and compare whether that fits the features which are dominant in the features resulting from dimensionality reduction. This is an important step to understand based on which information the prediction is made by the algorithm, which is relevant to double check its generalizability to new data.

Related to the first point, machine-learning algorithms are preferred which may be called interpretable, i.e. they provide some information on the prediction. Examples for such algorithms are sparse principle components analysis as used as an intermediate step in (Hughey 2017), and partial least squares regression, as used in (Laing et al. 2017). In both cases, the prediction is made based on a combination of the features into few most informative features, and it is for example possible to plot two of them against each other in order to see how the data of the training set and test set is distributed in these features. It is expected that subjects with optimal times that are neighboring are also neighbors in this component space. If this is not the case, the algorithm is unlikely to generalize well.

Then, prediction performance may be benchmarked using a neural network model, which may be used as an approximation for an upper border of prediction performance. Neural networks do not require normalization, as they are universal computing machines and can hence implement the optimal normalization for the problem at hand on their own. However, this is at the same time the problem with neural networks. As they decide for themselves which are the relevant features of the data, there is no controlling whether they use biologically relevant information, or noise information that - by chance - fits the prediction. Furthermore, their high flexibility facilitates overfitting of the data and the resulting algorithm are difficult to interpret, such that we cannot a posteriori enhance our trust in the method by understanding the information flows from input to prediction output. Despite these disadvantages, neural networks may be used at least as benchmark algorithms, to test which performance can be expected when the information is provided without constraints. The present invention aims at providing an algorithm with a performance similar to that of the neural network, but not by means of overfitting the experimental data, as suspected for the neural network, but by means of focusing on the biologically relevant information.

A linear support-vector-machine (SVM) can be used to predict two different outputs based on a high-dimensional input data (see below for details). Linear SVMs are extremely simple compared to the non-linear methods explained above. They have the advantage of a fast implementation, and, as their complexity is low, they are not so prone to overfitting. For these reasons, a linear SVM may be used to predict the optimal treatment timing, and it turned out that this was sufficient for prediction. However, it is noted that the prediction problem was "linearly separable", and as there is no reason to assume that any application is "linearly separable", it may be preferable to use in general non-linear methods. Yet, testing how well a linear model performs compared to the non-linear model can help to benchmark how much complexity is needed for the prediction. For example, if a linear model results in an accuracy of 0.85 and a non-linear model in an accuracy of 0.9, it is probably not worth using the non-linear model for the application, as it performs only slightly better on the test set, but has a larger probability of overfitting the data, which might lead to less performance on a new set of data. If the difference is larger, a non-linear model is likely more appropriate.

As mentioned above, linear support-vector-machine (SVM) can be used to predict two different outputs based on a high-dimensional input data. For training, the linear SVM is fed with multi-dimensional input data and a binary output. The training set consists of n subjects, and the input with *p* dimensions is denoted as $x_i \in R^p$, $i. The output $y_i$ is encoded as -1 for the first type of output and as +1 for the second type of output, $y \in 1, -1^n$. The training of the SVM fits a hyper-plane into the input space such that it separates the two output types as best as possible and such that the distance to the input data points is maximal.

Mathematically, the following minimization problem is solved:

$$\min_{w,b} \frac{1}{2} w^T w + C \sum_{i=1} \max\left(0, y_i(w^T \phi(x_i) + b)\right),$$

where $\phi$ is the identity function, $(w^T\phi(x_i) + b)$ is the predicted output for the *i*st input.

Predictions for some input $x_{test}$ then be calculated as $w^T\phi(x_{test}) + b$ with the *w* and *b* resulting from the above minimization, and compared with the correct output. Leave-one-subject-out cross-validation implies that this step is repeated n times, each time with another participant removed to form the training set.

The sample collection can be performed in almost any location. The samples of saliva are collected at the predetermined points in time in a tube containing an RNA stabilizing reagent followed by RNA extraction as described below. In order to minimize RNA degradation through material transfer, according to a preferred exemplary method an amount of 1mL of unstimulated saliva may be collected directly into a 5mL Eppendorf tube containing 1mL of a non-toxic RNA-stabilizing reagent called RNAprotect Tissue Reagent (Qiagen) which should be mixed immediately to stabilise the saliva RNA. The direct addition of saliva to the RNA stabilizing reagent, which is mixed immediately, was found to generate good quality/quantity RNA suitable for gene expression analysis and by using 5mL tubes instead of 2mL tubes (wider opening for sample collection), the sampling procedure was more comfortable to perform. Other tested sampling protocols had shown to lead to poor quality and quantity of RNA that was not suitable for the downstream application. For example, 200μL saliva was collected in a 50mL tube on ice, which was immediately transferred to a 2mL tube containing 1mL RNA stabilizing reagent followed by RNA. In another protocol, 1000μL saliva was collected in a 50mL tube and processed as described above, in which the extracted RNA did not pass the desired quality/quantity either.

With only four sampling time-points per day (9am, 1pm, 5pm and 9pm) and over two consecutive days, it is possible to assess precise circadian rhythms in gene expression of any individual. For best sampling quality, the individuals should refrain from eating and drinking one hour prior to sample collection. Individuals can optionally wash their mouths with water five minutes before sampling without swallowing the water. The stabilized samples can be kept at room temperature for a few days, optimally at 4□C during several weeks, for posterior molecular analyses via different possible methods, such as RT-qPCR, Nanostring, microarrays, and sequencing.

In one embodiment any other method known by a person skilled in the art to measure gene expression could be used. One could of course do the same with protein expression instead of gene expression in principle.

A method for RNA extraction from saliva samples is provided to effectively extract RNA, preferably using TRIzol (Invitrogen, Thermo Fisher Scientific) and the RNeasy Micro Kit (Qiagen). It has proven to be particularly beneficial to use a combination of both, rather than only one of them (typically either TRIzol or RNeasy Kit is used). For this, the samples were centrifuged at 10,000 x g for 10 min at room temperature to generate cell pellets. The supernatant was removed and the pellets were homogenized with 500μE TRIzol followed by the addition of 100μL chloroform and mixed for 15sec at room temperature. After a 2min incubation at room temperature, the samples were centrifuged at 12,000 x g for 15min at 4°C. The mixture will separate into a lower red phenol-chloroform phase, an interphase, and a colourless upper aqueous phase. The upper aqueous phase contains the RNA, which was transferred into a new 2.0mL microfuge tube using a 1ml pipette with filtered tip, being careful not to transfer any of the interphase layer. After the transfer, the samples were processed according to the manufacturer's instructions of the RNeasy Micro Kit (Qiagen) on a QIAcube Connect device (Qiagen). Finally, the RNA is eluted in RNA-free water and can be used directly for gene expression analysis. It has been developed the secondary purification and elution step of the saliva RNA using RNeasy Micro Kit in order to:

a) Increase sample quality and purity, which is necessary for downstream applications and is otherwise lost with the traditional and commercial methods, since the RNA content in saliva is low.
b) Automate the sample processing and RNA extraction using the QIAcube Connect automation device. With this, it is possible to perform sample handling much faster and reduce sample contamination induced by human errors.

In one embodiment, gene expression analysis is carried out via cDNA synthesis and RT-PCR as follows. For RT-qPCR analysis, the extracted RNA is reverse transcribed into cDNA using M-MLV reverse transcriptase (Invitrogen, Thermo Fisher Scientific), random hexamers (Thermo Fisher Scientific) and dNTPs Mix (Thermo Fisher Scientific). RT-PCR is performed using SsoAdvanced Universal SYBR Green Supermix (Bio-Rad laboratories) in 96-well plates (Bio-Rad laboratories). The RT-PCR reaction is performed using a CFX Connect Real-Time PCR Detection System (Bio-Rad laboratories) using primers from QuantiTect Primer Assay (Qiagen) as well as custom made primers.
The experimental data obtained from the saliva samples as explained above will be further analysed with a computational model in order to provide scientifically justified and personalized suggestions for best timing of medicine intake (wherein applications for other certain daily activities, such as light exposure, sleep, sports, or food intake may be envisioned), to avoid circadian rhythm disruption, and thus enhancing cancer treatment. As will be described in detail below, a mathematical model for the circadian clock is created, which may include core-clock and clock-controlled metabolic genes in about 50 elements, based on which models for relevant gene networks, particularly related to drug metabolism in connection to the circadian clock can be generated. By feeding each network with specific gene expression data obtained from saliva samples, accurate predictions for day-/night-time activities can be generated.
The experimental data obtained from the saliva samples as explained above will be further analysed with a computational model in order to provide scientifically justified and personalized suggestions for best timing of medicine intake (wherein applications for other certain daily activities, such as light exposure, sleep, sports, or food intake may be envisioned), to avoid circadian rhythm disruption, and thus enhancing cancer treatment. As will be described in detail below, a mathematical model for the circadian clock is created, which may include core-clock and clock-controlled metabolic genes in about 50 elements, based on which models for relevant gene networks, particularly related to physical performance in connection to the circadian clock can be generated. By feeding each network with specific gene expression data obtained from saliva samples, accurate predictions for day-/night-time activities can be generated.
Based on the experimental data from the saliva samples, more specifically the measured gene expressions and the resulting fitted oscillatory curves, a core-clock model will be generated, which may include a larger number of other genes that were not included in the measurements but that may be relevant for the desired prediction (this model is also referred to as "network computational model" in this disclosure). The core-clock is located in the brain (suprachiasmatic nucleus) and its oscillations entrain the peripheral clocks. The oscillations result from feedback loops, which can be investigated by experimental and theoretical means.
Rather than relying only on a model for the circadian rhythm that simply shows oscillations such as phase-oscillators, the present disclosure uses a molecular model, which models (part of) the molecular interactions underlying the circadian clock. This is because, as already mentioned, molecular models contain biological information that might be useful for predictions. Molecular models with simple feedback loops models are often based on Goodwin's oscillator, e.g. (Ruoff and Rensing 1996), but the level of detail may also be extensive (Forger and Peskin 2003). According to an exemplary embodiment of the present invention, a model at an intermediate state of complexity is generated, complex enough to capture a significant part of the genetic network, but not too complex, as this may affect fitting of the data to the model without significant overfitting. Relogio et al. have published a model at this level of complexity, with 19 dynamical variables, which is used in the following (Relogio et al. 2011).
Now referring to Fig. 2, two examples of fits of the saliva data to the core-clock model are shown. (Relogio et al. 2011). Left: Saliva data is plotted as dots, including data for BMAL1 and PER2, where the measurements of both measured days within the same 24 hours are plotted, which is then plotted for two consecutive days. The curves result from the model fit. Middle and right: The model contains 19 dynamical variables, 17 are plotted in these two panels.
Fig. 2 illustrates that the dynamical model may restrict the shape of the fitted curves. In this exemplary embodiment, the curves are more complex than a simple sine-cosine function, but they are also not perfectly fitted to the data, as may happen when a spline is used to fit the data, because the model can only produce shapes that result from the interacting dynamics.

The data base for the fit are the experimental, non-logarithmic gene expression values, $2^{\Delta CT}$. In order to get the experimental values on the same scale as the model output (which is on the order of one), the gene expression of both PER2 and BMAL1 are normalized in this exemplary embodiment by the mean of BMAL1 expression; that way the relative amplitude of both genes is preserved. In order to allow for a fairer comparison both the simulated and the experimental data may be normalized by their respective mean BMAL1 expression.

The complexity of the model with around 80 parameters makes a meaningful fit that prevents overfitting challenging. At least one of or a combination of one or more (including all) of the following approaches may be used to fit the model to the saliva data. It will be appreciated that other approaches may be used alternatively or in addition to adjust the model.

To constrain the model to parameter regions in which continuous oscillations occur, a bifurcation analysis may be used to delineate the regions with limit cycles (i.e. continuous oscillations), and restrict the parameter optimization to these regions. This prevents fits that show a (slow) relaxation to a steady state, as the model may be expected to have a stable limit cycle. Considering the bifurcation structure, fits will be faster because less parameters need to be checked and because less simulation time is required to ensure relaxation of the oscillatory behavior.

To minimize the number of parameters with large deviations from the original model, standard regression methods may be used that are also added to the cost function, such as ridge regression, which has proven useful so far.

Based on biological and dynamical considerations, certain parameters may be fixed at the original value and exclude them from the fit. This may be for example done for parameters that show only minor impact on the resulting curves, parameters for which no inter-individual variation is expected (i.e. diffusion constants which result from biochemical properties) and parameters which have been repeatedly measured in experiments for humans.

Finally, least-squares minimization (details see below) may be used to minimize the distance between experimental data and fitted curve. The associated cost function used for least-squared error minimization may be extended with additional terms that can restrict the period (should be between 20 and 28 hours for human material), amplitude (no constant amplitude, e.g.) and the position of peaks and troughs.

According to an exemplary fitting procedure, the two days of gene expression data are interpreted as replicates, and the model is fitted to both data points for 9h, 13h, 17h and 21h at the same time, as indicated by two data points for each time point in the above figure. In order to fit the model to the gene expression data, the model may be run for 72 hours with a time resolution of 0.01 hours. The last 48 hours are used for the analysis. As model and experiments have no common time, all possible time-shifts between experiments and model output are considered (0 up to 24 hours). For each shifted variant of the model output, the least-squares cost function *C* may be calculated between the experimental values $x_{exp}$ and the model output $X_{mod}$ as $C_0 = 2\sqrt{1 + \left(x_{exp} - x_{mod}\right)^2 - 2}$ for both genes (BMAL1 and PER2), and then the time-shift with the minimal summed cost for both genes may be selected. To optimize the fit, a selection of the following additional cost function terms may be added to $C_0$:

- A regression term, that penalizes large parameters: Given the parameter vector $p = (p_i)$ where $i$ is between 1 and the number of parameters, ridge regression adds a term $C_{ridge} = c\sum p_i^2$ to the cost function, with a prefactor $c$ that was set to 1.
- A term that penalizes deviating periods. One may first measure the period $p$ of the model, and then compare it to the standard human circadian period of 24.5 hours: $C_p = c_p(p - 24.5)^2$, with weighting factor $c_p = 1$.
- A term that penalizes the amplitude deviations: For experimental and simulated amplitudes $A_{exp}$ and $A_{sim}$, the cost function is $C_a = c_a(A_{sim} - A_{exp})^2$, with weighting factor $c_a = 0.05$.
- A term that penalizes if the peak or trough position deviates. Peak times of the experimental data $t_{exp}$ and of the model trace with the optimal time-shift applied $t_{sim}$ may be derived and the cost term calculated as $C_{top} = c_t(p - 24.5)^2$, with weighting factor $c_t = 0.1$ for the peaks. The same may be done for the trough, resulting in a cost $C_{down}$, with weighting factor $c_d = 0.05$.

The cost function sums the individual costs weighted by a factor chosen to optimize the influence of each cost. $C_{total} = C_0 + C_{ridge} + C_p + C_a + C_{top} + C_{down}$.

In one embodiment the present invention provides a method which allows assessing circadian rhythm or circadian profile of a subject having cancer and/or assessing a timing of administration of a medicament to said subject having cancer, wherein said method comprises the steps of:

- Providing at least three samples of saliva, more preferably four samples of saliva, from said subject, wherein said samples have been taken at different time points over the day;

- Determining gene expression of the following genes in each of said samples:

  a. of at least two members of the core-clock network in each of said samples, in particular of at least two members of the following genes, of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, and
  b. at least one gene involved in the metabolism of a medicament to be administered to said subject having cancer, including Ces2, and
  c. at least one drug target gene that is a target of the medicament to be administered, and

- Assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

In another embodiment the present invention provides a method, wherein gene expression is determined using a method selected from quantitative PCR (RT-qPCR), NanoString, sequencing and microarray.
In another embodiment the present invention provides a method, wherein gene expression is determined using quantitative PCR (RT-qPCR).
In another embodiment the present invention provides a method, wherein gene expression is determined using NanoString.
In another embodiment the present invention provides a method, which allows assessing the timing of administration of said medicament to said subject comprises predicting gene expression of at least one of said genes and/or at least one further gene involved in the metabolism of said medicament.
In another embodiment the present invention provides a method, wherein the at least one further gene involved in the metabolism of the medicament to be administered is at least one ofUgtlal and Abcb1.
In another embodiment the present invention provides a method, which allows assessing the timing of administration of said medicament to said subject comprises evaluating the predicted gene expression levels and/or evaluating expression phenotypes based on the determined and/or predicted gene expression levels.
In another embodiment the present invention provides a method, which allows assessing the circadian rhythm of said subject comprises in the computational step determining a periodic function for each of said determined genes that approximates said gene expression levels for each of said genes, preferably comprising curve fitting of a non-linear periodic model function to the respective gene expression levels, wherein the curve fitting is preferably carried out by means of harmonic regression.
In another embodiment the present invention provides a method, wherein the computational step comprises processing the determined gene expression levels to derive characteristic data for each of said genes, said processing comprising determining the mean expression level of expression of a gene and normalizing the gene expression levels using the mean expression level.
In another embodiment the present invention provides a method, wherein said characteristic data comprise:

- the amplitude of change of expression of a gene, and/or the amplitude relative to one of the other genes, and/or
- the mean expression level of expression of a gene, and/ or and/or the mean relative to one of the other genes, and/or
- the peak expression level of a gene, and/or the peak relative to one of the other genes., and/or
- the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the amplitudes of change of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the mean expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/ or
- the relative difference of the mean expression levels of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or

- the relative difference of the peak expression levels of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the time of the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
- the relative difference of the times of the peak expression level of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,

wherein the amplitude, period and phase expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

In another embodiment the present invention provides a method, wherein the computational step further comprises

- fitting a network computational model to the derived characteristic data that comprises a representation of the periodic time course of the expression levels for each of said determined genes as well as a representation of the periodic time course of the expression level for at least one, preferably a plurality of further gene(s) included in a gene regulatory network that includes said genes; and/or
- training a machine learning algorithm on the derived characteristic data to form the network computational model, particularly optimize in terms of the representation of the periodic time course of the expression level for the at least one further gene.

In another embodiment the present invention provides a method, which allows assessing the timing of administration of said medicament to said subject comprises in the computational step fitting a prediction computational model on data obtained from said fitted periodic functions and/or said network computational model, wherein the prediction computational model is based on machine learning including at least one classification method and/or at least one clustering method, wherein said method(s) are preferably selected from the group comprising:
K-nearest neighbor algorithm, unsupervised clustering, deep neural networks, random forest algorithm, and support vector machines.

In another embodiment the present invention provides a method, wherein additional physiological data of the subject are provided for fitting the prediction computational model.

In another embodiment the present invention provides a method, wherein the network computational model and/or the prediction computational model form a personalized model for said subject.

In another embodiment the present invention provides a method, wherein samples of at least two consecutive days of said subject are provided and the gene expression levels of said genes in said samples are determined and used, preferably at least four samples per day.

In another embodiment the present invention provides a method, wherein

- the medicament is Filgrastim and the target gene is *Csf3r* and the indication is acute myeloid leukemia; or
- the medicament is Rituximab and the target gene is selected from the group comprising *Fcgr2b, Ms4a1, and Fcgr3;* and the indication is rheumatoid arthritis and Non-Hodgkin's lymphoma; or
- the medicament is bevacizumab and the target gene is *Fcgr2b, Vegfa, Fcgr3;* and the indication is Colorectal cancer and Non-small cell lung cancer; or
- the medicament is trastuzumab and the target gene is Fcgr2b, Erbb2, Egfr, Fcgr3 and the indication is Breast cancer; or
- the medicament is Imatinib and the target gene is Ptgs1, Kit, Slc22a2, Abcg2, Pdgfra, Pdgfrb, Ddr1, Abca3, Abl1, Ret, Abcbla and the indication is Chronic myeloid leukemia; or
- the medicament is Pemetrexed and the target gene is Tyms, Atic, Gart, Slc29a1 and the indication is Mesothelioma and Non-small cell lung cancer; or
- the medicament is Capecitabine and the target gene is Cda, Tymp, Tyms, Ceslg, Dpyd and the indication is Breast cancer and colorectal cancer; or
- the medicament is Erlotinib (tyrosine kinase inhibitor, anticancer drug) and the target gene is EGFR, Ras/Raf/MAPK, and PIK3/AKT (tumour) and the indication is Tumour inhibition (ZT1 >> ZT13); or
- the medicament is Sunitinib (tyrosine kinase inhibitor, anticancer drug) and the target gene is Cyp3a11 (liver, duodenum, jejunum) abcbla (liver, duodenum, jejunum, lung) and the indication is renal cell cancer and pancreatic neuroendocrine tumours; or
- the medicament is Lapatinib (dual tyrosine kinase inhibitor interrupting the HER2/neu and EGFR pathways, anti-cancer drug) and the target gene is EGFR/Ras/Raf/MAPK, Errfi1, Dusp1 (liver), Hbegf, Tgfα, Eref (liver) and the indication is solid tumours such as breast and lung cancer; or
- the medicament is Roscovitine (seliciclib, CDK inhibitor, anticancer drug) and the target gene is Cyp3a11, Cyp3a13(liver) and the indication is non-small cell lung cancer (NSCLC) and leukemia; or
- the medicament is Everolimus (mTOR inhibitor, anticancer drug, immunosuppressant) and the target gene is mTOR/Fbxw7/P70S6K (tumour) and the indication is breast cancer; or
- the medicament is Irinotecan (Top1 inhibitor, anticancer drug) and the target gene is Ces2, Ugt1a1, abcbla, abcblb (liver and ileum), abcc2 (ileum) and the indication for colorectal cancer, advanced pancreatic cancer and small cell lung cancer; or
- the medicament is Tamoxifen (antiestrogenic, anticancer drug) and the target gene is Cyp2d10, Cyp2d22, Cyp3a11 (liver) and the indication is breast cancer; or
- the medicament Bleomycin (toxicant and anticancer drug) and the target gene is NRF2/glutathione antioxidant defence and the indication is pulmonary fibrosis, palliative treatment in the management malignant neoplasm (trachea, bronchus, lung), squamous cell carcinoma, and lymphomas.

In another embodiment the present invention provides a method, wherein each of the time points at which said samples are obtained are at least 2-4 hours apart, and/or wherein the time points span a time period of at least 12 hours of the day, wherein preferably the time points are 4 hours apart, e.g. at 9h, 13h, 17h and 21h.

In another embodiment the present invention provides a kit for sampling saliva, comprising

- sampling tubes for receiving the samples of saliva, wherein each of the sampling tubes contains RNA protect reagent and is configured to enclose one of the samples of saliva to be taken together with the reagent,

wherein preferably each of the sampling tubes is labelled with the time point at which the respective sample is to be taken and/or includes an indication about the amount of saliva for one sample.

In another embodiment the present invention provides a kit, further comprising at least one of:

- a box,
- a cool pack,
- at least one form including instructions and/or information about the kit and the method for the subject.

In another embodiment the present invention provides a kit, wherein the RNA protect reagent is selected from the group comprising EDTA disodium, dihydrate; sodium citrate trisodium salt, dihydrate; ammonium sulfate, powdered; sterile water.

In another embodiment the present invention provides a kit, wherein said sampling tubes are configured to receive a sample of saliva of 1 mL in addition to 1 mL of the RNA protect reagent, wherein the sampling tubes preferably are at least 2 mL tubes, preferably at least 3 mL tubes, more preferably at least 4 mL tubes, still preferably at least 5 mL tubes.

In another embodiment the present invention provides a kit, comprising at least six sampling tubes, preferably at least eight sampling tubes.

In another embodiment the present invention provides a kit for collecting samples of saliva for providing the collected samples of saliva.

In another embodiment the present invention provides a method of RNA extraction for gene expression analysis from a sampling tube for receiving the sample of saliva comprising the steps of:

- Separating the sample of saliva by means of centrifugal force and generating a cell pellet;
- Separating the pellet from supernatant and homogenizing the pellet in an acid-guanidinium-phenol based reagent, preferably TRIzol;
- Adding an organic compound, preferably chloroform, and mixing said homogenate with a shaking device, preferably a vortexer, and obtaining a mixture;
- Separating said mixture by means of centrifugal force resulting in a solution having more than one phase with an upper aqueous phase comprising the RNA to be extracted; and
- Removing said RNA to be extracted in said aqueous phase from said solution having more than one phase.

In another embodiment the present invention provides a method, comprising additionally the steps of:

- Performing optionally a processing step for preparation of the extracted RNA samples for determining gene expression;
- Performing gene expression analysis.

In another embodiment the present invention provides a method, wherein the extracted RNA is reverse described into cDNA and subsequently amplified.

Examples

In view of the aforementioned explanations, an exemplary embodiment for a general workflow to establish a prediction for the best time for a "behavior B" is outlined below. After that, more specific aspects of the workflow are explained for the "behavior B" being the peak time for sport performance.

1. A priori gene selection:
A set of relevant genes is selected: Core-clock genes, saliva specific oscillating genes (which may show stronger oscillations than the core-clock genes in saliva), and a set of genes that should relate to the behavior B (for sports metabolic genes; for cancer treatment-related genes or drug target genes, etc.). To identify the latter, existing databases are scanned for (1) connections to the core-clock in the genetic network, (2) oscillatory behavior (at least for some tissue, potentially from mice or human), (3) expression level in saliva of healthy human samples or saliva from non-healthy people (particularly having the type of cancer to be treated).

2. Establishment of a data set:
Subjects are asked to perform behavior B several times per day, and their performance is recorded. From the same subjects, saliva is sampled for two days 4 times a day.

3. Experimental analysis:
Gene expression is measured from the saliva samples.

4. Computational analysis (see also description above):

i. The gene expression data is screen for oscillatory behavior, non-oscillating genes are excluded from the analysis, see .e.g. Stroebel, A.M., Bergner, M., Reulbach, U., Biermann, T., Groemer, T.W., Klein, I. and Kornhuber, J., 2010. Statistical methods for detecting and comparing periodic data and their application to the nycthemeral rhythm of bodily harm: A population based study. Journal of Circadian Rhythms, 8, p.Art. 10.
ii. The expression data of oscillating genes is used to predict the optimal time for behavior B, as recorded for the subjects. The resulting machine learning algorithm can then be used to predict the optimal time for further subjects for which only saliva data is available.
iii. A core-clock model is supplemented with the genetic network that includes the oscillatory genes identified in step 4i. The model is fitted to the gene expression data.
iv. The model is used to generate data for the prediction of the optimal timing of behavior B. This data has three advantages: 1. The temporal resolution of the data is arbitrarily detailed. 2. In consequence, peak time, phase, amplitude and period can be extracted with more precision. 3. The data includes all genes implemented in the model, which are far more than the genes measured in step 2. With this data as input, step 4ii is repeated, and the performance is compared to the prediction based directly on the data, while accounting for the enhanced potential for overfitting due to a larger amount of processing (more parameters and potentially more data points for the prediction). Although also a simple fit of the data as in step i gives us curves with high temporal resolution, this is not the same as the model fit: A prediction based on the simple fit cannot outperform the prediction from step 4ii, because no information was added. By contrast, the model fit contains information on the biological interaction between the genes (the gene dynamics), which, when added to the prediction, can improve the prediction.
v. Even more important, the model can illuminate the mechanism behind the prediction of step 4ii and iv. This is an important step to enhance trustworthiness of the prediction - the more we understand, the more we can evaluate whether the prediction makes sense (see sports example below: The correlation between the peak times of PER2 and sport performance is likely to explain why PER2 can be used to predict sports performance. Although we have a small sample size of only 10 participants, this gives us confidence that the prediction is not happening by chance, but is really catching some salient feature in the data).
vi. With the idea about the working of the prediction mechanism from step 4v, genes with an even higher expected predictive power can be identified. Those genes can be added to the set from step 1, if the whole workflow is repeated to optimize the prediction even further.

5. A posteriori gene selection:
Based on the computational analysis, the a priori set of genes is stripped to the genes essential for prediction, in order to minimize the cost of the analysis.

6. Commercial application:
People provide saliva samples, and the machine learning algorithm resulting from step 4 is used to predict optimal timing of behavior B based on the restricted set of genes from step 5.

According to the present invention, an individual-based (machine learning) prediction of optimal timing for administering drugs in a cancer treatment is provided. An exemplary embodiment is explained hereinafter, wherein it is referred to the following publications where appropriate.

Time-course measurements of unstimulated saliva show fluctuations in gene expression across 45 hours are exemplarily shown in Fig. 3. (A) Sampling schemes for saliva collection at 8 time points in two consecutive days (Day 1 - 9h, 13h, 17h, 21h; Day 2 - as day 1). (B) Time-course RT-qPCR measurements of human saliva normalized to the mean of all time points (ΔΔCT) of *ARNTL (BMAL1)* (black) and *PER2* (grey dashed) of 15 participants (7 female and 8 male) with a fitted linear sine-cosine function. Furthermore, table C shows the harmonic regression analysis and table D provides additional information on the participants and tests performed.

(Li et al. 2013): "The application of a maximum-a-posteriori Bayesian inference method identified a linear model based on Rev-erba and Bmall circadian expressions that accurately predicted for optimal irinotecan timing. The assessment of the Rev-erba and Bmall regulatory transcription loop in the molecular clock could critically improve the tolerability of chemotherapy through a mathematical model-based determination of host-specific optimal timing." "The optimal circadian timing of an anticancer drug was predicted despite its variation by up to 8-hour along the 24 hours among six mouse categories. This prediction relied on a mathematical model using liver circadian expression of clock genes Rev-erba and Bmall as input data and treatment tolerability as output parameter." This predicts toxicity for mice and as the database is extremely small, the authors doubt the generalizability of the prediction. The model is no molecular model, as in our case, but just a linear model for the prediction, not meant to model biological information.

(Dulong et al. 2015): "Irinotecan cytotoxicity was directly linked to clock gene BMAL1 expression: The least apoptosis resulted from drug exposure near BMAL1 mRNA nadir ($P < 0.001$), whereas clock silencing through siBMAL1 exposure ablated all the chronopharmacology mechanisms. Mathematical modeling highlighted circadian bioactivation and detoxification as the most critical determinants of irinotecan chronopharmacology." In this study, the circadian variation does not result from an explicit model of the core-clock, as in our case, but the circadian variation is modeled as a time-dependent cosine function fitted to the experimental data.

The behavior of the circadian network differs between individuals. Hence, it is crucial to evaluate the expression phenotypes in order to predict daily variations in cytotoxicity in a personalized manner. While related work gives a general prediction of optimal irinotecan timing (Li et al. 2013, Dulong et al. 2015), the approach of the present invention is personalized because prediction of optimal treatment time is based on the individual gene expression as measured in the saliva samples, and the model is fitted accordingly to these data, which allows for even better personalized predictions of optimal treatment time. The general predictions rely implicitly on the assumption that the regulation of the drug target gene by the core clock is the same in patients. However, by sampling directly the target gene plus circadian genes, the approach of the present invention predicts optimal treatment time better.

With reference to the drawings, a unifying model to optimize cancer treatment timing is described below.

Biological constituent of the human body form an intricate network of interactions. Because the level of interactions is extremely high, it is difficult to consider one pathway without considering its neighbors. It has been established a unifying model of the interaction between cancer and the core-clock. This improves the prediction of treatment timing in comparison to the currently existing sub-studies by explicitly considering their interactions, and allowing for a quantitative comparison of their effect size. This permits to optimize the treatment timing with respect to all relevant influences at the same time, compromising for example between the optimal cell cycle for treatment and the minimal toxicity for non-cancer cells. The model will include the following (with all cited references being from the group around the inventors of the present invention).

The core-clock network (Relogio et al. 2011). The detailed model of the core-clock allows for example to include the mutations in core-clock genes seen in around 4% of cancer patients (Yalçin et al. 2020).

Interaction between cancer and clock. A strong bidirectional interaction has been shown between cancer-related genes and circadian genes. Several potential clock-controlled genes have been identified which show a strong association with cancer and cancer progression, in particular related to metabolism (Yalçin et al. 2020, @abreu_reciprocal_2018, @fuhr_circadian_2018). In vitro, in vivo and in mathematical models we found that the core-clock impacts tumor growth (Basti et al. 2020). Furthermore, the experimental, bioinformatics and modelling data showed the inverse, that cancer-related genes impacting the core clock (Relogio et al. 2014). For example, using an inducible RAS expression system, we show that overexpression of RAS disrupts the circadian clock and leads to an increase of the circadian period while RAS inhibition causes a shortening of period length, as predicted by our mathematical simulations (Relogio et al. 2014).

The cell cycle network (El-Athman et al. 2017), which allows to take advantage of the minimization of treatment toxicity for non-cancer cells by timing treatment to their cell cycle. The relation was shown in experiments on cell cultures and in modelling simulations (El-Athman et al. 2017): The observed changes can be attributed to in silico phase shifts in the expression of core-clock elements. A genome-wide analysis revealed a set of differentially expressed genes that form an intricate network with the circadian system with enriched pathways involved in opposing cell cycle phenotypes. In addition, a machine learning approach complemented by cell cycle analysis classified the observed cell cycle fate decisions as dependent on Ink4a/Arf and the oncogene RAS and highlighted a putative fine-tuning role of Bmal1 as an elicitor of such processes, ultimately resulting in increased cell proliferation in the Ink4a/Arf knock-out scenario.

The irinotecan network (see below and (Ballesta et al. 2011)), which allows to access the impact of treatment on cancer and non-cancer cells (maximizing toxicity for cancer cells while minimizing toxicity for non-cancer cells by taking advantage of their differential circadian rhythms).

Tumor motility network. It was found that the core-clock impacts > metastasis (Basti et al. 2020) The reasoning here is that treatment timing > that suppresses metastasis might be more relevant for the > long-term survival of the patient than optimizing treatment to > kill existing cancer cells.

Host-microenvironment: Cancer cells live in an environment that contains other tissues such as fibroblasts. For fibroblasts it was shown that their communication with (benign) tumor cells affect the molecular clockwork and seem to aggravate malignant cell phenotypes (Fuhr et al. 2019). This communication will be modeled as a bath solution in which the cancer cell lives, and which also interacts with the core-clock.

Given the range of molecular time-dependent processes that it regulates, including metabolism, DNA repair and the cell cycle, the circadian clock has the potential to act as a tumour suppressor (El-Athman et al. 2017). The present invention helps to reach the full potential of the circadian clock as tumour suppressor by strengthening the circadian clock rhythm (i.e. increasing the amplitude), not only by optimizing the treatment but also by optimizing other daily activities that influence the circadian clock (eating, sleep rhythm, day light exposure, etc.).

Modelling the irinotecan network is one aspect of the prediction for optimal cancer treatment in accordance with the present invention. The 24-hour light/dark transitions of the Earth led organisms to evolve an endogenous timing-mechanism: the circadian clock, which accounts for rhythmic oscillations in physiological, behavioural, and cellular functions. Circadian rhythms influence drug pharmacology and ultimately tolerability and efficacy of drugs. This impacts the treatment of several diseases, including cancer. Here we focus on the irinotecan (CPT11) treatment of colorectal cancer (CRC). Both, the target gene of CPT11 (Top1) and the genes involved in its pharmacokinetics and pharmacodynamics (PK-PD), show a circadian oscillation in their expression. Hence, CPT11 efficacy and toxicity display circadian rhythms in cancer patients. To identify the optimal time for drug delivery, it has been established a new ODE mathematical model of the circadian clock which includes genes involved in the processing of CPT11, influencing its cytotoxicity. The personalization of the model is achieved by fitting the model parameters to experimental data of gene expression from different CRC cell lines.

In this exemplary embodiment for the drug irinotecan for which the target gene is TOPI, the core clock network by Relogio et al. (Relogio et al. 2011) is extended with equations that are derived from the Irinotecan network published by reference (Ballesta et al. 2011). It will be appreciated that this may be applied to other drugs and their respective target gene accordingly. Basically, irinotecan (CPT11) is a TOPI inhibitor. TOPI binds to DNA and relaxes supercoiled strands for replication and transcription. TOPI then dissociates from DNA allowing the reconnection of the strands. The active metabolite of CPT11 (SN38) prevent TOPI relegation by creating DNA/TOP1/SN38 complexes, inducing lethal DNA strand breaks.

Selection of genes based on (Dulong et al. 2015).

- Drug target gene: Top 1.
- Measured genes: Bmall, Per2, Top1, Ces2.
- Predicted genes: Ugt1a1, Abcb1.

The model simulates gene expression of the core-clock genes and clock-regulated genes via two interconnected feedback loops (Per/Cry loop and Rev-Erb/Ror/Bmal loop). The model parameters were fitted to the measured data of gene expression (see below). The estimation of irinotecan (CPT11)-induced cytotoxicity does not depend on the action of a single protein, but rather on the combinatory performance of the proteins encoded by the aforementioned genes.

CPT11 in the extracellular medium diffuses through the cell membrane and reaches the intracellular compartment, where it is bioactivated into SN38 via CES2. SN38 is detoxified into SN38G through UGT1A1. CPT11, SN38 and SN38G are transported out of the cell by ABCB1. *Top1* relaxes supercoiled DNA by creating TOP1/DNA complexes, which in combination to SN38 may form irreversible complexes ($I_{compl}$) leading to cell apoptosis.

The temporal evolution of apoptosis is calculates based on: (1) DNA repair mechanisms and apoptosis pathways, which follows a circadian rhythm and (2) intracellular concentration of irreversible SN38/DNA/TOP1 complexes, which in turns depends on the protein activity of UGT1A1, CES2, TOPI, and ABCB1.

Fig. 4 (from Dulong et al. 2015) illustrates a scheme depicting molecular PK-PD of irinotecan (CPT11). CPT11 in the extracellular medium diffuses passively through the cell membrane and reaches the intracellular compartment. It is then bioactivated into SN38 through carboxylesterases (CES) enzymatic activities. SN38 is detoxified into SN38G through UGT1As. CPT11, SN38 and SN38G are effluxed outside of the cells by ABC (ATP-binding cassette) transporters (ABC_CPT, ABC_SN, and ABC_SNG, respectively). Topoisomerase 1 (TOPI) is an enzyme that relaxes supercoiled DNA by creating transient DNA/TOP1 complexes. SN38 and CPT11, to a lesser extent, stabilize them into reversible complexes, which may become irreversible after collision with replication or transcription mechanisms. This may trigger the apoptotic machinery though the cleavage of caspase-3, ultimately leading to cell apoptosis. Proteins involved in irinotecan efflux (ABC_CPT, ABC_SN, ABC_SNG), bioactivation (CES), and deactivation (UGTIAs), together with irinotecan target (TOPI) present experimentally demonstrated circadian rhythms.

Fig. 5 schematically illustrates the model extension: The core-clock network is complemented with additional genes associated with the irinotecan metabolism.

Harmonic regression is then applied to the experimental data to establish oscillatory behavior, i.e. to fit periodic (oscillatory) curves to the experimental data. The genes of the molecular clock (core-clock genes) regulates the transcription of clock-controlled genes. In turn, clock-controlled genes govern the expression of genes involved in irinotecan PK-PD. It is observed 24h-oscillations in the expression of these genes in colorectal adenocarcinoma cell lines (SW480 (Fuhr et al. 2018)). 24h-period harmonic regression (dashed line) for experimental data (dots) from SW480 cell lines is shown in Fig. 6.

For obtaining a personalized model fit (see Fig. 7), the elements of the model are fitted to RNA experimental measurements retrieved from colorectal adenocarcinoma cell lines (SW480 (Fuhr et al. 2018)). The model fitting was achieved via a coordinate search method in which the badness of fit (BOF) was minimized by cycling through the parameters one at a time.

$$BOF = \sqrt{\sum_{i}^{\#genes}\sum_{j}^{n_i}\frac{(s_{i,j}-e_{i,j})^2}{n_i}\cdot w_1 + \sum_{i}^{\#genes} f_{p,i}\cdot w_2 + \sum_{i}^{\#genes} f_{t,i}\cdot w_3 + \sum_{i}^{\#genes}(A_{s,i}-A_{e,i})^2\cdot w_4}$$

$$f_{p,i} = \begin{cases} 0 & |t_{e_{\max,i}} - t_{s_{\max},i}| < \Delta t_e \\ (t_{e_{\max,i}} - t_{s_{\max},i})^2, & \text{otherwise} \end{cases} \qquad f_{t,i} = \begin{cases} 0 & |t_{e_{\min,i}} - t_{s_{\min},i}| < \Delta t_e \\ (t_{e_{\min,i}} - t_{s_{\min},i})^2, & \text{otherwise} \end{cases}$$

A personalized chronotherapy can then be generated. The rhythmic expression of core-clock genes differs between tumour and normal cells. This impacts the circadian expression of the drug target gene and genes involved in the drug detoxification in tumour vs normal cells. Consequently, it is possible to predict a time window for treatment delivery that maximizes treatment efficacy and, at the same time, minimizes side effects. Fig. 8 shows a schematic representation for the rationale of cancer chronotherapy, where virtual data, not real data, have been used to create the scheme to show how the model works.

The data of the exemplary embodiment of Figs. 9a and 9b shows the gene expression measurements of two core-clock genes (*Bmal 1* and *Per2)* and two clock-regulated genes *(Top1, Ces)* crucial for irinotecan metabolism. The dots indicate the measured expression values for *Bmal1, Per2, Top1* and *Ces2.* The solid line represents the *in silico* gene expression generated with our mathematical model, which was fitted to the experimental data of the previously mentioned genes. The model additionally predicts the expression levels for *Ugtla1* and *Abcb1,* important for irinotecan pharmacokinetics and pharmacodynamics. It is shown a personalized model fit of core-clock genes (Fig. 9a) and the genes involved in the irinotecan metabolism (9b) based on the experimental data (dots).

With reference to Fig. 10 it is shown how the model allows to predict in addition the temporal dynamics of two additional genes involved in the irinotecan metabolism (Fig. 10a). Overall, the model fit allows for a prediction of the optimal treatment time with irinotecan (Fig. 10b). Here, the predicted time window for irinotecan delivery to minimize cytotoxicity in healthy tissue is 10:30 - 15:00 hours.

Fig. 11 illustrates a schematic representation of the core-clock network extended with Irinotecan-treatment relevant genetic network.

According to an exemplary embodiment of the method, the equations below are used to model the irinotecan network (illustrated in Fig. 11). As the skilled person will recognize, each drug used for a treatment would have a slight different network and thus a slightly different set of equations. Each treatment has a different model. In addition and because the circadian profile of core-clock genes and the drug target for each patient are measured, this is used as input data for the network and thus a very personalized model is produced for each patient (note that this applies for the differential equations model (ODE), which is different than the machine learning approach).

The equations below are neither in the Relogio et al, Plos Comp Bio 2011, nor in the Ballesta model ("A combined experimental and mathematical approach for molecular-based optimization of irinotecan circadian delivery" Annabelle Ballesta, Sandrine Dulong, Chadi Abbara, Boris Cohen, Alper Okyar, Jean Clairambault, Francis Levi). The interactions are directly taken from the complicated interaction network in the application. For the full circadian model for irinotecan, all equations from the Relogio et al, plus the equations below, plus equations (1) to (11) from Ballesta et al. are needed.

The medication pathway is regulated by the core-clock regulated genes that are modelled, and eventually results in a non-reversible triple complex which leads to DNA repair or apoptosis. The amount of this complex is the measure of toxicity (as also used in the Ballesta model).

Citation from Ballesta et al: "CPT11 activity is assessed by the amount of irreversible DNA/TOP1/SN38 complexes, chosen as the output variable because of its experimentally-proven correlation with CPT11 cytotoxicity."

For the full core-clock model reference is made to (Relogio et al. 2011). The model is extended by equations (1) to (11) from (Ballesta et al. 2011), and their equation (12) for the dynamics of protein amount is replaced by the actual core-clock regulated dynamics of the mRNA and cytoplasmic and nuclear proteins of the associated genes. The following list gives an overview over the additional dynamical variables, which are chosen in extension of the nomenclature of (Relogio et al. 2011):

TOP, $TOP_c$ and $TOP_n$ are mRNA, cytoplasmic and nuclear proteins associated with the gene TOPI.

NF, $NF_c$ and $NF_n$ are mRNA, cytoplasmic and nuclear proteins associated with the gene NFIL3.

CES, $CES_c$ and $CES_n$ are mRNA, cytoplasmic and nuclear proteins associated with the gene CES2.

$ABC_{CPT}$, $ABC_{CPTc}$ and $ABC_{CPTn}$ are mRNA, cytoplasmic and nuclear proteins associated with the gene group ABCB1, ABCC1, ABCC2.

$ABC_{SN}$, $ABC_{SNc}$ and $ABC_{SNn}$ are mRNA, cytoplasmic and nuclear proteins associated with the gene group ABCC1, ABCC2, ABCG1.

UGT, $UGT_c$ and $UGT_n$ are mRNA, cytoplasmic and nuclear proteins associated with the gene group of UGT1As.

The protein abundances in (Ballesta et al. 2011), equation (12) correspond here to the dynamical variables with the index n associated with the same genes or gene group.

From the core-clock model:

$x_1$, $x_5$, $x_6$ and , the pool of nuclear complexes PER/CRY, as defined in (Relogio et al. 2011).

Parameters:

*sf* = 1 is an overall scaling factor,

*ki51* = 3.3 is the inhibition constant of BMAL1 transcription by TOP1.

o = 1 is the Hill coefficient of inhibition of BMAL1 transcription by TOPI,

u = 1 is the Hill coefficient of inhibition of TOPI transcription by $NFIL3_n$,

*ki*6*b* is the inhibition constant of Top transcription by $NFIL3_n$,

Furthermore the following parameters for the mRNA *i* are defined:

- the degradation rate of the associated nuclear protein *dn*(*i*),
- the degradation rate of the associated cytoplasmic protein *dc*(*i*),
- the production rate of the associated cytoplasmic protein *kp*(*i*),
- the import rate to the nucleus of the associated cytoplasmic protein *kn*(*i*),
- the maximal transcription rate *Vmax*(*i*),
- the degradation rate of the mRNA *d*(*i*),
- the Hill coefficient of activation of transcription *act*(*i*),

- the Hill coefficient of inhibition of transcription *inh*(*i*),
- the rate constant of transcription *kt*(*i*),
- the inhibition constant of transcription *ki*(*i*),
- and the fold activation of transcription $f_{Act}$ (*i*),

The nuclear protein associated with TOPI is created by importing the cytoplasmic protein associated with TOPI and it is degraded:

$$\frac{dTOP_n}{dt} = sf(kn(TOP)TOP_c - dn(TOP)TOP_n)$$

Production of TOPI: The cytoplasmic protein associated with TOPI is created based on the available mRNA and is reduced by being transported into the nucleus or by degradation:

$$\frac{dTOP_c}{dt} = sf(kp(TOP)(TOP + TOP_0) - kn(TOP)TOP_c - dc(TOP)TOP_c)$$

where $TOP_0$ is the initial value of TOPI gene expression.

The equations for the import into the nucleus and the production of the cytoplasmic protein of NF, CES, ABC_, and UGT1A1 are formulated in analogue.

To account for the activation of BMAL1 by TOP1, the transcription term for BMAL1, $V5max\frac{1+i(x_6/kt5)^n}{1+(x_5/ki5)^m+(x_6/kt5)^n}$ is extended as $V5max\frac{1+i(x_6/kt5)^n}{1+(x_5/ki5)^m+(x_6/kt5)^n}\frac{(x_8/ki51)^o}{1+(x_8/ki51)^o}$, for parameters see (Relogio et al. 2011).

The transcription of the gene is given for the mRNA *i* as

$$\frac{di}{dt} = sf(Vmax(i)trans(i) - d(i)i)$$

with *trans*(*i*) the following terms for transcription:

$$trans(TOP) = \frac{1 + \left(x_1/kt\,(TOP)\right)^{act(TOP)}}{1 + \left(\left(PC/ki\,(TOP)\right)^{inh(TOP)} + 1\right)\left(x_1/kt\,(TOP)\right)^{act(TOP)}}\frac{1}{1 + (NF_n/ki\,6b)^u}$$

$$trans(NF) = \frac{1}{1 + \left(x_6/ki\,(NF)\right)^{inh(Nf)}}$$

$$trans(CES) = \frac{1 + f_{Act}(CES)\left(NF_n/kt\,(CES)\right)^{act(CES)}}{1 + \left(\left(x_5/ki\,(CES)\right)^{inh(CES)} + 1\right)\left(NF_n/kt\,(CES)\right)^{act(CES)}}$$

$$trans(ABC_{SN}) = \frac{1 + f_{Act}(ABC_{SN})\left(P\,C/k\,t(ABC_{SN})\right)^{act(ABC_{SN})}}{1 + \left(\left(NF_n/ki\,(ABC_{SN})\right)^{inh(ABC_{SN})} + 1\right)\left(PC/kt\,(ABC_{SN})\right)^{act(ABC_{SN})}}$$

$$trans(ABC_{CPT}) = \frac{1 + f_{Act}(ABC_{CPT})\left(P\,C/k\,t(ABC_{CPT})\right)^{act(ABC_{CPT})}}{1 + \left(\left(NF_n/ki\,(ABC_{CPT})\right)^{inh(ABC_{CPT})} + 1\right)\left(PC/kt\,(ABC_{CPT})\right)^{act(ABC_{CPT})}}$$

$$trans(UGT) = \frac{1 + f_{Act}(UGT)\left(PC/kt\,(UGT)\right)^{act(UGT)}}{+\left(PC/kt\,(UGT)\right)^{act(UGT)}}$$

Several studies show that the timing of drug administration affects how, for example, cancer cells react to the drug. It was shown that in colorectal cancer, the effect of drug administration of Oxaliplatin, a platinum complex that is used effectively for CRC treatment and WZB117, a glucose transporter inhibitor, is time-dependent and this effect is lost when the circadian clock of the cells is impaired after downregulating the core-clock gene *BMAL1* (Fig. 12, (Fuhr et al., 2018)).

Fig. 12 (which is Fig. 4 from Fuhr et al. EBioMedicine (2018) illustrates that BMAL1- and HKDC1-KD leads to metabolic changes in SW480 colorectal cancer cells and altered drug response in a time-dependent manner. (a) Glycolysis of SW480 control, shBMAL1 and shHKDC1 cells at different time-points after synchronization. Cells were either untreated or treated with WZB117 or Oxaliplatin. Mean ± SEM, n = 5. (b) Glycolytic capacity of SW480 control, shBMAL1 and shHKDC1 cells treated at three different time points after synchronization (18 h, 21 h, 24 h). Cells were either untreated or treated with WZB117 or Oxaliplatin. Mean ± SEM, n = 5.

In addition to the colon cancer cell line SW480, data were obtained for two different colon cancer cell lines HCT116 and SW620, which are derived from a primary tumour and a metastatic lymph node, respectively, that point towards to regulation of drug response via the circadian core-clock gene *EMAL1.* Here, the three cell lines were treated with several anti-cancer drugs, including WZB117, Oxaliplatin and cisplatin, and the rhythmic promoter activity of *BMAL1* was measured over several days (Fig. 13). The data shows that the drug application altered the rhythmicity of the core-clock gene *BMAL1* and hence the circadian clock machinery, indicating an important interplay between drug effect and the circadian clock in cancer cells. More specifically, HCT116, SW480 and SW620 colon cancer cells were treated with Oxaliplatin, Cisplatin and WZB117. BMAL1-Promoter activity was measured using live-cell bioluminescence recording over five days. Shown is one representative plot for the control (black) and the treated (grey dashed) condition, n = 3.

It was also shown a similar effect of drug administration on the circadian clock using the drug Irinotecan, which is widely used in combination with other anti-cancer drugs (e.g. Oxaliplatin) in treating colorectal cancer patients. It is known that Irinotecan, a topoisomerase I inhibitor with complex metabolism and known activity against colorectal cancer is directly linked to clock gene *BMAL1* expression (Dulong et al., 2015). The data shows that Irinotecan administration alters *BMAL1* rhythmic promoter activity in in three colon cancer cells investigated differently (Fig. 14). More specifically, HCT116, SW480 and SW620 colon cancer cells were treated with Irinotecan at different concentrations. BMAL1-Promoter activity was measured using live-cell bioluminescence recording over five days. Shown is one representative plot for the control (black) and the treated (grey) condition, n = 3.

In addition to the *BMAL1* rhythmicity data, preliminary data were obtained pointing towards the differential regulation of cancer cell proliferation based on the timing of drug administration. Here stable core-clock downregulated (shBMAL1, shNR1D1 and shPER2) HCT116 cancer cell lines treated with 10$\mu$M Cisplatin at 18h, 21h and 24h after cell synchronization are used. There was detected reduced cell proliferation for shNR1D1 and shPER2 cells, when cells were treated at 24h after synchronization compared to the other time-points (Fig. 15). Cell proliferation was measured using confluence over five days. n = 3.

In another study on pancreatic cancer, it was shown that the timing of treatment administration has different effects in cell survival depending also on the additional perturbations generated to the cells (Fig. 16). Here, it was shown that pancreatic cancer cells at different tumour stage have different response to the time-dependent administration of the chemotherapy drug gemcitabine, which is widely used for treating pancreatic cancer.

In particular, it was shown that the SMAD4 proficient pancreatic cancer cells (Panc1) are more resistant to treatment, while for the SMAD4 deficient AsPC1 cells this effect is not observed. Also, after the downregulation of core-clock gene *NR1D1* or clock-regulated gene *SMAD4* in Panc1, the differential drug response to different treatment time points is impaired.

Again, referring to Fig. 16 (Figure from Li et al. (2020), manuscript under revision), cytotoxicity assays for time-dependent gemcitabine treatment are illustrated with (A) Pipeline for the timing administration treatment and (B-C) At 17h, 20h, 23h after cell synchronization, gemcitabine was added to the cells. 72h after treatment, the number of living cells was quantified. Depicted are the comparisons to the 17h time point (mean ± SEM, n=3, *p < 0.05, **p < 0.01, ***p < 0.001).

Fig. 17 illustrates BMAL1 and PER2 expression display variation during the day in human blood, hair and saliva samples. (A) Three time-point comparison of BMAL1 and PER2 expression for the averaged data of all Participants in figure 1. Expression data is compared to the first time-point (Early). For hair and saliva data Early, Middle and Late time-points represent 9h, 17h and 21h, respectively. For PBMCs data Early, Middle and Late time-points represent 10h, 16h and 19h, respectively. Depicted are mean + SEM. (B) Time-course RT-qPCR measurements normalized to the mean of all time points (ΔΔCT) of BMAL1 (and PER2 of Participant 1, 2, and 13 with a fitted linear sine-cosine function (period = 24 h). For Participant 1, we collected one additional sample at 21h on the 2nd day. Harmonic regression best p-values for tested periods (20-28h): Participant 1; BMAL1 (0.517, period = 21.4h), PER2 (0.353, period = 24.0h). Participant 2; BMAL1 (0.038, period = 20.0h), PER2 (0.276, period = 28.0h). Participant 13; BMAL1 (0.014, period = 20h), PER2 (0.086, period = 21.4h). (C) Time-course RT-qPCR measurements of human PBMCs normalized to the mean of all time points (ΔΔCT) of BMAL1, CLOCK, NPAS2, PER2, CRY2, NR1D1, and RORB of Participant 2 and 5 with a fitted linear sine-cosine function (period = 24 h). Harmonic regression best p-values: Participant 2; BMAL1 (3.05E-01, period = 20h), CLOCK (6.31E-02, period = 28h), NPAS2 (1.67E-01, period = 20h), PER2 (4.78E-04, period = 20.8h), CRY2 (7.17E-01, period = 20h), *NR1D1* (1.48E-01, period = 28h) and RORB (7.58E-01, period = 20h). Participant 5; BMAL1 (5.56E-01, period = 20h), CLOCK (6.81E-01, period = 28h), NPAS2 (9.75E-02, period = 28h, PER2 (1.23E-01, period = 28h), CRY2 (5.40E-01, period = 28h), *NR1D1* (6.43E-01, period = 28h) and RORB (7.73E-01, period = 28h). (D) Average PER2 expression compared to BMAL1 using saliva time-course data for each participant (mean + SEM).

Figure 18 illustrates Gene expression of BMAL1 and AKT1 covary. (A) Mean-normalized gene expression profile for the three participants for whom the gene AKT1 was measured besides BMAL1 and PER2. The two days were treated as repetitions. The diurnal variation of AKT1 follows BMAL1. (B) The data points from the mean-normalized time-series of BMAL1 and AKT1 correlate, linear regression with $p = 0.018$. (C) Harmonic regression plots for the participants with at least 5 time points. Depicted values are based on individual best fitting period (20h - 28h, see also table A).

Figure 19 illustrates HST base line measurements. Depicted are mean values for three participants (9h-18h in one-hour intervals, N = 3, mean ± SEM). The HST measurements were randomly distributed across three measurement days with one day break in between. The red full circles represent the time point chosen for the subsequent training sessions.

Figure 20 illustrates Myotonometric analysis shows daily variation in muscle tone (frequency, F) for female and male participants. Only participants who completed all training sessions were included in the MyotonPRO measurements (N=12). Mean of normalized scores for the myotonometric parameter frequency [Hz] for each training session (T1-9h, T2-12h, T3-15h, T4-18h) and each muscle: M. Deltoideus, M. Triceps Brachii, M.adductor pollicis, M. rectus femoris, M. biceps femoris, M. gastrocnemius. The measurements were carried out from top to bottom on the right (Right bar) and the corresponding left (Left bar) side of the body. Corresponding statistics for intrapersonal variation between each time points can be found in Supplementary Table B. * $p < 0.05$, compared to time point 9h.For detailed overview see table B.

Figure 21 illustrates Standard deviations of normalized sports and muscle tone data (L: group with low BMAL1, H: group with high BMAL1). Mean standard deviation calculated on the normalized sports performance and the normalized muscle tone data for different (i) repetitions and timepoints, (ii) timepoints, (iii) repetitions (for details see Methods). (A) HST, (B) CMJ, (C) SRT (no repetitions were measured, thus the standard deviation (i) over all data is the same as (ii) over timepoints), (D) muscle tone of the leg muscles (M. rectus femoris, M. biceps femoris, M. gastrocnemius).

## Tables

Table A- Harmonic regression results of AKT1 for the best fitting period (see Fig. 18).

| Participant | qvals | pvals | Acrophase [h] | amplitude | Period [h] |
|---|---|---|---|---|---|
| Participant 3 | 0.249 | 0.178 | 18 | 1.506 | 28 |
| Participant 5 | 0.061 | 0.017 | 12 | 1.042 | 28 |
| Participant 6 | 0.011 | 0.001 | 11 | 1.068 | 26.6 |
| Participant 12 | 0.696 | 0.229 | 14 | 1.067 | 20 |
| Participant 21 | 0.249 | 0.167 | 14 | 1.386 | 28 |

Table B: Harmonic regression analysis (Figure 20).

| **Mesor** | **qvals** | **pvals** | **Acrophase [h]** | **Acrophase [radians]** | **Amplitude** 1.22 | **Period [h]** | **Condition** |
|---|---|---|---|---|---|---|---|
| -0.50 | 0.49 | 0.18 | 15.83 | 4.14 | | 23.6 | BMAL1_Participant 5 |
| 0.42 | 0.60 | 0.42 | 25.32 | 6.62 | 0.92 | 26.2 | PER2_Participant 5 |
| -0.12 | 0.24 | 0.01 | 6.67 | 1.74 | 1.80 | 28 | BMAL1_Participant 15 |
| -0.03 | 0.70 | 0.54 | 6.60 | 1.72 | 0.58 | 28 | PER2_Participant 15 |
| -0.03 | 0.31 | 0.05 | 0.59 | 0.15 | 1.49 | 20.9 | BMAL1_Participant 21 |
| 0.29 | 0.35 | 0.06 | 6.30 | 1.70 | 1.48 | 20 | PER2_Participant 21 |
| -2.03 | 0.52 | 0.23 | 15.08 | 3.94 | 4.38 | 25.2 | BMAL1_Participant 8 |
| -0.63 | 0.70 | 0.58 | 12.54 | 3.28 | 1.26 | 28 | PER2_Participant 8 |
| -0.71 | 0.21 | 0.01 | 18.33 | 4.79 | 3.85 | 22.9 | BMAL1_Participant 9 |
| -0.06 | 0.51 | 0.15 | 3.29 | 0.86 | 2.33 | 20 | PER2_Participant 9 |
| 0.02 | 0.15 | 0.01 | 20.33 | 5.32 | 1.78 | 20.8 | BMAL1_Participant 17 |
| 0.38 | 0.70 | 0.40 | 0.51 | 0.13 | 0.72 | 28 | PER2_Participant 17 |
| -0.01 | 0.48 | 0.14 | 21.05 | 5.51 | 0.28 | 22.1 | BMAL1_Participant 13 |
| 0.05 | 0.56 | 0.15 | 24.33 | 6.36 | 0.11 | 28 | PER2_Participant 13 |
| -0.16 | 0.70 | 0.54 | 17.04 | 4.46 | 0.42 | 28 | PER2_Participant 11 |
| -0.16 | 0.73 | 0.63 | 15.67 | 4.10 | 0.34 | 28 | BMAL1_Participant 11 |
| -1.47 | 0.45 | 0.06 | 14.34 | 3.75 | 3.50 | 26.6 | BMAL1_Participant 1 |
| 0.29 | 0.31 | 0.02 | 21.82 | 5.71 | 1.71 | 26.9 | PER2_Participant 1 |
| -0.85 | 0.21 | 0.02 | 16.80 | 4.39 | 2.65 | 23.5 | BMAL1_Participant 3 |
| -0.61 | 0.21 | 0.01 | 13.75 | 3.59 | 1.63 | 22.6 | PER2_Participant 3 |
| -0.12 | 0.10 | 0.03 | 13.36 | 3.49 | 1.43 | 20 | BMAL1_Participant 19 |
| 0.38 | 0.56 | 0.07 | 27.44 | 7.18 | 0.76 | 28 | PER2_Participant 19 |
| 0.17 | 0.88 | 0.58 | 4.98 | 1.30 | 1.07 | 20 | BMAL1_Participant 2 |
| 0.02 | 0.69 | 0.29 | 11.28 | 2.95 | 0.50 | 20.3 | PER2_Participant 2 |
| 0.00 | 0.60 | 0.31 | 21.15 | 5.53 | 0.56 | 23.4 | BMAL1_Participant 4 |
| -0.04 | 0.63 | 0.23 | 20.07 | 5.25 | 0.28 | 20.7 | PER2_Participant 4 |
| -0.01 | 0.67 | 0.28 | 1.88 | 0.49 | 0.71 | 20 | BMAL1_Participant 12 |
| -0.13 | 0.84 | 0.53 | 18.94 | 4.95 | 0.76 | 20 | PER2_Participant 12 |
| -0.60 | 0.46 | 0.09 | 12.22 | 3.19 | 1.46 | 26.7 | BMAL1_Participant 6 |
| -0.05 | 0.40 | 0.09 | 12.83 | 3.35 | 0.42 | 22 | PER2_Participant 6 |

Table C: List of participants and tests (MEQ, Sports tests, Molecular tests,

| | | | sports tests | | | molecular tests | | | myotonometry |
|---|---|---|---|---|---|---|---|---|---|
| **Participant #** | **gender** | **MEQ** | **# training sessions** | **# round of tests** | **HST_long** | **saliva** | **hair** | **blood** | **MyotonPRO** |
| 1 | male | intermediate | - | - | - | Y | Y | - | - |
| 2 | male | intermediate | - | - | - | Y | Y | Y | - |
| 3 | female | intermediate | - | - | **Y** | Y | - | - | - |
| 4 | male | moderate morning | - | - | **Y** | Y | Y | - | - |
| 5 | female | moderate morning | 4 | 1 | Y | Y | - | Y | Y |
| 6 | female | moderate evening | 4 | 1 | - | Y | - | - | Y |
| 7 | female | intermediate | 4 | 1 | - | - | - | - | Y |
| 8 | female | intermediate | 4 | 1 | - | Y | - | - | Y |
| 9 | female | moderate evening | 4 | 1 | - | Y | - | - | Y |
| 10 | male | intermediate | 4 | 1 | - | - | - | - | Y |
| 11 | male | intermediate | 4 | 1 | - | Y | - | - | Y |
| 12 | female | intermediate | - | - | - | Y | - | - | - |
| 13 | male | intermediate | 4 | 1 | - | Y | Y | - | Y |
| 14 | male | intermediate | 4 | 1 | - | - | - | - | Y |
| 15 | male | intermediate | 4 | 1 | - | Y | - | - | Y |
| 16 | male | moderate evening | 4 | 1 | - | - | - | - | Y |
| 5 | female | moderate morning | 3 | 2 | - | - | - | - | - |
| 8 | female | intermediate | 3 | 2 | - | - | - | - | - |
| 17 | female | moderate evening | 4 | 2 | - | Y | - | - | - |
| 18 | male | intermediate | 3 | 2 | - | - | - | - | - |
| 10 | male | intermediate | 4 | 2 | - | - | - | - | - |
| 19 | male | moderate morning | 3 | 2 | - | Y | - | - | - |
| 20 | male | moderate evening | 4 | 2 | - | - | - | - | - |

(continued)

| | | | sports tests | | | molecular tests | | | myotonometry |
|---|---|---|---|---|---|---|---|---|---|
| Participant # | gender | MEQ | # training sessions | # round of tests | HST_long | saliva | hair | blood | MyotonPRO |
| 21 | male | moderate morning | 4 | 2 | - | Y | - | - | - |
| Myotonometry) performed. Y = Yes, participant has carried out the test (see Fig. 3). | | | | | | | | | |

Altogether, the data shows that in the context of cancer treatment, the timing of drug application has a crucial role on cancer cell survival, which in turn, affects the overall outcome of the therapy in cancer patients. In this regard, the model applied in the method can be used to not only predict the most effective timing of therapy based on the individual's rhythm (e.g. of core-clock or drug target genes) but also to monitor and overcome circadian rhythm disruptions induced by (chemo)therapy regimens.

## Claims

1. Method of assessing circadian rhythm or circadian profile of a subject having cancer and/or assessing a timing of administration of a medicament to said subject having cancer, wherein said method comprises the steps of:

    • Providing at least three samples of saliva, more preferably four samples of saliva, from said subject, wherein said samples have been taken at different time points over the day;
    • Determining gene expression of the following genes in each of said samples:

        a. of at least two members of the core-clock network in each of said samples, in particular of at least two members of the following genes, of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, and
        b. at least one gene involved in the metabolism of a medicament to be administered to said subject having cancer, including Ces2, and
        c. at least one drug target gene that is a target of the medicament to be administered, and

    • Assessing and predicting by means of a computational step based on said expression levels of said genes over the day the circadian rhythm of said subject and/or assessing a timing of administration of said medicament to said subject, comprising assessing the optimal time of administration of said medicament to said subject and/or assessing the non-optimal time of administration of said medicament to said subject.

2. Method according to claim 1, wherein gene expression is determined using a method selected from quantitative PCR (RT-qPCR), NanoString, sequencing and microarray.

3. Method according to any of claims 1 or 2, wherein gene expression is determined using NanoString.

4. Method according to claim 3, wherein the at least one further gene involved in the metabolism of the medicament to be administered is at least one of Ugt1a1 and Abcb1.

5. Method according to any of claims 1 to 4, wherein said assessing the timing of administration of said medicament to said subject comprises evaluating the predicted gene expression levels and/or evaluating expression phenotypes based on the determined and/or predicted gene expression levels.

6. Method according to any of claims 1 to 5, wherein the computational step comprises processing the determined gene expression levels to derive characteristic data for each of said genes, said processing comprising determining the mean expression level of expression of a gene and normalizing the gene expression levels using the mean expression level.

**7.** Method according to claim 6, wherein said characteristic data comprise:

- the amplitude of change of expression of a gene, and/or the amplitude relative to one of the other genes, and/or
- the mean expression level of expression of a gene, and/ or and/or the mean relative to one of the other genes, and/or
- the peak expression level of a gene, and/or the peak relative to one of the other genes., and/or
- the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the amplitudes of change of expression of any two of ARNTL (BMAL1) and/or ARNTL2 and/or CLOCK, and/or NPAS2 and/or PERI and/or PER2 and/or PER3 and/or CRY1 and/or CRY2 and/or NR1D1 and/or NR1D2 and/or RORA and/or RORB and/or RORC, and/or
- the mean expression level of expression of ARNTL (BMAL1) and/or ARNTL2 and/or CLOCK, and/or NPAS2 and/or PERI and/or PER2 and/or PER3 and/or CRY1 and/or CRY2 and/or *NR1D1* and/or NR1D2 and/or RORA and/or RORB and/or RORC, and/ or
- the relative difference of the mean expression levels of expression of any two of ARNTL (BMAL1) and/or ARNTL2 and/or CLOCK, and/or NPAS2 and/or PERI and/or PER2 and/or PER3 and/or CRY1 and/or CRY2 and/or NR1D1 and/or NR1D2 and/or RORA and/or RORB and/or RORC, and/or
- the peak expression level of ARNTL (BMAL1) and/or ARNTL2 and/or CLOCK, and/or NPAS2 and/or PERI and/or PER2 and/or PER3 and/or CRY1 and/or CRY2 and/or *NR1D1* and/or NR1D2 and/or RORA and/or RORB and/or RORC over the day, and/or
- the relative difference of the peak expression levels of any two of ARNTL (BMAL1) and/or ARNTL2 and/or CLOCK, and/or NPAS2 and/or PERI and/or PER2 and/or PER3 and/or CRY1 and/or CRY2 and/or NR1D1 and/or NR1D2 and/or RORA and/or RORB and/or RORC, and/or
- the time of the peak expression level of ARNTL (BMAL1) and/or ARNTL2 and/or CLOCK, and/or NPAS2 and/or PERI and/or PER2 and/or PER3 and/or CRY1 and/or CRY2 and/or *NR1D1* and/or NR1D2 and/or RORA and/or RORB and/or RORC,
- the relative difference of the times of the peak expression level of any two of ARNTL (BMAL1) and/or ARNTL2 and/or CLOCK, and/or NPAS2 and/or PERI and/or PER2 and/or PER3 and/or CRY1 and/or CRY2 and/or NR1D1 and/or NR1D2 and/or RORA and/or RORB and/or RORC,

wherein the amplitude, period and phase expression level of expression of ARNTL (BMAL1) and/or ARNTL2 and/or CLOCK, and/or NPAS2 and/or PERI and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

**8.** Method according to claim 6 or 7, wherein the computational step further comprises

- fitting a network computational model to the derived characteristic data that comprises a representation of the periodic time course of the expression levels for each of said determined genes as well as a representation of the periodic time course of the expression level for at least one, preferably a plurality of further gene(s) included in a gene regulatory network that includes said genes; and/or
- training a machine learning algorithm on the derived characteristic data to form the network computational model, particularly optimize in terms of the representation of the periodic time course of the expression level for the at least one further gene.

**9.** Method according to any of claims 6 to 8, wherein assessing the timing of administration of said medicament to said subject comprises in the computational step fitting a prediction computational model on data obtained from said fitted periodic functions and/or said network computational model, wherein the prediction computational model is based on machine learning including at least one classification method and/or at least one clustering method, wherein said method(s) are preferably selected from the group comprising:
K-nearest neighbor algorithm, unsupervised clustering, deep neural networks, random forest algorithm, and support vector machines.

**10.** Method according to any of claims 1 to 9, wherein

- the medicament is Filgrastim and the target gene is *Csf3r* and the indication is acute myeloid leukemia; or
- the medicament is Rituximab and the target gene is selected from the group comprising *Fcgr2b, Ms4a1, and Fcgr3;* and the indication is rheumatoid arthritis and Non-Hodgkin's lymphoma; or

- the medicament is bevacizumab and the target gene is *Fcgr2b, Vegfa, Fcgr3;* and the indication is Colorectal cancer and Non-small cell lung cancer; or
- the medicament is trastuzumab and the target gene is Fcgr2b, Erbb2, Egfr, Fcgr3 and the indication is Breast cancer; or
- the medicament is Imatinib and the target gene is Ptgs1, Kit, Slc22a2, Abcg2, Pdgfra, Pdgfrb, Ddr1, Abca3, Abl1, Ret, Abcbla and the indication is Chronic myeloid leukemia; or
- the medicament is Pemetrexed and the target gene is Tyms, Atic, Gart, Slc29a1 and the indication is Mesothelioma and Non-small cell lung cancer; or
- the medicament is Capecitabine and the target gene is Cda, Tymp, Tyms, Ceslg, Dpyd and the indication is Breast cancer and colorectal cancer; or
- the medicament is Erlotinib (tyrosine kinase inhibitor, anticancer drug) and the target gene is EGFR, Ras/Raf/MAPK, and PIK3/AKT (tumour) and the indication is Tumour inhibition (ZT1 >> ZT13); or
- the medicament is Sunitinib (tyrosine kinase inhibitor, anticancer drug) and the target gene is Cyp3a11 (liver, duodenum, jejunum) abcb1a (liver, duodenum, jejunum, lung) and the indication is renal cell cancer and pancreatic neuroendocrine tumours; or
- the medicament is Lapatinib (dual tyrosine kinase inhibitor interrupting the HER2/neu and EGFR pathways, anticancer drug) and the target gene is EGFR/Ras/Raf/MAPK, Errfi1, Dusp1 (liver), Hbegf, Tgf$\alpha$, Eref (liver) and the indication is solid tumours such as breast and lung cancer; or
- the medicament is Roscovitine (seliciclib, CDK inhibitor, anticancer drug) and the target gene is Cyp3a11, Cyp3a13(liver) and the indication is non-small cell lung cancer (NSCLC) and leukemia; or
- the medicament is Everolimus (mTOR inhibitor, anticancer drug, immunosuppressant) and the target gene is mTOR/Fbxw7/P70S6K (tumour) and the indication is breast cancer; or
- the medicament is Irinotecan (Top1 inhibitor, anticancer drug) and the target gene is Ces2, Ugt1a1, abcbla, abcblb (liver and ileum), abcc2 (ileum) and the indication for colorectal cancer, advanced pancreatic cancer and small cell lung cancer; or
- the medicament is Tamoxifen (antiestrogenic, anticancer drug) and the target gene is Cyp2d10, Cyp2d22, Cyp3a11 (liver) and the indication is breast cancer; or
- the medicament Bleomycin (toxicant and anticancer drug) and the target gene is NRF2/glutathione antioxidant defence and the indication is pulmonary fibrosis, palliative treatment in the management malignant neoplasm (trachea, bronchus, lung), squamous cell carcinoma, and lymphomas.

**11.** Kit for sampling saliva for use in a method according to any of claims 1 to 10, comprising

- sampling tubes for receiving the samples of saliva, wherein each of the sampling tubes contains RNA protect reagent and is configured to enclose one of the samples of saliva to be taken together with the reagent, wherein preferably each of the sampling tubes is labelled with the time point at which the respective sample is to be taken and/or includes an indication about the amount of saliva for one sample.

**12.** Kit according to claim 11, wherein said sampling tubes are configured to receive a sample of saliva of 1 mL in addition to 1 mL of the RNA protect reagent, wherein the sampling tubes preferably are at least 2 mL tubes, preferably at least 3 mL tubes, more preferably at least 4 mL tubes, still preferably at least 5 mL tubes.

**13.** Use of the kit of any of claims 11 or 12 for collecting samples of saliva for providing the collected samples of saliva for a method of any of claims 1 to 10.

**14.** Method of RNA extraction for gene expression analysis from a sampling tube for receiving the sample of saliva comprising the steps of:

- Separating the sample of saliva by means of centrifugal force and generating a cell pellet;
- Separating the pellet from supernatant and homogenizing the pellet in an acid-guanidinium-phenol based reagent, preferably TRIzol;
- Adding an organic compound, preferably chloroform, and mixing said homogenate with a shaking device, preferably a vortexer, and obtaining a mixture;
- Separating said mixture by means of centrifugal force resulting in a solution having more than one phase with an upper aqueous phase comprising the RNA to be extracted; and
- Removing said RNA to be extracted in said aqueous phase from said solution having more than one phase.

**15.** Method according to claim 14, comprising additionally the steps of:

• Performing optionally a processing step for preparation of the extracted RNA samples for determining gene expression;
• Performing gene expression analysis.

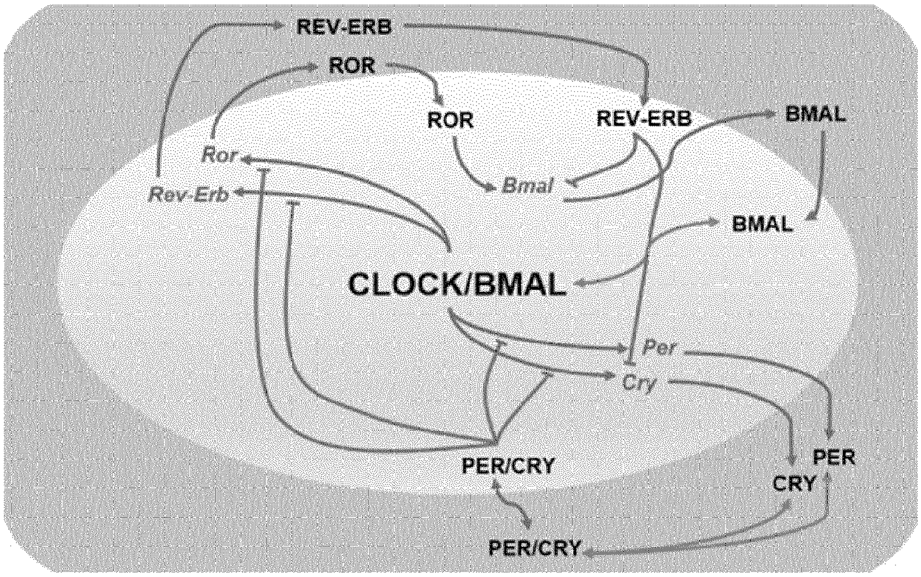
REV-ERB
ROR
ROR
REV-ERB
BMAL
Ror
Rev-Erb
Bmal
BMAL
CLOCK/BMAL
Per
Cry
PER/CRY
PER
CRY
PER/CRY

Fig. 1

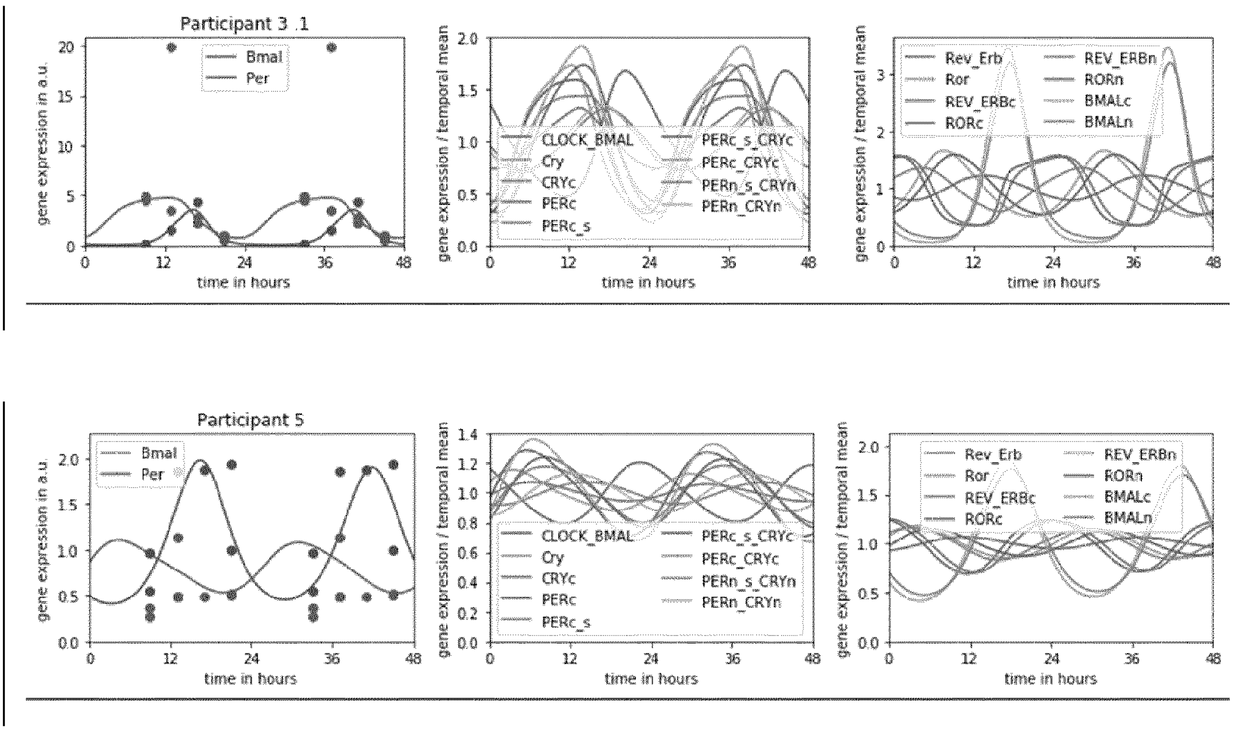
Participant 3 .1
Bmal
Per
gene expression in a.u.
time in hours
gene expression / temporal mean
CLOCK_BMAL
Cry
CRYc
PERc
PERc_s
PERc_s_CRYc
PERc_CRYc
PERn_s_CRYn
PERn_CRYn
Rev_Erb
Ror
REV_ERBc
RORc
REV_ERBn
RORn
BMALc
BMALn
Participant 5

Fig. 2

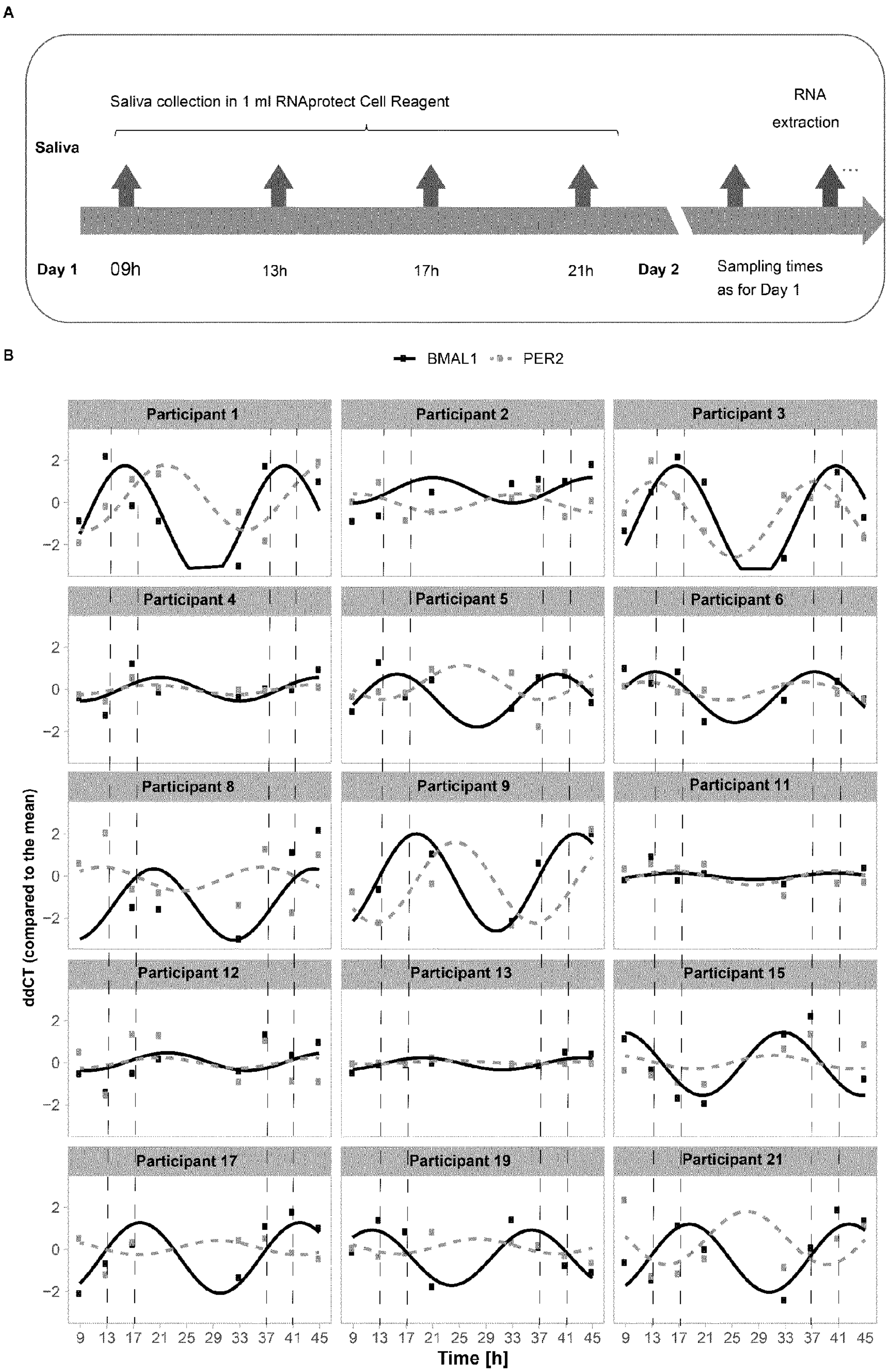
A
Saliva collection in 1 ml RNAprotect Cell Reagent
RNA
extraction
Saliva
Day 1
09h
13h
17h
21h
Day 2
Sampling times
as for Day 1
B
BMAL1
PER2
Participant 1
Participant 2
Participant 3
Participant 4
Participant 5
Participant 6
Participant 8
Participant 9
Participant 11
Participant 12
Participant 13
Participant 15
Participant 17
Participant 19
Participant 21
ddCT (compared to the mean)
Time [h]

Fig. 3

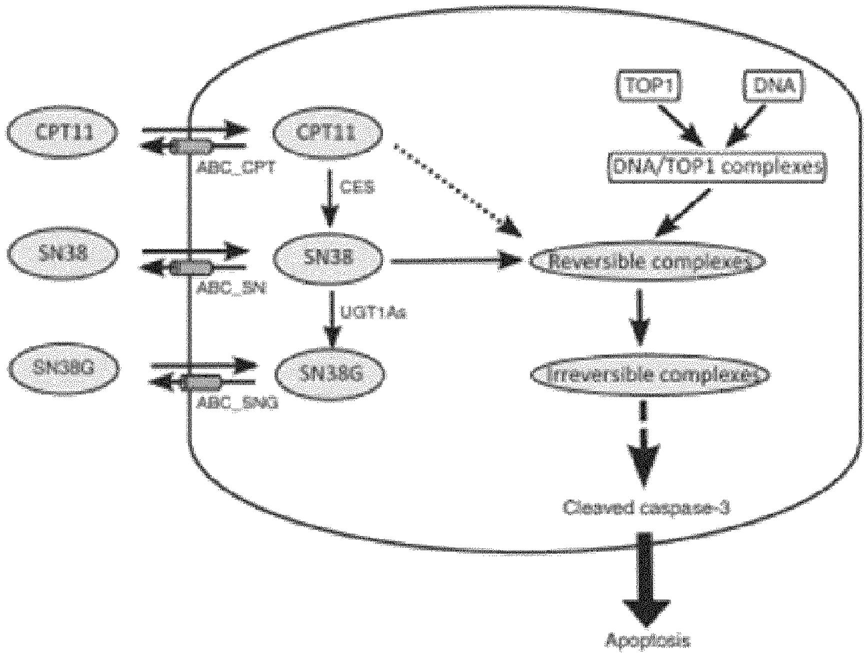
TOP1
DNA
CPT11
CPT11
ABC_CPT
CES
DNA/TOP1 complexes
SN38
SN38
ABC_SN
UGT1As
Reversible complexes
SN38G
SN38G
ABC_SNG
Irreversible complexes
Cleaved caspase-3
Apoptosis

Fig. 4

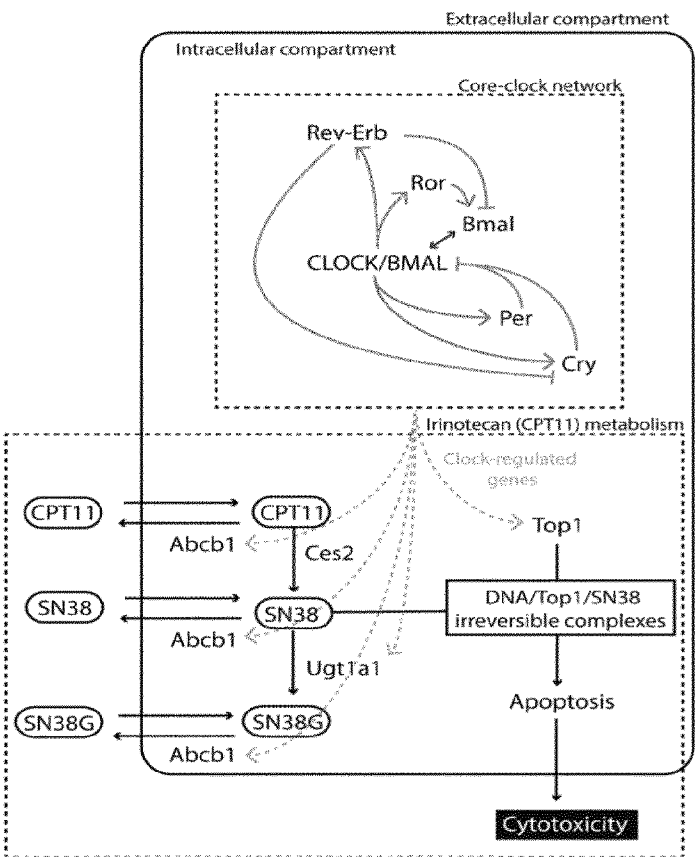
Extracellular compartment
Intracellular compartment
Core-clock network
Rev-Erb
Ror
Bmal
CLOCK/BMAL
Per
Cry
Irinotecan (CPT11) metabolism
Clock-regulated genes
CPT11
CPT11
Top1
Abcb1
Ces2
SN38
SN38
DNA/Top1/SN38 irreversible complexes
Abcb1
Ugt1a1
Apoptosis
SN38G
SN38G
Abcb1
Cytotoxicity

Fig. 5

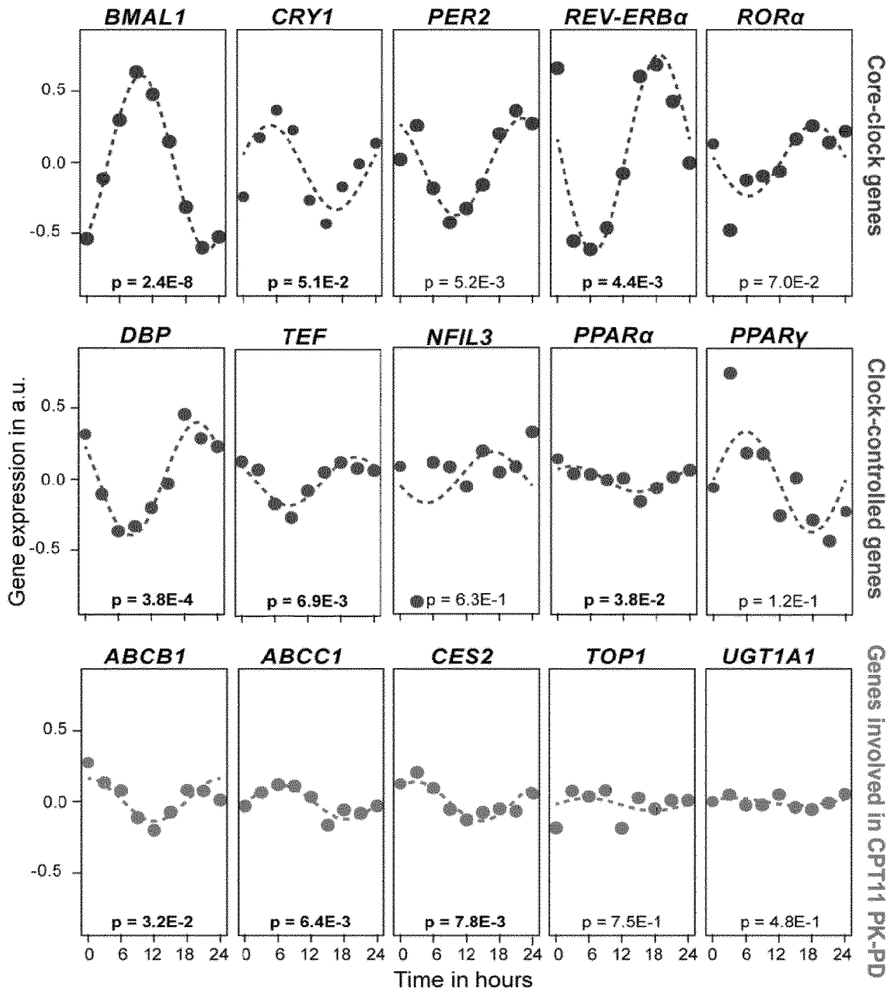

BMAL1
CRY1
PER2
REV-ERBα
RORα
p = 2.4E-8
p = 5.1E-2
p = 5.2E-3
p = 4.4E-3
p = 7.0E-2
Core-clock genes
DBP
TEF
NFIL3
PPARα
PPARγ
p = 3.8E-4
p = 6.9E-3
p = 6.3E-1
p = 3.8E-2
p = 1.2E-1
Clock-controlled genes
ABCB1
ABCC1
CES2
TOP1
UGT1A1
p = 3.2E-2
p = 6.4E-3
p = 7.8E-3
p = 7.5E-1
p = 4.8E-1
Genes involved in CPT11 PK-PD
Gene expression in a.u.
Time in hours


Fig. 6

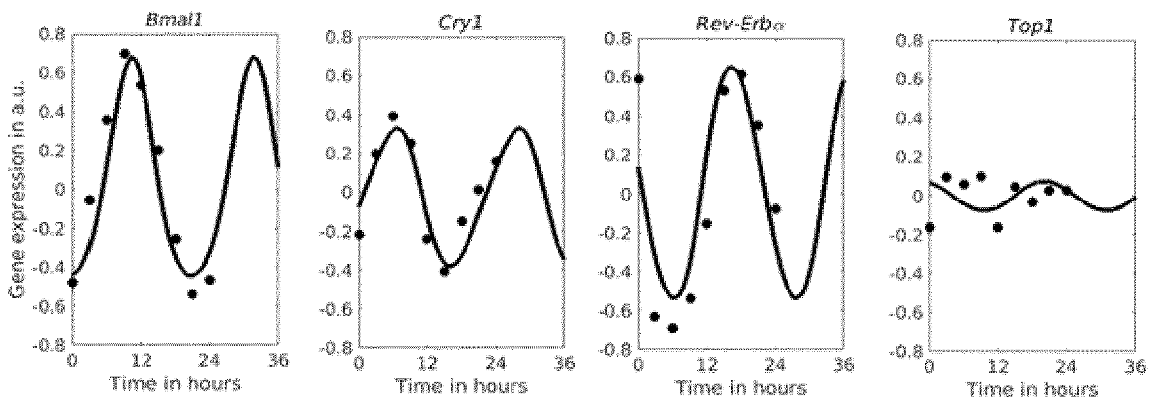

Bmal1
Cry1
Rev-Erbα
Top1
Gene expression in a.u.
Time in hours


Fig. 7

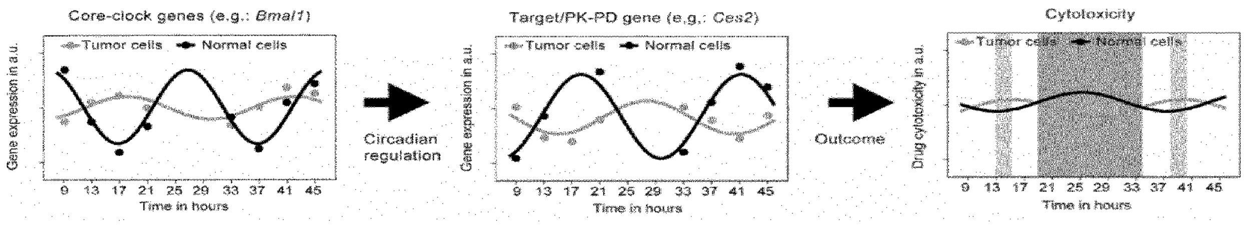


Fig. 8

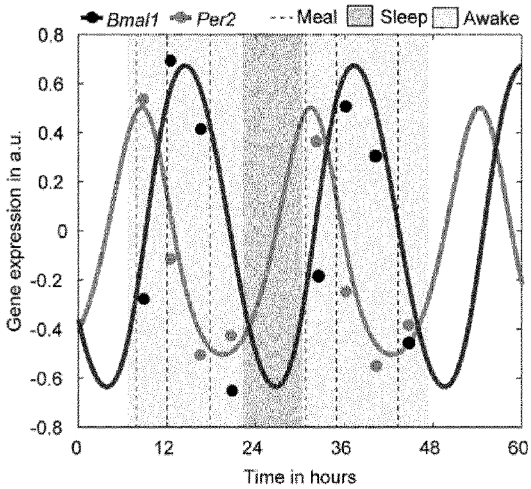


Fig. 9a

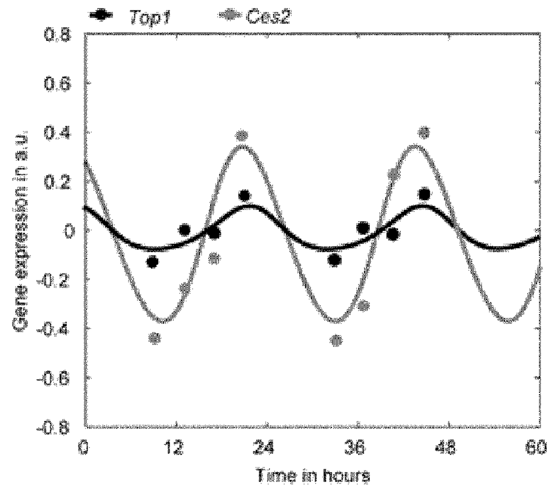


Fig. 9b

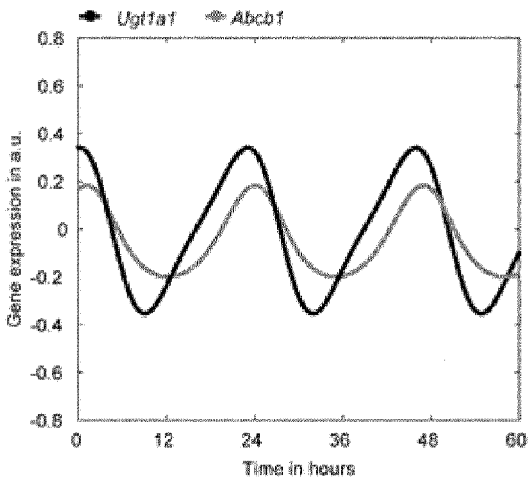


Fig. 10a

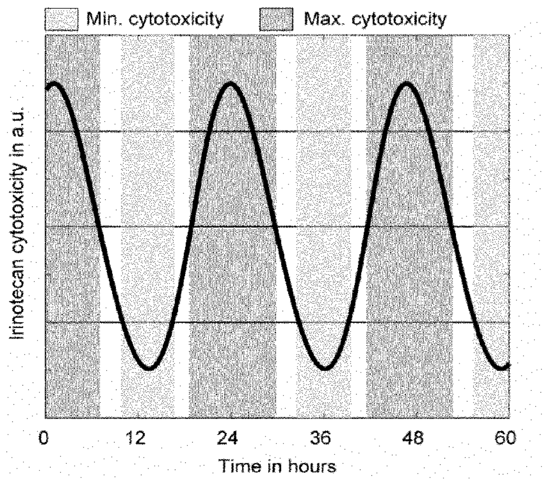


Fig. 10b

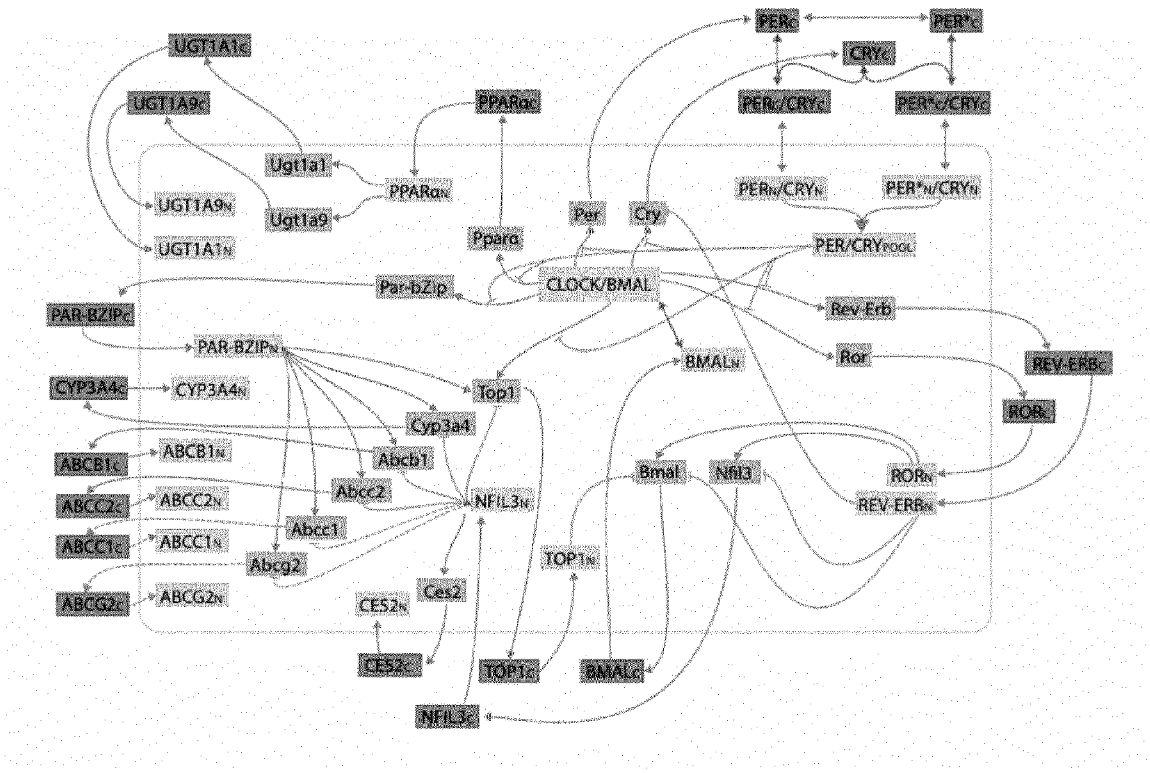

Fig. 11

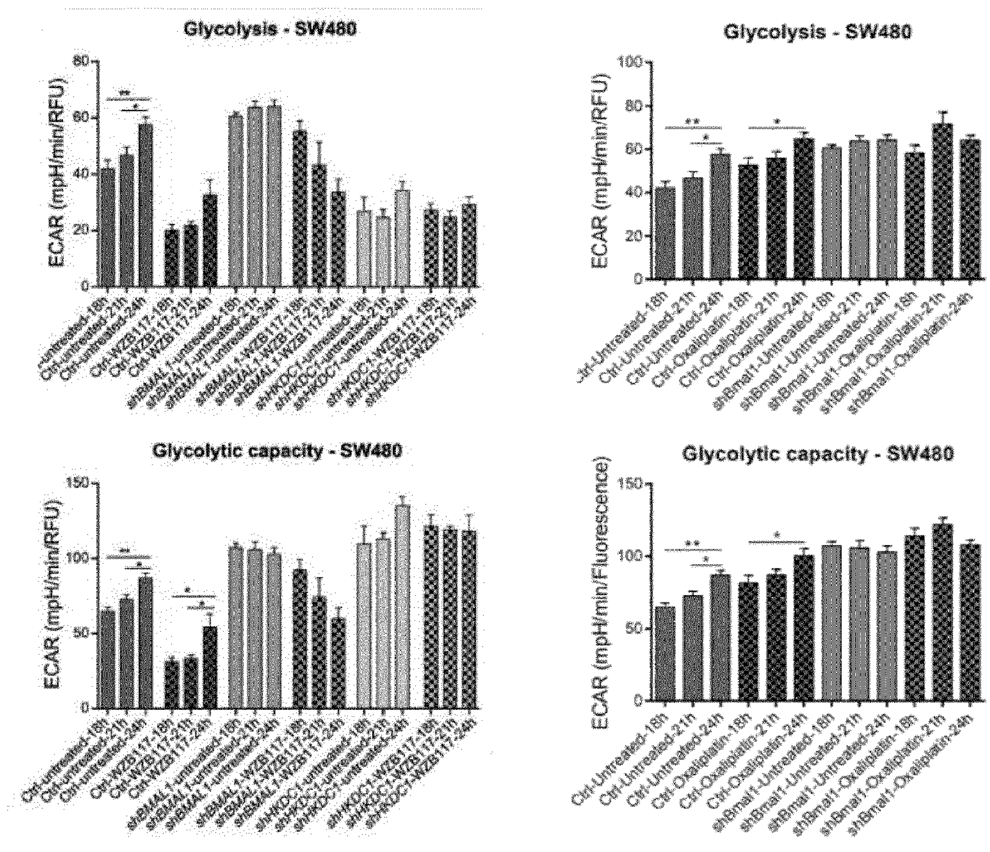

Fig. 12

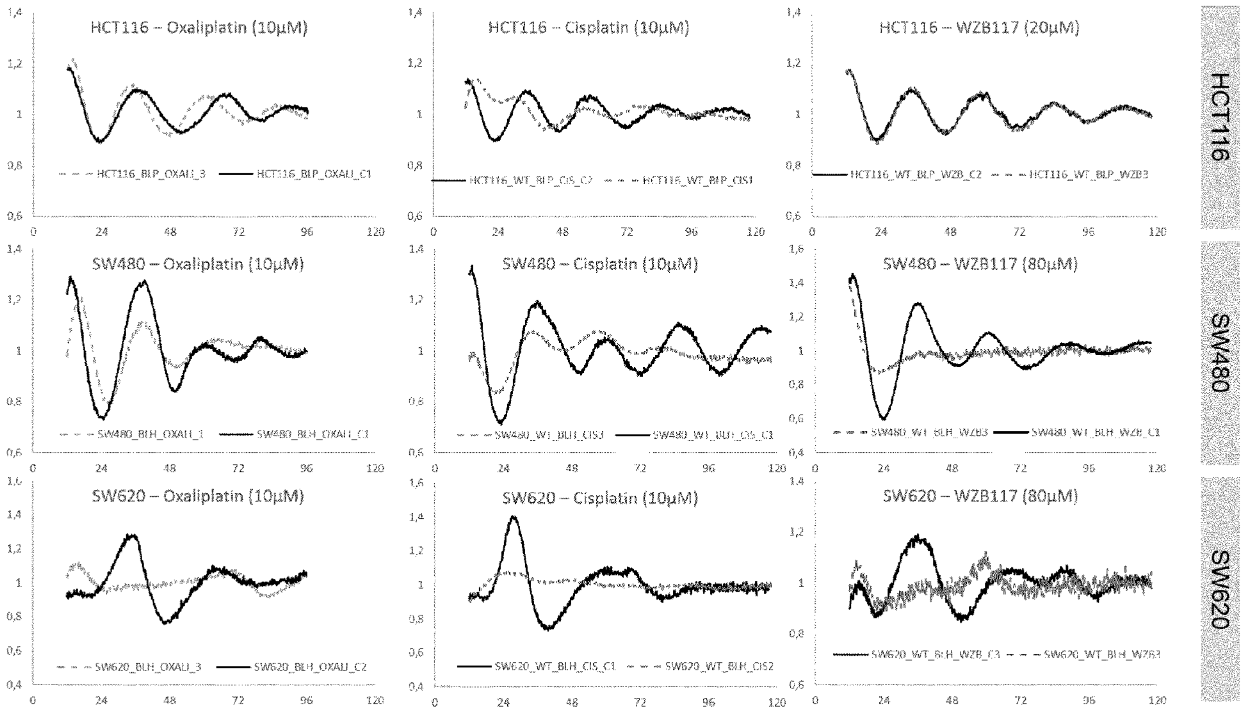
HCT116 – Oxaliplatin (10µM)
HCT116 – Cisplatin (10µM)
HCT116 – WZB117 (20µM)
HCT116
SW480 – Oxaliplatin (10µM)
SW480 – Cisplatin (10µM)
SW480 – WZB117 (80µM)
SW480
SW620 – Oxaliplatin (10µM)
SW620 – Cisplatin (10µM)
SW620 – WZB117 (80µM)
SW620

Fig. 13

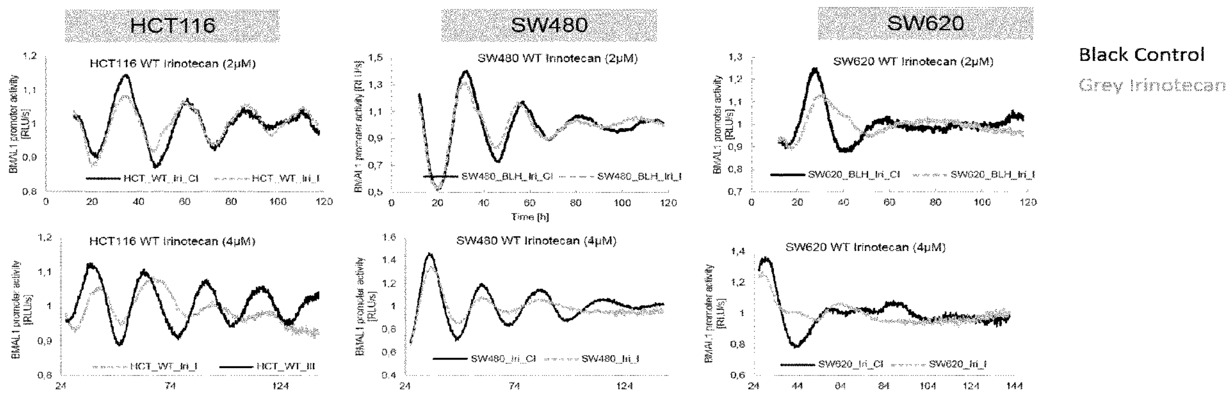
HCT116
SW480
SW620
HCT116 WT Irinotecan (2µM)
SW480 WT Irinotecan (2µM)
SW620 WT Irinotecan (2µM)
HCT116 WT Irinotecan (4µM)
SW480 WT Irinotecan (4µM)
SW620 WT Irinotecan (4µM)
BMAL1 promoter activity [RLU/s]
Time [h]
Black Control
Grey Irinotecan

Fig. 14

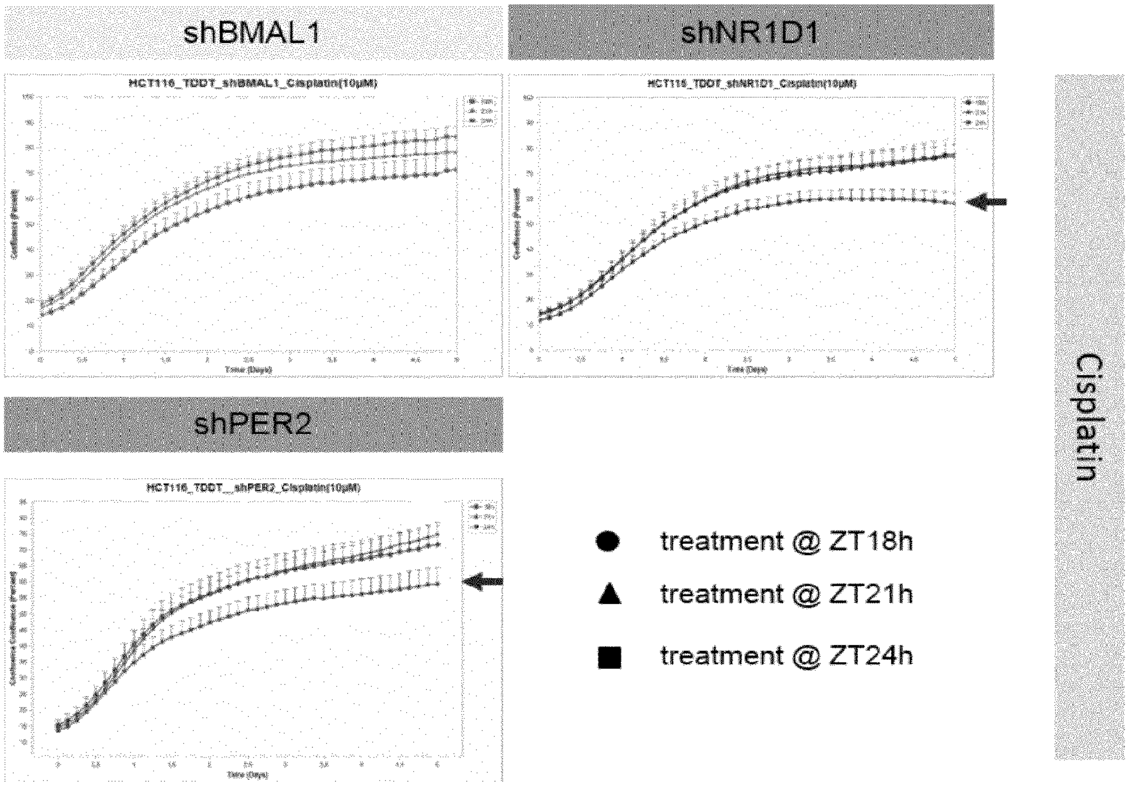
shBMAL1
shNR1D1
shPER2
Cisplatin
treatment @ ZT18h
treatment @ ZT21h
treatment @ ZT24h

Fig. 15

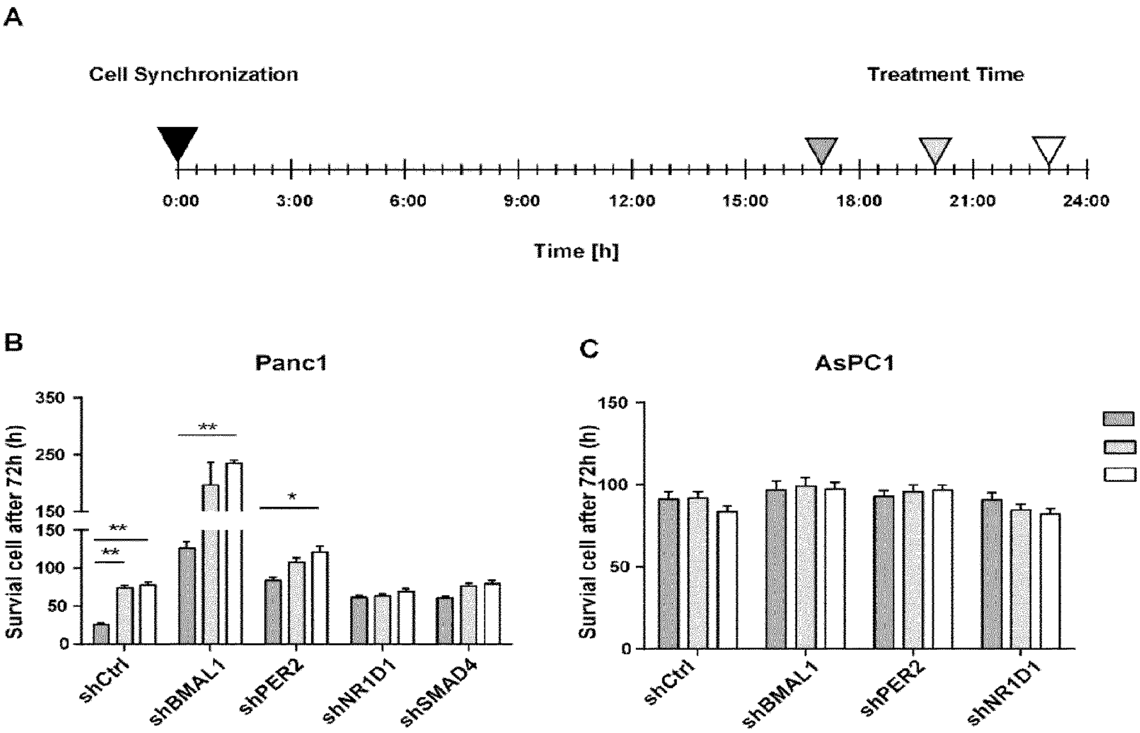
A
Cell Synchronization
Treatment Time
0:00
3:00
6:00
9:00
12:00
15:00
18:00
21:00
24:00
Time [h]
B
Panc1
Survial cell after 72h (h)
shCtrl
shBMAL1
shPER2
shNR1D1
shSMAD4
C
AsPC1
Survial cell after 72h (h)
shCtrl
shBMAL1
shPER2
shNR1D1
17h
20h
23h

Fig. 16

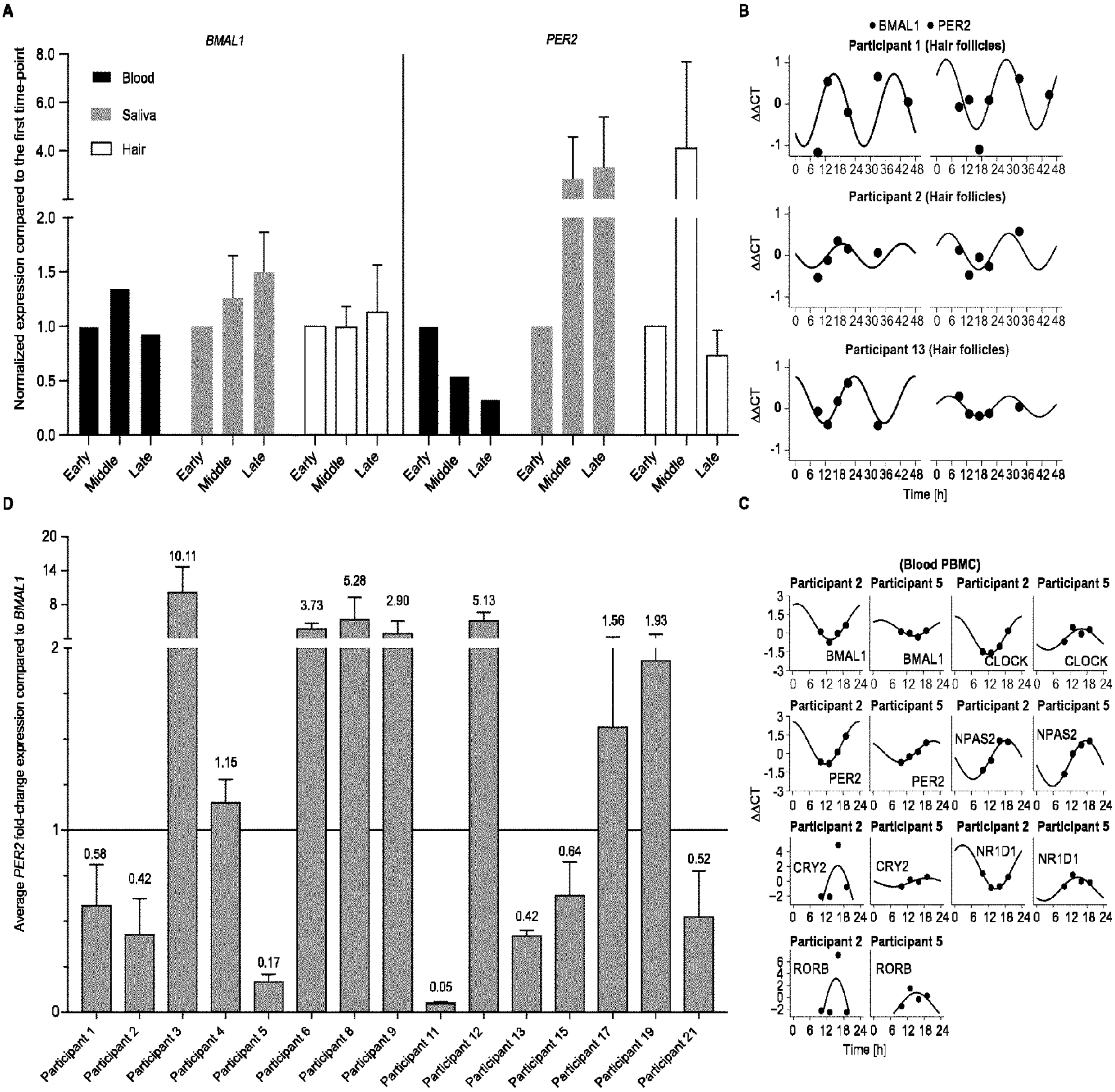
A
BMAL1
PER2
Blood
Saliva
Hair
Normalized expression compared to the first time-point
Early
Middle
Late
B
• BMAL1 • PER2
Participant 1 (Hair follicles)
Participant 2 (Hair follicles)
Participant 13 (Hair follicles)
ΔΔCT
Time [h]
C
(Blood PBMC)
Participant 2
Participant 5
BMAL1
CLOCK
PER2
NPAS2
CRY2
NR1D1
RORB
D
Average PER2 fold-change expression compared to BMAL1
0.58
0.42
10.11
1.15
0.17
3.73
5.28
2.90
0.05
5.13
0.42
0.64
1.56
1.93
0.52
Participant 1
Participant 2
Participant 3
Participant 4
Participant 5
Participant 6
Participant 8
Participant 9
Participant 11
Participant 12
Participant 13
Participant 15
Participant 17
Participant 19
Participant 21

Fig. 17

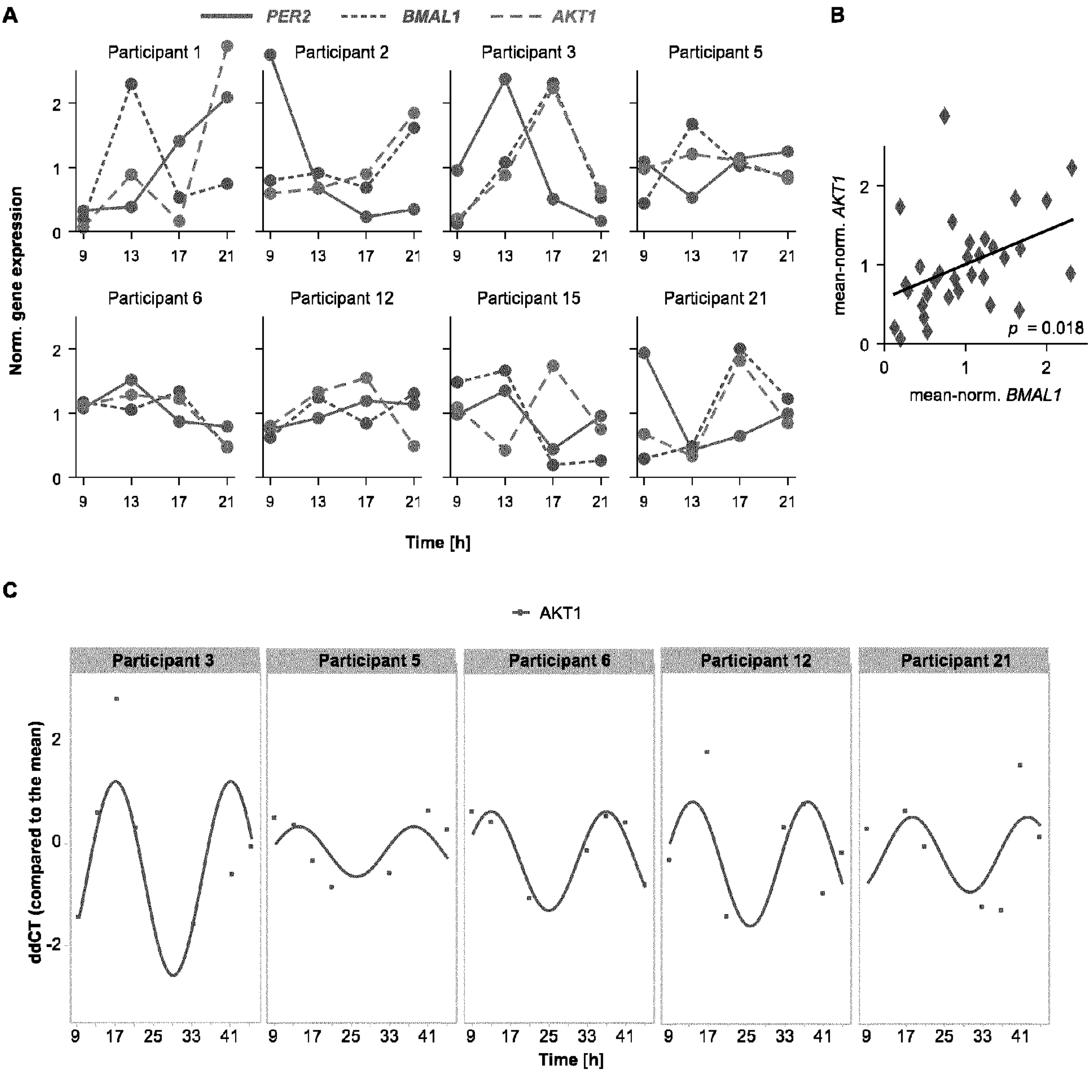
A
PER2
BMAL1
AKT1
Participant 1
Participant 2
Participant 3
Participant 5
Participant 6
Participant 12
Participant 15
Participant 21
Norm. gene expression
Time [h]
B
mean-norm. AKT1
mean-norm. BMAL1
p = 0.018
C
AKT1
Participant 3
Participant 5
Participant 6
Participant 12
Participant 21
ddCT (compared to the mean)
Time [h]

Fig. 18

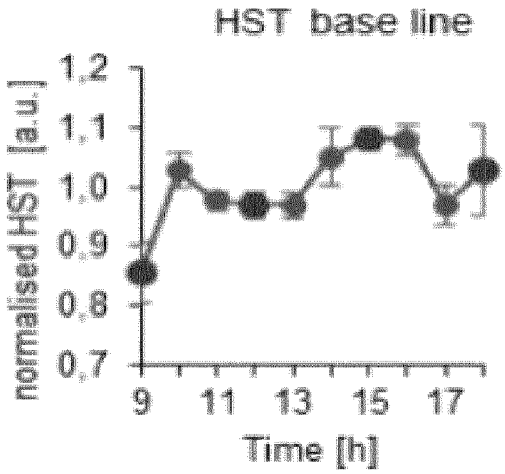
HST base line
normalised HST [a.u.]
1.2
1.1
1.0
0.9
0.8
0.7
9
11
13
15
17
Time [h]

Fig. 19

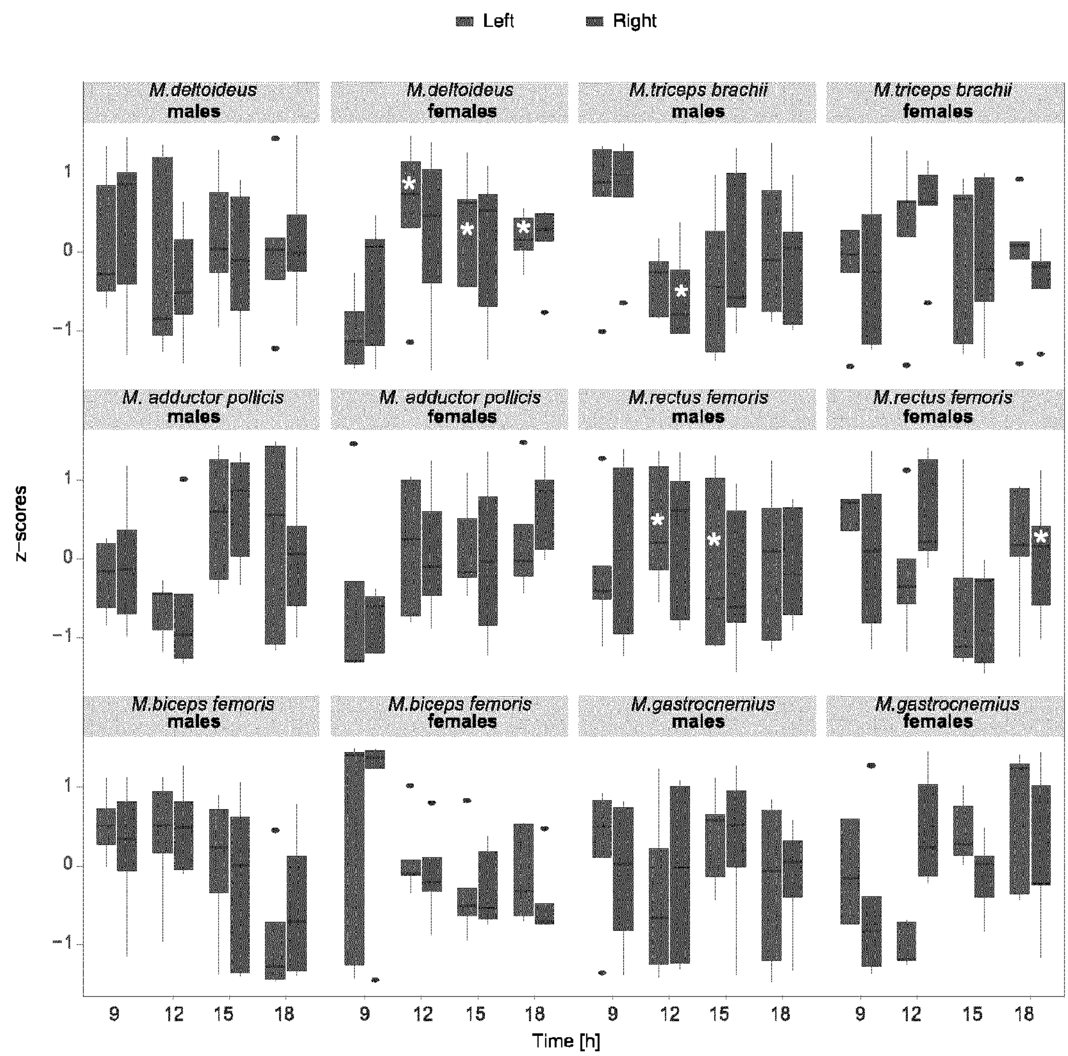
Left
Right
M.deltoideus males
M.deltoideus females
M.triceps brachii males
M.triceps brachii females
M. adductor pollicis males
M. adductor pollicis females
M.rectus femoris males
M.rectus femoris females
M.biceps femoris males
M.biceps femoris females
M.gastrocnemius males
M.gastrocnemius females
z-scores
1
0
-1
9
12
15
18
Time [h]

Fig. 20

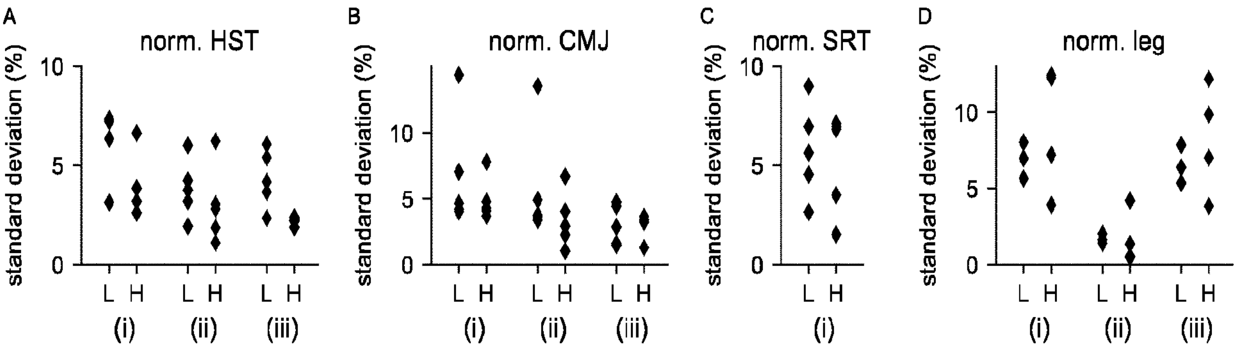
A
norm. HST
standard deviation (%)
10
5
0
L H
L H
L H
(i)
(ii)
(iii)
B
norm. CMJ
standard deviation (%)
10
5
0
L H
L H
L H
(i)
(ii)
(iii)
C
norm. SRT
standard deviation (%)
10
5
0
L H
(i)
D
norm. leg
standard deviation (%)
10
5
0
L H
L H
L H
(i)
(ii)
(iii)

Fig. 21

Europäisches Patentamt
European Patent Office
Office européen des brevets

# EUROPEAN SEARCH REPORT

Application Number
EP 20 19 3231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SOLOVYOV I A ET AL: "Genetic control of circadian rhythms and aging", RUSSIAN JOURNAL OF GENETICS, MOSCOW, RU, vol. 52, no. 4, 19 May 2016 (2016-05-19), pages 343-361, XP035707820, ISSN: 1022-7954, DOI: 10.1134/S1022795416040104 [retrieved on 2016-05-19] * the whole document * | 1-15 | INV. C12Q1/6886 |
| X | ROISIN SULLIVAN ET AL: "An optimised saliva collection method to produce high-yield, high-quality RNA for translational research", PLOS ONE, vol. 15, no. 3, 9 March 2020 (2020-03-09), page e0229791, XP055765879, DOI: 10.1371/journal.pone.0229791 | 11,12, 14,15 | |
| Y | * the whole document * | 1-10,13 | |
| X | JESSICA A. DIETZ ET AL: "Optimal Techniques for mRNA Extraction from Neonatal Salivary Supernatant", NEONATOLOGY, vol. 101, no. 1, 1 January 2012 (2012-01-01), pages 55-60, XP055765757, CH ISSN: 1661-7800, DOI: 10.1159/000328026 | 11,12, 14,15 | TECHNICAL FIELDS SEARCHED (IPC) |
| Y | * the whole document * | 1-10,13 | C12Q |
| X | WO 2011/094745 A2 (OASIS DIAGNOSTICS CORP; GIDDINGS JASON [US]; SLOWEY PAUL D [US]) 4 August 2011 (2011-08-04) | 11,12, 14,15 | |
| Y | * claims 1-19 * * paragraphs [0007], [0014], [0016] * | 1-10,13 | |
| | -/-- | | |

The present search report has been drawn up for all claims

1

| Place of search | Date of completion of the search | Examiner |
|---|---|---|
| The Hague | 27 January 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches Patentamt
European Patent Office
Office européen des brevets

# EUROPEAN SEARCH REPORT

Application Number
EP 20 19 3231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ANNABELLE BALLESTA ET AL: "A Combined Experimental and Mathematical Approach for Molecular-based Optimization of Irinotecan Circadian Delivery", PLOS COMPUTATIONAL BIOLOGY, vol. 7, no. 9, 8 September 2011 (2011-09-08), page e1002143, XP055768530, DOI: 10.1371/journal.pcbi.1002143 * the whole document * ----- | 1-10,13 | |
| Y | CONSTANCE AHOWESSO ET AL: "Sex and Dosing-Time Dependencies in Irinotecan-Induced Circadian Disruption", CHRONOBIOLOGY INTERNATIONAL, vol. 28, no. 5, 1 May 2011 (2011-05-01), pages 458-470, XP055768533, GB ISSN: 0742-0528, DOI: 10.3109/07420528.2011.569043 * the whole document * ----- | 1-10,13 | TECHNICAL FIELDS SEARCHED (IPC) |
| Y | DONG DONG ET AL: "Circadian rhythm in pharmacokinetics and its relevance to chronotherapy", BIOCHEMICAL PHARMACOLOGY, ELSEVIER, US, vol. 178, 22 May 2020 (2020-05-22), XP086219947, ISSN: 0006-2952, DOI: 10.1016/J.BCP.2020.114045 [retrieved on 2020-05-22] * the whole document * ----- -/-- | 1-10,13 | |

The present search report has been drawn up for all claims

1

| Place of search | Date of completion of the search | Examiner |
|---|---|---|
| The Hague | 27 January 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 19 3231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | INNOMINATO P F ET AL: "Chronotherapy and the molecular clock: Clinical implications in oncology", ADVANCED DRUG DELIVERY REVIEWS, ELSEVIER, AMSTERDAM , NL, vol. 62, no. 9-10, 31 July 2010 (2010-07-31), pages 979-1001, XP027212621, ISSN: 0169-409X [retrieved on 2010-06-23] * the whole document * ----- | 1-15 | |
| A | OHDO S ET AL: "Chronopharmacological strategies: Intra- and inter-individual variability of molecular clock", ADVANCED DRUG DELIVERY REVIEWS, ELSEVIER, AMSTERDAM , NL, vol. 62, no. 9-10, 31 July 2010 (2010-07-31), pages 885-897, XP027212613, ISSN: 0169-409X [retrieved on 2010-06-02] * the whole document * ----- | 1-15 | TECHNICAL FIELDS SEARCHED (IPC) |
| A | RANA SOBIA ET AL: "Circadian rhythm and its role in malignancy", JOURNAL OF CIRCADIAN RHYTHMS, BIOMED CENTRAL, LONDON, GB, vol. 8, no. 1, 31 March 2010 (2010-03-31), page 3, XP021079322, ISSN: 1740-3391, DOI: 10.1186/1740-3391-8-3 * the whole document * ----- | 1-15 | |
| | -/-- | | |

The present search report has been drawn up for all claims

1

| Place of search | Date of completion of the search | Examiner |
|---|---|---|
| The Hague | 27 January 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 19 3231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Prasanna V Ashok Kumar ET AL: "It's About Time: Advances in Understanding the Circadian Regulation of DNA Damage and Repair in Carcinogenesis and Cancer Treatment Outcomes", The Yale journal of biology & medicine, 1 June 2019 (2019-06-01), pages 305-316, XP055766015, United States Retrieved from the Internet: URL:https://www.ncbi.nlm.nih.gov/pmc/articles/PMC6585512/pdf/yjbm_92_2_305.pdf * the whole document * ----- | 1-10 | |
| | | | TECHNICAL FIELDS SEARCHED (IPC) |

1

The present search report has been drawn up for all claims

| Place of search | Date of completion of the search | Examiner |
|---|---|---|
| The Hague | 27 January 2021 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 4 of 4

## ANNEX TO THE EUROPEAN SEARCH REPORT ON EUROPEAN PATENT APPLICATION NO. EP 20 19 3231

This annex lists the patent family members relating to the patent documents cited in the above-mentioned European search report. The members are as contained in the European Patent Office EDP file on The European Patent Office is in no way liable for these particulars which are merely given for the purpose of information.

27-01-2021

| Patent document cited in search report | Publication date | Patent family member(s) | Publication date |
|---|---|---|---|
| WO 2011094745 A2 | 04-08-2011 | AU 2011210523 A1 | 27-09-2012 |
| | | EP 2537016 A2 | 26-12-2012 |
| | | US 2012310113 A1 | 06-12-2012 |
| | | WO 2011094745 A2 | 04-08-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KIM, JAE KYOUNG ; DANIEL B FORGER.** A Mechanism for Robust Circadian Timekeeping via Stoichiometric Balance. *Molecular Systems Biology,* December 2012, vol. 8, 630, https://doi.org/10.1038/msb.2012.62
- **GEISS G et al.** Direct multiplexed measurement of gene expression with color-coded probe pairs. *Nature Biotechnology,* 08 February 2008, vol. 26, 317-25
- **STROEBEL, A.M. ; BERGNER, M. ; REULBACH, U. ; BIERMANN, T. ; GROEMER, T.W. ; KLEIN, I. ; KORNHUBER, J.** Statistical methods for detecting and comparing periodic data and their application to the nycthemeral rhythm of bodily harm: A population based study. *Journal of Circadian Rhythms,* 2010, vol. 8
- **FUHR et al.** *EBioMedicine,* 2018